# EUROPEAN PATENT APPLICATION

(11) **EP 2 698 426 A1**
(43) Date of publication of application: **19.02.2014**
(21) Application number: 12771369.1
(22) Date of filing: 11.04.2012
(51) Int. Cl.: C12M 3/00, C12M 1/00, C12N 1/00, C12N 1/02

(54) **CELL-ADHERING LIGHT-CONTROLLABLE SUBSTRATE**

(30) Priority: 11.04.2011 JP 2011087647
(71) Applicant: The Toho University, Tokyo 143-8540 (JP); Hitachi High-Technologies Corporation, Tokyo 105-8717 (JP)
(72) Inventor: FURUTA Toshiaki, Funabashi-shi Chiba 274-8510 (JP); SUZUKI Akinobu, Funabashi-shi Chiba 274-8510 (JP); SUGIYAMA Hisashi, Tokyo 105-8717 (JP); OZAWA Satoshi, Tokyo 100-8220 (JP); TADA Hiroko, Tokyo 100-8220 (JP)
(74) Representative: Moore, Graeme Patrick
(86) International application number: PCT/JP2012/059880
(87) International publication number: WO 2012/141202

(57) **Abstract**

An object of the present invention is to enable simpler operation in real time and culture while removing unnecessary cells from cultured cells for purification in analyzing, fractionating, and culturing the cells alive and to analyze and fractionate desired cells from the cultured cells to increase the purity, recovery rate, and viability of the cells. The present invention employs a cell-adhesive photocontrollable base material, wherein light irradiation causes the bond dissociation of a photolabile group comprising a coumarinylmethyl skeleton to produce the separation of a cell-adhesive material to leave a non-cell-adhesive material. As a result, cell images can be detected and analyzed to obtain the positional information of desired cells. Based on the positional information thus obtained, the cells can be analyzed and fractionated alive.

## Description

### Technical Field

The present invention relates to the fields of regenerative medicine and stem-cell research, and particularly relates to a technique for analysis, fractionation and culture of cells.

### Background Art

In the field of regenerative medicine, operations are performed which involve identifying and isolating only a few somatic stem cells or progenitor cells contained in somatic cells and culturing, differentiating and inducing the somatic stem cells or progenitor cells to prepare somatic cells. Attempt is also made for differentiating and inducing pluripotent stem cells such as iPS cells or ES cells into somatic stem cells or somatic cells. However, the iPS cells or ES cells are not uniform and cells differentiation-induced therefrom are also not uniform, and various cells occur. Since usual pluripotent stem cells are often cocultured with cells called feeder cells, products obtained as a result of culture may be contaminated not only with the pluripotent stem cells or cells obtained by differentiation induction therefrom but with the feeder cells. Cells or tissues used for regenerative medicine are required to have uniformity as somatic cells, to contain somatic stem cells, and to be free of cancer cells or cancer stem cells or pluripotent stem cells such as iPS cells and ES cells, or feeder cells.

Thus, techniques for analyzing, fractionating, and culturing cells become increasingly important in the field of regenerative medicine. To fractionate cell groups of a plurality of types, an analysis technique is necessary for discriminating them. In addition, a single type of cells should be fractionated from the discriminated cell groups of a plurality of types. Only if the single type of cells can be fractionated therefrom, their molecular biological properties or cell biological properties can be analyzed. Also, only if the single type of cells can be fractionated or analyzed, a technique for strictly controlling differentiation induction can be developed. Such a single type of cells can be obtained at high purity if unnecessary cells can be removed in the intermediate stage of research and development. An experimental system equivalent to the achievement of a differentiation induction efficiency of 100% can therefore be constructed and is very effective for accelerating research and development.

Devices for analyzing cells alive include a light microscope, a fluorescence microscope for observing fluorescently labeled cells, and a fluorometric imaging device which are well known; however, these devices cannot fractionate cells. On the other hand, devices for fractionating cells alive include an device for fractionating and collecting desired cells by the antigen-antibody reaction between an antigen on the cell surface and an antibody added to magnetic beads; however, this device cannot analyze cells and has a problem in the purity, recovery rate, and the like thereof. Devices for fractionating cells also include a laser microdissection device; however, it is mainly used for isolation of a dead cell from a tissue section embedded in paraffin.

Devices for analyzing and fractionating cells alive include a sorting device applying a principle of a flow cytometry, which is well known. The device is an device which analyzes and discriminate cells by exposing the individual cells in a sample stream imposed on a sheath stream to laser light and observing scattered light or fluorescence, followed by giving charges to droplets containing the individual cells based on the information and performing fractionation and applying the electric field. A multicolored laser light can be irradiated to analyze many fluorescent markers; however, this is cumbersome in fluorescence correction or optical axis adjustment, the stable formation of droplets, and the adjustment of a timing at which charges are given to the droplets. For applying flow cytometry to cells cultured on a culture base material, the cells must be once removed from the culture base material. In addition, cell masses should be separated into individual cells in advance. When trypsin treatment or the like is performed for this purpose, the cells are not a little damaged. Such trypsin treatment also degrades proteins on the cell surface and might therefore interfere with the analysis of cell surface antigens. In addition, there are problems including that the viability of the sorted cells is reduced by impact during sorting.

Techniques for analyzing, fractionating, and culturing cells alive include a method as described in Patent Literature 1 (hereinafter, referred to as conventional example 1). This technique is associated with a device for using a cell culture base material on which a film formation of photoresponsive material whose physical properties are changed by light irradiation is conducted, adhering a cell to the cell-adhesive surface, discriminating between cultured cells with a monitor, locating desired cells, subjecting the desired cell position to light pattern irradiation, and detaching the desired cells from the culture base material. As the "photoresponsive material whose physical properties are changed by light irradiation" described there, one is mentioned which has a function by which cells are detached from the culture base material by the isomerization of the structure thereof by light irradiation to change the polarizability and hydophilic-hydrophbic property thereof; particularly, changes in these physical properties are considered to be preferably reversible.

A method described in Patent Literature 5 (hereinafter, referred to as conventional example 2) is known as a method for preparing a cell-immobilized substrate capable of formation of cell adhesion patterns and changes in the size of the patterns under cell culture. In the first aspect of this technique, a compound having a terminal functional group protected with a photolabile protecting group is immobilized on a substrate. A portion of the photolabile protecting group is eliminated by light irradiation to expose the terminal functional group. Cells are adsorbed onto the exposed terminal functional group. In the second aspect of this technique, which is however similar to the first aspect, a cell adhesion inhibitory substance is adsorbed onto the photolabile protecting group. A portion of the photolabile protecting group with the cell adhesion inhibitory substance adsorbed thereon is eliminated by light irradiation to expose the terminal functional group. Cells are adsorbed onto the exposed terminal functional group. In the third aspect of this technique, which is however similar to the second aspect, a cell adhesion promoting substance is adsorbed onto the exposed terminal functional group. Cells are adsorbed onto the cell adhesion promoting substance.

According to conventional example 2, the compound having a terminal functional group protected with a photolabile protecting group refers to a compound whose photolabile protecting group is eliminated by light irradiation to expose the terminal functional group. The photolabile protecting group mentioned therein refers to a protecting group onto which neither cells nor a cell adhesion promoting substance is adsorbed. Examples of the protecting group mentioned therein include highly hydrophilic ones and neutral compounds having a group with a hydrogen-binding acceptor. Specific examples of the cell adhesion inhibitory substance mentioned therein include serum albumin. According to conventional example 2, the terminal functional group refers to a functional group onto which cells are easily adsorbed. Specific examples thereof mentioned therein include charged functional groups such as a carboxyl group and an amino group. Specific examples of the cell adhesion promoting substance mentioned therein include fibronectin.

In any of these aspects of conventional example 2, a time on the order of 2 to 3 hours is required for causing cells to adhere to the compound immobilized on the substrate.

### Citation List

### Patent Literature

Patent Literature 1: JP Patent No. 3975266
Patent Literature 2: JP Patent No. 3472723
Patent Literature 3: JP Patent Publication (Kokai) No. 2004-170930 A
Patent Literature 4: JP Patent Publication (Kokai) No. 2008-167695 A
Patent Literature 5: JP Patent Publication (Kokai) No. 2006-6214 A

### Non Patent Literature

Non Patent Literature 1: Kazuhiko Ishihara, Seitai Zairyo (Biocompatible Material) 18 (1): 33 (2000).
Non Patent Literature 2: Y. Arima et al., J. Meter. Chem., 17, 4079(2007).
Non Patent Literature 3: Y. Arima et al., Biomaterials 28, 3074(2007).
Non Patent Literature 4: M. N. Yousaf et al., PNAS 98(11), 5992(2001).
Non Patent Literature 5: Toshiaki Furuta, Kogaku (Optics) 34 (4): 213 (2005)
Non Patent Literature 6: J. Edahiro et al., Biomacromolecules, 6(2), 970(2005).
Non Patent Literature 7: J. Nakanishi et al., Analytical Sciences, 24, 67 (2008).

### Summary of Invention

### Technical Problem

As the photoresponsive material whose physical properties are changed by light irradiation, described in the above conventional example 1 (JP Patent No. 3975266), the material whose structure is reversibly changed by light is difficult to be caused to 100% have one of the two isomers, which reduces the selectivity of cell adhesion. The material responsive to light of a long wavelength as described in the examples therein will be changed in adhesion, for example, by responding to exciting light by fluorescent observation, which will not provide compatibility between fluorescent observation and adhesive maintaining. In addition, while the technique can detach cells from a culture base material, it does not contemplate detachment of the adhesion between cells. Thus, the technique will wholly detach isolated cells or a cell mass present in the culture base material, leaving a problem that a cell mass consisting of a plurality of types of cells adhering to each other cannot be fractionated to single cells.

The specification of conventional example 1 does not describe specific conditions for causing cells to adhere to the material. Examples of conventional example 1 do not describe an experiment itself in which cells are caused to adhere to the material. This means that this technique does not give special consideration to cell adhesion.

In the first aspect of the above conventional example 2 (JP Patent Publication (Kokai) No. 2006-6214 A), it is required that the photolabile protecting group should permit no adsorption of cells whereas the terminal functional group exposed by eliminating the photolabile protecting group should permit exceedingly favorable adsorption of cells. A photolabile protecting group that exhibits such drastic changes in cell adhesiveness has not been known yet. In fact, no Example corresponding to this first aspect is described therein. Thus, there is a problem that this first aspect is difficult to carry out. The second aspect of conventional example 2 involves the step of allowing a cell adhesion inhibitory substance to be adsorbed onto the photolabile protecting group. It is also required that cells should be exceedingly favorably adsorbed onto the terminal functional group exposed by eliminating the photolabile protecting group. A terminal functional group having such exceedingly high cell adhesiveness has not been known yet. In fact, no Example corresponding to this second aspect is described therein. Thus, there is a problem that this second aspect is cumbersome due to many steps and is also difficult to carry out. The third aspect of conventional example 2 involves the steps of: allowing a cell adhesion inhibitory substance to be adsorbed onto the photolabile protecting group; and allowing a cell adhesion promoting substance to be adsorbed onto the exposed terminal functional group. The cell adhesion inhibitory substance has an unstable structure merely adsorbed on the photolabile protecting group. In allowing the cell adhesion promoting substance to be adsorbed, unintended side reaction through which the cell adhesion inhibitory substance adsorbed on the photolabile protecting group is desorbed and replaced with the cell adhesion promoting substance may occur depending on conditions. Once this side reaction occurs, an unintended region might be changed to a cell-adhesive region, resulting in the reduced selectivity of cell adhesion. Thus, there is a problem that this third aspect is cumbersome due to many steps and results in the low selectivity of cell adhesion depending on conditions. In any of these aspects of conventional example 2, a time on the order of 2 to 3 hours is required for causing cells to adhere to the compound immobilized on the substrate. Those skilled in the art generally regard this amount of time as being necessary for the adhesion of cells to a base material. However, for cell fractionation/isolation application for analytical purposes, cell adhesion, which is merely a part of this process, should be performed rapidly and conveniently. The time as long as 2 to 3 hours is not acceptable. In other words, there is a problem that the time required for cell adhesion of this conventional example is too long.

In view of the foregoing prior art, the present invention is directed to provide a cell-adhesive photocontrollable base material for analyzing, fractionating, and culturing cells alive.

An object of the present invention is to enable simpler operation in real time and culture while removing unnecessary cells from cultured cells for purification in analyzing, fractionating, and culturing the cells alive and to analyze and fractionate desired cells from the cultured cells to increase the purity, recovery rate, and viability of the cells as compared to before.

A second object of the present invention is to perform cell adhesion with reliability and in a short time in analyzing, fractionating, and culturing the cells alive.

### Solution to Problem

To solve the above prior art problems, the present invention has adopted the following means.

That is, the cell-adhesive photocontrollable base material of the present invention is obtained by conducting a film formation of a cell-adhesive photocontrollable material on a base material wherein a cell-adhesive material binds to a non-cell-adhesive material via a photolabile group in the cell-adhesive photocontrollable material in which the non-cell-adhesive material, the photolabile group and the cell-adhesive material are bound in this order from the base material side.

In the cell-adhesive photocontrollable base material of the present invention, light irradiation causes the bond dissociation of a photolabile group to separate a cell-adhesive material and leave a non-cell-adhesive material.

In the cell-adhesive photocontrollable base material of the present invention, light irradiation also causes the bond dissociation of a photolabile group to irreversibly change the surface of the irradiated portion thereof from the cell-adhesive material to a non-cell-adhesive material.

The cell-adhesive photocontrollable base material of the present invention is obtained by conducting a film formation of a cell-binding photocontrollable material on a base material wherein a cell-binding material binds to a non-cell-adhesive material via a photolabile group in the cell-adhesive photocontrollable material in which the non-cell-adhesive material, the photolabile group and the cell-binding material are bound in this order from the base material side. As used herein, the cell-binding material refers to a material capable of firmly binding to cells through a covalent binding or the like, or an animal species-specific antibody.

A method for analyzing and fractionating cells using the cell-adhesive photocontrollable base material of the present invention comprises the following steps.

A step of seeding and culturing cells on a cell-adhesive photocontrollable base material obtained by conducting a film formation of a cell-adhesive photocontrollable material on a base material wherein a cell-adhesive material or cell-binding material binds to a non-cell-adhesive material via a photolabile group in the cell-adhesive photocontrollable material in which the non-cell-adhesive material, the photolabile group and the cell-adhesive material or cell-binding material are bound in this order from the base material side, or a cell-adhesive photocontrollable base material, wherein light irradiation causes the bond dissociation of a photolabile group to separate a cell-adhesive material and leave a non-cell-adhesive material, or a cell-adhesive photocontrollable base material, wherein light irradiation causes the bond dissociation of a photolabile group to irreversibly change the surface of the irradiated portion thereof from the cell-adhesive material to a non-cell-adhesive material.

A step of detaching and recovering desired cells from the base material by first light irradiation to desired cellular regions.

A method for analyzing and fractionating cells using the cell-adhesive photocontrollable base material of the present invention also comprises the following step.

A step of providing cell-adhesive regions and a non-cell-adhesive region by first light irradiation on a cell-adhesive photocontrollable base material obtained by conducting a film formation of a cell-adhesive photocontrollable material on a base material wherein a cell-adhesive material or cell-binding material binds to a non-cell-adhesive material via a photolabile group in the cell-adhesive photocontrollable material in which the non-cell-adhesive material, the photolabile group and the cell-adhesive material or cell-binding material are bound in this order from the base material side, or a cell-adhesive photocontrollable base material, wherein light irradiation causes the bond dissociation of a photolabile group to separate a cell-adhesive material and leave a non-cell-adhesive material, or a cell-adhesive photocontrollable base material, wherein light irradiation causes the bond dissociation of a photolabile group to irreversibly change the surface of the irradiated portion thereof from the cell-adhesive material to a non-cell-adhesive material.

A method for analyzing and fractionating cells using the cell-adhesive photocontrollable base material of the present invention comprises the following steps.

A step of seeding cells on the cell-adhesive photocontrollable base material and then allowing centrifugal force to act in a direction toward the cell-adhesive photocontrollable base material, particularly preferably perpendicularly to the surface of the base material, in the step of the analysis and fractionation method described above.

A device for analyzing and fractionating cells using the cell-adhesive photocontrollable base material of the present invention comprises a cell-adhesive photocontrollable base material obtained by conducting a film formation of a cell-adhesive photocontrollable material on a base material wherein a cell-adhesive material or cell-binding material binds to a non-cell-adhesive material via a photolabile group in the cell-adhesive photocontrollable material in which the non-cell-adhesive material, the photolabile group and the cell-adhesive material or cell-binding material are bound in this order from the base material side, or a cell-adhesive photocontrollable base material, wherein light irradiation causes the bond dissociation of a photolabile group to separate a cell-adhesive material and leave a non-cell-adhesive material, or a cell-adhesive photocontrollable base material, wherein light irradiation causes the bond dissociation of a photolabile group to irreversibly change the surface of the irradiated portion thereof from the cell-adhesive material to a non-cell-adhesive material, and a first light irradiation means for subjecting the cell-adhesive photocontrollable material on the base material to photoreaction.

The present specification encompasses the contents of the specification and/or drawings of Japanese Patent Application No. 2011-087647 on which the priority of the present application is based.

### Advantageous Effects of Invention

In analyzing, fractionating, and culturing cells alive, operations can be more simply made in real time and culture can be performed while removing unnecessary cells from cultured cells for purification. Desired cells can also be analyzed and fractionated from the cultured cells to increase the purity, recovery rate, and viability of the cells. Analysis can also be made with more reliability and in a short time

### Brief Description of Drawings

[Figure 1] Figure 1 is a diagram showing a configuration of a method (Examples 1 and 2) for analyzing cells during culture and fractionating desired cells.
[Figure 2] Figure 2 is a diagram showing a configuration of a method (Examples 3, 5, 6 and 7) for analyzing individually separated cells and fractionating desired cells.
[Figure 3] Figure 3 is a diagram showing a configuration of a method (Example 4) for analyzing individually separated cells and fractionating desired cells.

### Description of Embodiments

Embodiments of the present invention will be described below. However, the present invention is not intended to be limited thereto.

One embodiment of the present invention is a cell-adhesive photocontrollable base material obtained by conducting a film formation of a cell-adhesive photocontrollable material on a base material wherein a cell-adhesive material or cell-binding material binds to a non-cell-adhesive material via a photolabile group in the cell-adhesive photocontrollable material in which the non-cell-adhesive material, the photolabile group and the cell-adhesive material or cell-binding material are bound in this order from the base material side. In the cell-adhesive photocontrollable material, light irradiation can cause the bond dissociation of the photolabile group to separate the cell-adhesive material from the base material. The light bond dissociation may be between the non-cell-adhesive material and the photolabile group or between the photolabile group and the cell-adhesive material. The light irradiation leaves the non-cell-adhesive material in the base material. A remaining portion of a photolabile group which is subjected to photoreaction acts as a non-cell-adhesive group. The irreversible photodissociation reaction can efficiently and reliably change the cell-adhesive one into the non-cell-adhesive one, enabling the enhancement of adhesion selectivity. As used herein, the cell-adhesive material includes even a material capable of firmly binding to cells through a covalent binding or the like, or a cell-binding material including an animal species-specific antibody, or the like. The term "cell-adhesive material" described in the present invention includes both of the material capable of firmly binding to cells through a covalent binding or the like and the cell-binding material capable of binding to cells, such as an antibody.

The cell-adhesive photocontrollable base material described above can be used to analyze and fractionate particular cells. As used herein, the analysis and fractionation refers to analyzing cells and fractionating them from other cells.

Examples of the non-cell-adhesive material include a biocompatible material with a phosphorylcholine group, having a structure similar to that of a cell membrane. The non-cell-adhesive material is, for example, a (meth)acrylic ester polymer with a phosphorylcholine group, represented by general formula (1) below.

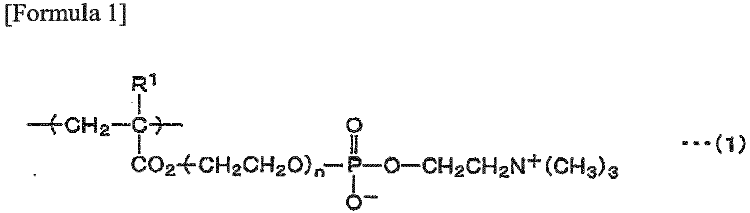

In the formula, R¹ represents hydrogen or a methyl group and n represents a number of 1 to 20.

A (meth)acrylic ester polymer represented by general formula (2) below may also be used as the non-cell-adhesive material.

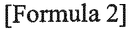

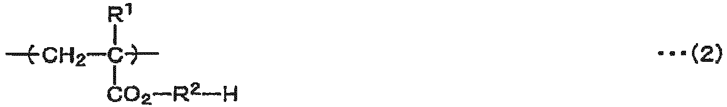

In the formula, R¹ is the same as that in the general formula (1), and R² represents 1 to 20 alkylene groups or 1 to 20 polyoxyethylene groups.

The non-cell-adhesive material may be a copolymer of (meth)acrylic ester polymers represented by the general formulas (1) and (2). In addition, an alkoxysilane represented by general formula (3) below can be used as the non-cell-adhesive material.
[Formula 3]

(R³O)₃Si-R²-H (3)

In the formula, R² is the same as that in the general formulas (1) and (2), and R³ represents hydrogen or an alkyl group.

Examples of the cell-adhesive material include a material having a cell-adhesive group in the terminal end. Examples of the cell-adhesive group include a group represented by general formula (4) below.
[Formula 4]

-X (4)

In the formula, X represents a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, or a nucleic acid group.

The cell-adhesive group X of the general formula (4) can be varied to provide a variation in adhesion to various cells. In addition, the cell-adhesive material encompasses a material in which an extracellular matrix promoting adhesion to cells or an antibody capable of binding to a surface antigen of cells and a protein or the like for the binding of the antibody thereto binds or adheres to a group represented by the general formula (4) described above. Examples of the extracellular matrix include collagens, non-collagenous glycoproteins (fibronectin, vitronectin, laminin, nidogen, teneinosine, thrombospondi, von Willebrand, osteopontin, fibrinogen, and the like), elastins, and proteoglycans. Examples of the protein capable of causing the antibody to bind include avidin/biotin, protein A, and protein G.

The photolabile group can be dissociated by reaction to light of particular wavelength. The wavelength of the photoreaction of the photolabile group should be 360 nm or more which is non-cytotoxic and a shorter wavelength than the wavelength of incident light for light microscopical observation or exciting light for fluorescent observation. This can ensure that a change in adhesion due to light for cell observation does not occur during cell observation. Examples of the photolabile group include an O-nitrobenzyl group, a hydroxyphenacyl group, and a coumarinylmethyl group; however, a material comprising a coumarinylmethyl skeleton is preferable because it is non-cytotoxic and has a longer photoreaction wavelength region and a high photoreaction efficiency. Particularly, one can be preferably used which comprises a divalent coumarinylmethyl skeleton represented by general formula (5) below as a linker.

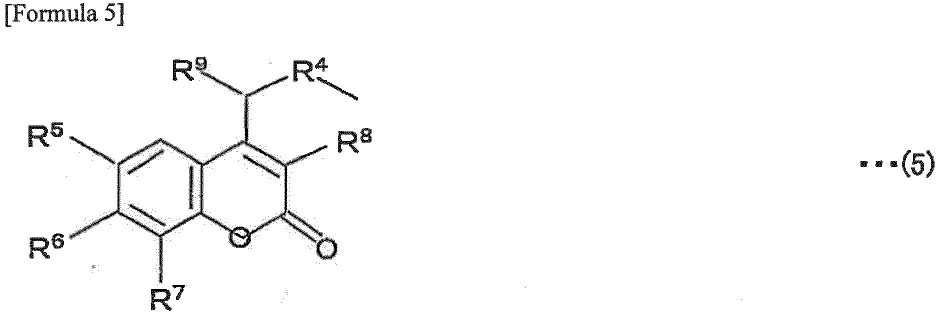

Here, in the formula, R⁴ is divalent and represents O, CO, CO₂, OCO, OCO₂, OCONH, OCONR, NHCO₂, NH, SO₃, or (OPO(OH))₁₋₃OPO₂; R⁵ represents hydrogen, a halogen group, or an alkoxy group; R⁶ represents hydrogen, a hydroxy group, an alkoxy group, or a dialkylamino group or is absent; R⁷ represents hydrogen or a halogen group or is absent; R⁸ represents hydrogen or a halogen group; R⁹ represents hydrogen or is absent; and one of two bonds formed by the divalent linker of the general formula (5) is positioned at R⁴, and the other bond is positioned at R⁶ or R⁷ on the benzene skeleton or R⁹ on the coumarinylmethyl group. The term "absent" for R⁶, R⁷, and R⁹ is used in that context.

More specifically, the efficient photoreaction of R⁴ can be achieved with less cytotoxic light of a longer wavelength by positioning two bonds formed by the divalent linker of the general formula (5) at (R⁴ and R⁶ on the benzene skeleton), (R⁴ and R⁷ on the benzene skeleton), or (R⁴ and R⁹ on the coumarinylmethyl group) and appropriately selecting the other substituents.

The photolabile group and the cell-adhesive material form a structure in which a cell-adhesive group represented by the general formula (4) directly or indirectly binds to a divalent photolabile group represented by the general formula (5) at one of the binding positions. Photodissociation occurs at a position of R⁴. For example, when binding is performed at a position of R⁶ on the benzene skeleton, the binding may be carried out via a divalent linking group R¹⁰, as represented by general formula (6) below. Alternatively, when binding is performed at a position of R⁷ on the benzene skeleton, the binding may be carried out via a divalent linking group R¹¹, as represented by general formula (7) below. Further, when binding is performed at R⁹ on the coumarinylmethyl group, the binding may be carried out via a divalent linking group R¹², as represented by general formula (8) below.

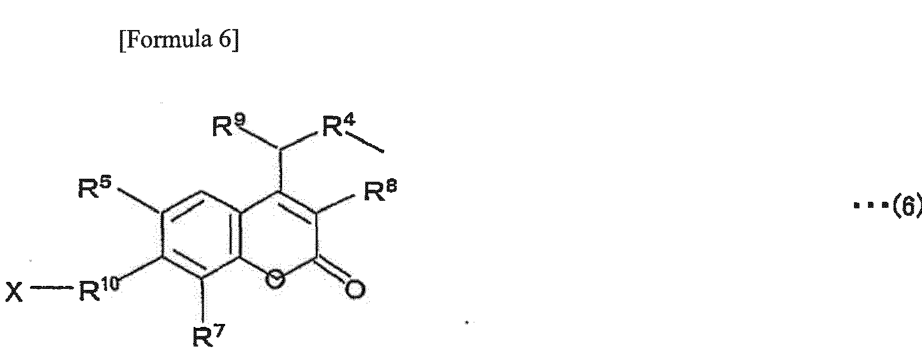

Here, R⁴, R⁵, R⁷, R⁸, and R⁹ are the same as those in the general formula (5).

As the divalent linking group R¹⁰, O(CH₂)ₘ, O(CH₂CH₂O)ₘ, OCO(CH₂)ₘ, OCOCH₂O(CH₂CH₂O)ₘ, OCH₂CO₂(CH₂CH₂O)ₘ(CH₂)_{m'}, OCH₂CONHCH₂(C₂HN₃)(CH₂)_{m'}(OCH₂CH₂)ₘ, OCH₂CONCH₃CH₂(C₂HN₃)(CH₂)_{m'}(OCH₂CH₂)ₘ, OCH₂CON(CH₂(C₂HN₃)(CH₂)_{m'}(OCH₂CH₂)ₘX) CH₂(C₂HN₃)(CH₂)_{m'}(OCH₂CH₂)ₘ (where m and m' are an integer of 0 to 20), or the like may be used.

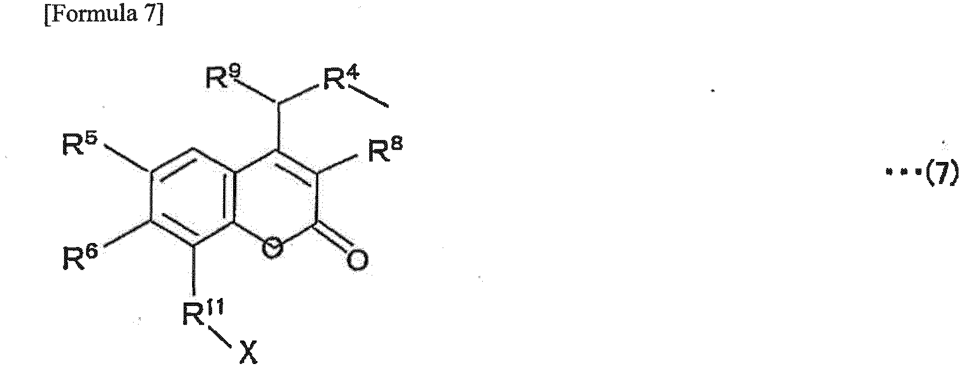

Here, R⁴, R⁵, R⁶, R⁸, and R⁹ are the same as those in the general formula (5).

As the divalent linking group R¹¹, CH₂NH(CH₂CH₂O)ₘ(CH₂)_{m'}, CH₂N((CH₂CH₂O)ₘ(CH₂)_{m'}X) (CH₂CH₂O)ₘ(CH₂)_{m'}, CH₂NHCH₂(C₂HN₃)(CH₂)_{m'}(OCH₂CH₂)ₘ, CH₂N(CH₂(C₂HN₃)(CH₂)_{m'}(OCH₂CH₂)ₘX) CH₂(C₂HN₃)(CH₂)_{m'}(OCH₂CH₂)ₘ, CH₂N(CH₃), CH₂N(CH₃)CH₂(C₂HN₃)(CH₂)_{m'}(OCH₂CH₂)ₘ (where m and m' are an integer of 0 to 20), or the like may be used.

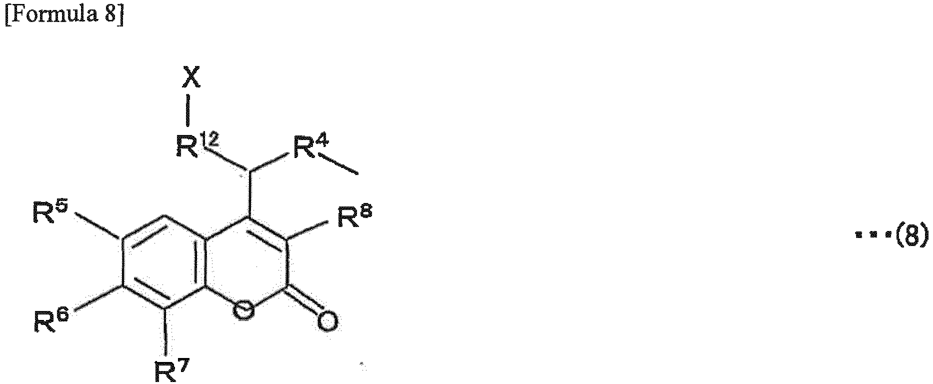

Here, R⁴, R⁵, R⁶, R⁷ and R⁸ are the same as those in the general formula (5).

As the divalent linking group R¹², CH₂OCO(CH₂CH₂)_{m'}, (CH₂)_{m"}(C₂HN₃)(CH₂)_{m'}(OCH₂CH₂)ₘ (wherein each of m, m', and m" is an integer of 0 to 20), or the like can be used.

R¹⁰, R¹¹, and R¹² have only the action of causing the photolabile group to bind to the cell-adhesive group

If the direction of the photolabile group is reversed, photodissociation can occur between the photolabile group and the cell-adhesive group. Examples thereof can include a structure represented by general formula (9) below as a structure in which the binding is made at a position of R⁴.

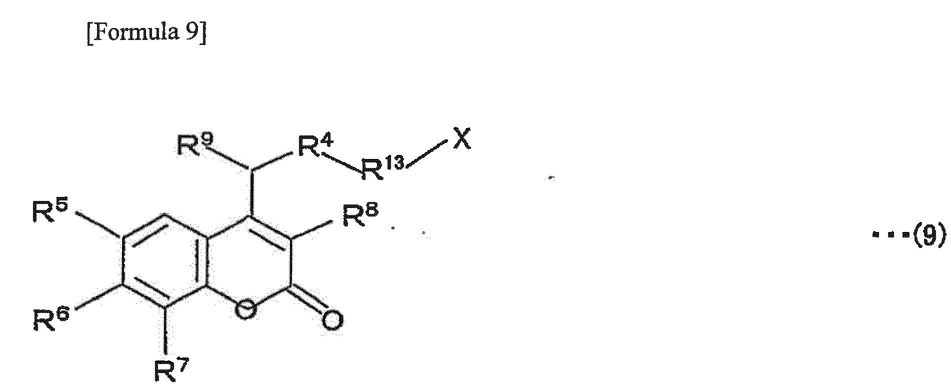

Here, R⁴, R⁵, R⁶, R⁷, R⁸, and R⁹ are the same as those in the general formula (5).

As the divalent linking group R¹³, (CH₂CH₂O)ₘ(CH₂)_{m'}, (CH₂)ₘ(C₂HN₃)(CH₂)_{m'} (wherein each of m and m' is an integer of 0 to 20), or the like can be used.

The structure in which the cell-adhesive material binds to the photolabile group as described above is directly or indirectly bound to the non-cell-adhesive material.

For example, the structure may be made in the form of a (meth)acrylic acid ester polymer represented by general formula (10), (11), (12), (13), (14), or (15) below and incorporated into the non-cell-adhesive material.

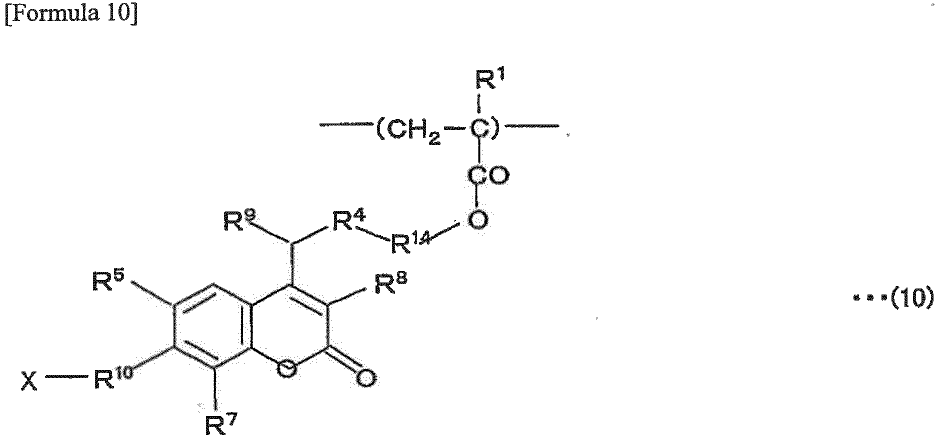

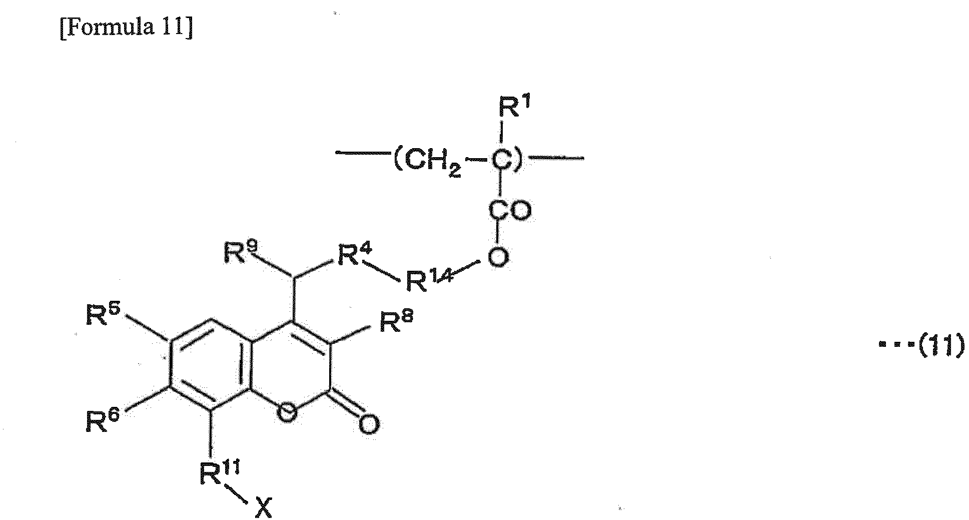

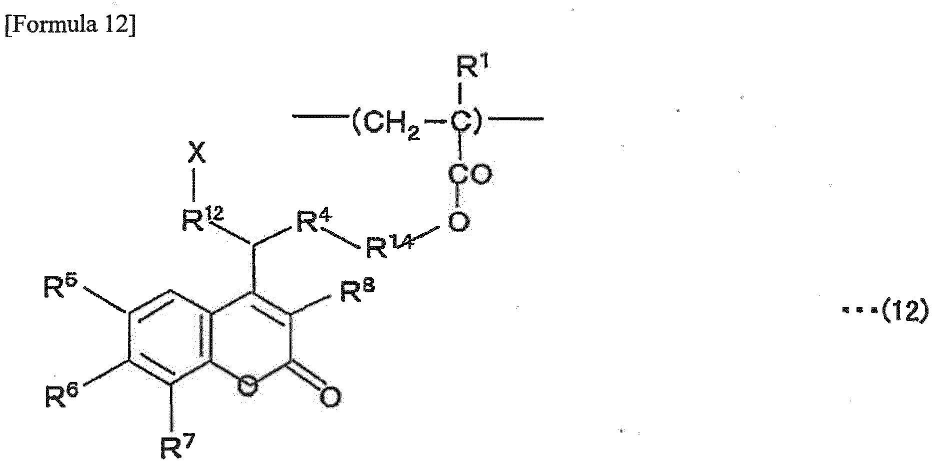

In the general formulas (10) to (12) described above, R¹ is the same as that in the general formula (1); R⁴, R⁵, R⁶, R⁷, R⁸, and R⁹ are the same as those in the general formula (5); R¹⁰ is the same as that in the general formula (6); R¹¹ is the same as that in the general formula (7); R¹² is the same as that in the general formula (8); and R¹⁴ represents an alkylene, (CH₂)_{p'}CO(OCH₂CH₂)ₚ, (CH₂)_{p"}(C₂HN₃)(CH₂)_{p'}CO(OCH₂CH₂)ₚ (wherein each of p, p', and p" is an integer of 1 to 20), or the like.

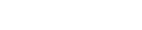

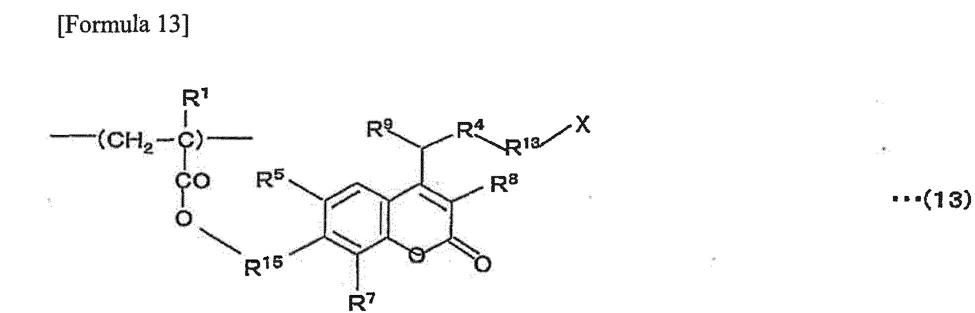

In the general formula (13), R¹ is the same as that in the general formula (1); R⁴, R⁵, R⁷, R⁸, and R⁹ are the same as those in the general formula (5); the divalent linking group R¹³ is the same as that in the general formula (9); and R¹⁵ represents (CH₂CH₂O)ₚCOCH₂O (wherein p is an integer of 1 to 20), or the like.

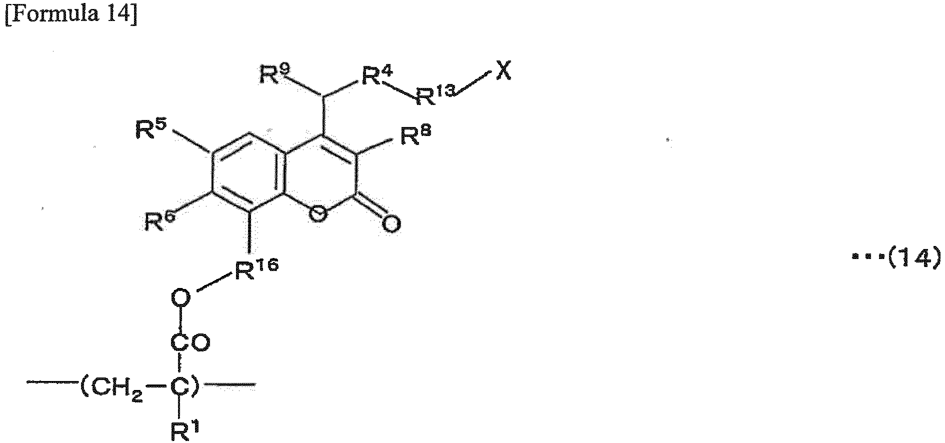

In the general formula (14), R¹ is the same as that in the general formula (1); R⁴, R⁵, R⁶, R⁸, and R⁹ are the same as those in the general formula (5); the divalent linking group R¹³ is the same as that in the general formula (9); and R¹⁶ represents (CH₂CH₂O)ₚCOCH₂NHCH₂, (CH₂CH₂O)ₚCOCH₂N(CH₃)CH₂, (CH₂CH₂O)ₚCO(CH₂)_{p'}(N₃C₂H)CH₂NHCH₂, (CH₂CH₂O)ₚCO(CH₂)_{p'}(N₃C₂H)CH₂N(CH₃)CH₂, (wherein each of p and p' is an integer of 1 to 20), or the like.

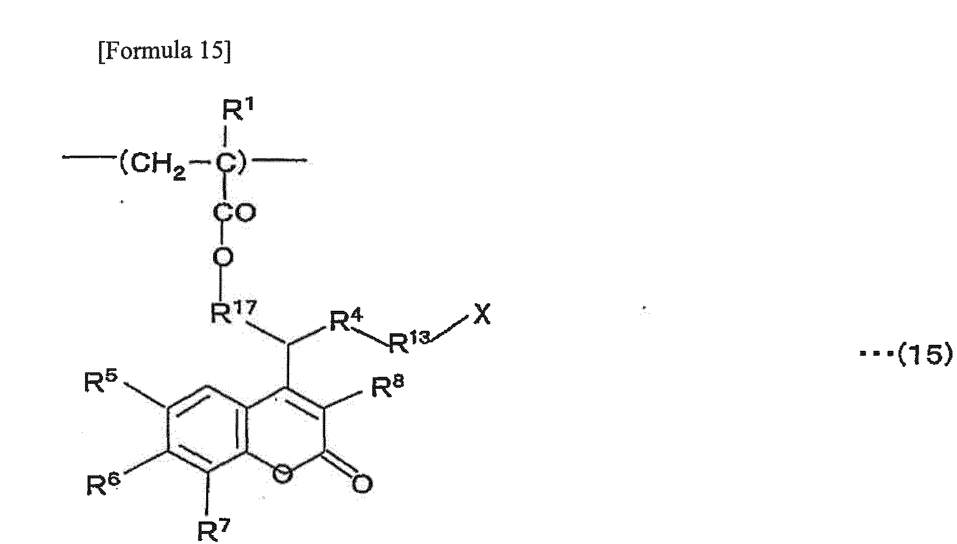

In the general formula (15), R¹ is the same as that in the general formula (1); R⁴, R⁵, R⁶, R⁷, and R⁸ are the same as those in the general formula (5); the divalent linking group R¹³ is the same as that in the general formula (9); and R¹⁷ represents (CH₂)ₚCO₂CH₂ (wherein p is an integer of 1 to 20), or the like.

Examples of the material in which the structure having the cell-adhesive group bound to the photolabile group according to the present invention binds to the non-cell-adhesive material can include a copolymer of (meth)acrylic ester represented by the general formulas (1) or (2), and (10) described above; the general formulas (1) or (2), and (11) described above; the general formulas (1) or (2), and (12) described above; the general formulas (1) or (2), and (13) described above; the general formulas (1) or (2), and (14) described above; the general formulas (1) or (2), and (15) described above; the general formulas (1), (2), and (10) described above; the general formulas (1), (2), and (11) described above; the general formulas (1), (2), and (12) described above; the general formulas (1), (2), and (13) described above; the general formulas (1), (2), and (14) described above; or the general formulas (1), (2), and (15) described above. The copolymer can be made to optionally change the ratio of the cell-adhesive material to the non-cell-adhesive material, and the changed ratio results in providing a variation in adhesion to various cells. The copolymerization of a (meth)acrylic ester containing an alkoxysilane at the side chain of each of these polymers can increase adhesion to the base material.

Although these systems take the form of copolymers, they may be used in the form of homopolymers. For example, a system having a structure represented by any of the general formulas (10) to (15) described above may be used alone. A cell-adhesive photocontrollable base material can be produces by conducting a film formation of a material in which each of these structures having cell-adhesive groups bound to photolabile groups binds to a non-cell-adhesive material on a base material.

Alternatively, the structure in which the cell-adhesive material bound to the photolabile group, represented by the general formula (6), (7), (8), or (9) may be made in the form of an alkoxysilane and directly or indirectly incorporated into the non-cell-adhesive material.

For example, a cell-adhesive photocontrollable base material to which a compound having a structure into which a cell-adhesive group X represented by general formula (19), (20) or (21) below binds can also be made by conducting a film formation in the form of an alkoxysilane represented by general formula (16), (17), or (18) below on a base material by silane coupling, and then using Huisgen reaction. Such use of Click reaction can prevent the hydrolysis of the alkoxysilane due to the cell-adhesive group X.

Here, the cell-adhesive group X represented by general formula (57) below can be formed in the terminal end by conducting a film formation of an alkoxysilane represented by general formula (16), (17), or (18) below on a base material, and then by reaction with an azide compound comprising the cell-adhesive group X.
[Formula 16]

X-R²¹-N₃ (57)

Here, in the formula, X represents a group selected from the group consisting of a carboxylic acid, a mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; and the divalent linking group R²¹ represents a group (CH₂)_{q'}(OCH₂CH₂)_{q} (wherein each of q and q' is an integer of 0 to 20).

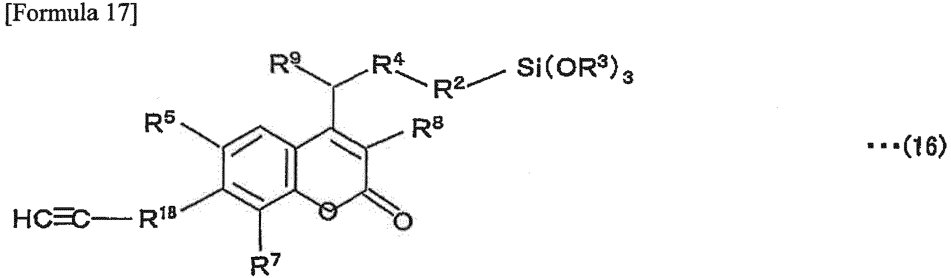

In the general formula (16), R² is the same as that in the general formula (2) or is (CH₂)_{q}CONH(CH₂)_{q'} (wherein each of q and q' is an integer of 0 to 20), or the like; R³ is the same as that in the general formula (3); R⁴, R⁵, R⁷, R⁸, and R⁹ are the same as those in the general formula (5); and the divalent linking group R¹⁸ represents CH₂NHCOCH₂O, CH₂NCH₃COCH₂O, CH₂N(CH₂CCH)COCH₂O, or the like.

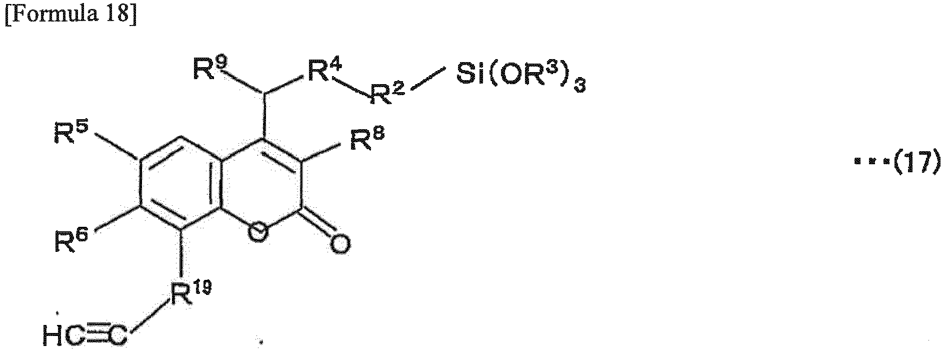

In the general formula (17), R² is the same as that in the general formula (2) or is (CH₂)_{q}CONH(CH₂)_{q'} (wherein each of q and q' is an integer of 0 to 20), or the like; R³ is the same as that in the general formula (3); R⁴, R⁵, R⁶, R⁸, and R⁹ are the same as those in the general formula (5); and the divalent linking group R¹⁹ represents CH₂NHCH₂, CH₂NCH₃CH₂, CH₂N(CH₂CCH)CH₂, or the like.

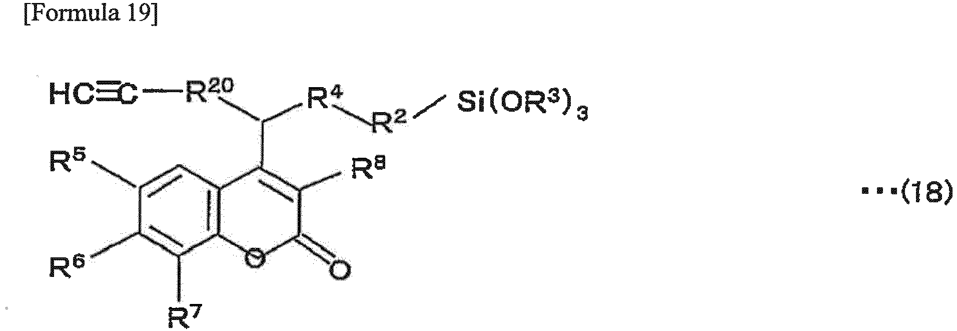

In the general formula (18), R² is the same as that in the general formula (2) or is (CH₂)_{q}CONH(CH₂)_{q'} (wherein each of q and q' is an integer of 0 to 20), or the like; R³ is the same as that in the general formula (3); R⁴, R⁵, R⁶, R⁷, and R⁸ are the same as those in the general formula (5); and the divalent linking group R²⁰ represents (CH₂)_{q} (wherein q is an integer of 0 to 20), or the like.

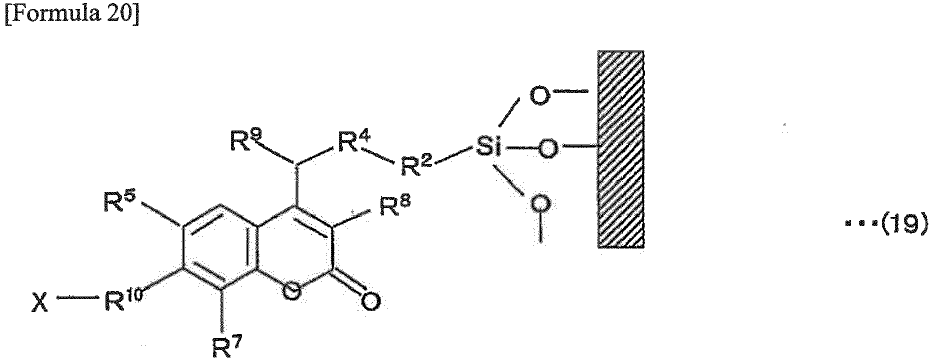

In the general formula (19), R² is the same as that in the general formula (2) or is (CH₂)_{q}CONH(CH₂)_{q'} (wherein each of q and q' is an integer of 0 to 20), or the like; R⁴, R⁵, R⁷, R⁸, and R⁹ are the same as those in the general formula (5); and the divalent linking group R¹⁰ is the same as that in the general formula (6).

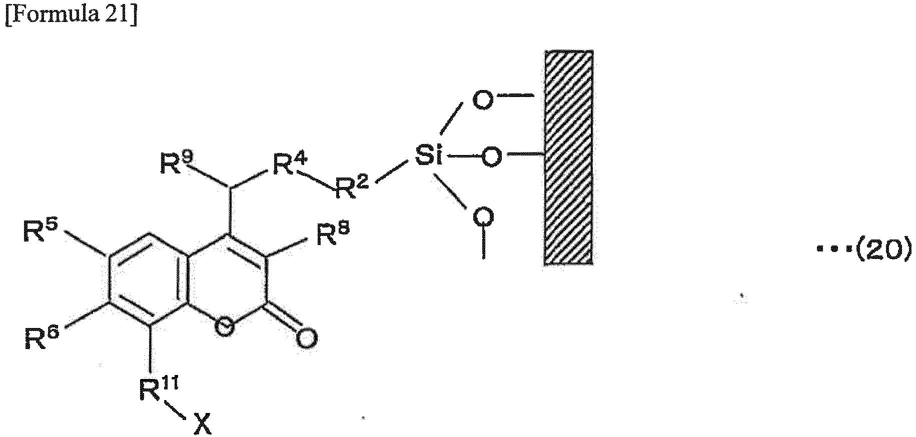

In the general formula (20), R² is the same as that in the general formula (2) or is (CH₂)_{q}CONH(CH₂)_{q'} (wherein each of q and q' is an integer of 0 to 20), or the like; R⁴, R⁵, R⁶, R⁸, and R⁹ are the same as those in the general formula (5); and the divalent linking group R¹¹ is the same as that in the general formula (7).

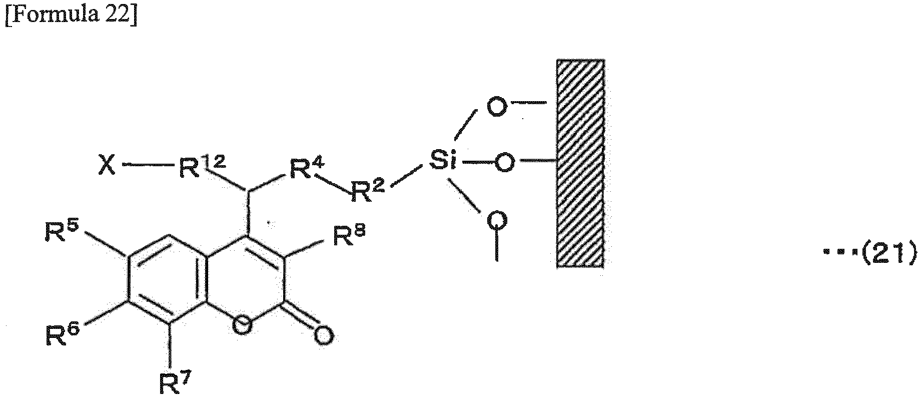

In the general formula (21), R² is the same as that in the general formula (2) or is (CH₂)_{q}CONH(CH₂)_{q'} (wherein each of q and q' is an integer of 0 to 20), or the like; R⁴, R⁵, R⁶, R⁷, and R⁸ are the same as those in the general formula (5); and the divalent linking group R¹² is the same as that in the general formula (8).

Such a form of the alkoxysilane can be effectively prepared using silane coupling reaction and Click reaction. Taking an example in which two bonds formed by the divalent coumarinylmethyl skeleton of the general formula (5) are positioned at R⁴ and R⁷, an intermediate of general formula (22) or (23) below, the azide compound of the general formula (57), and the alkoxysilane of the general formula (58) can be effectively used. 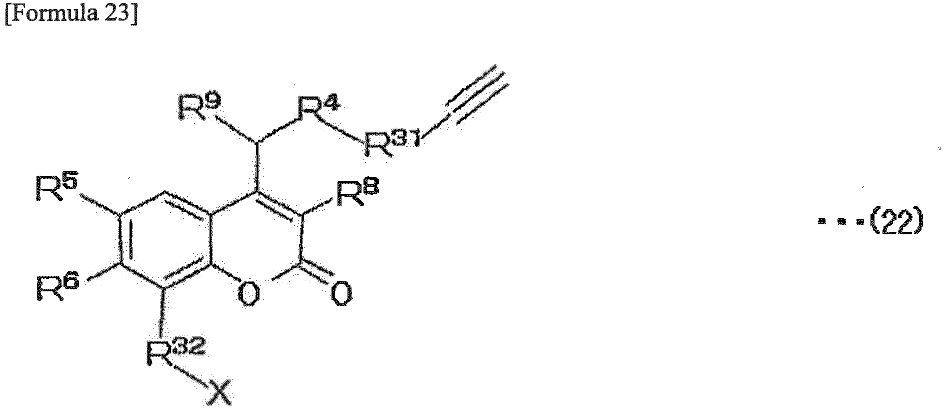 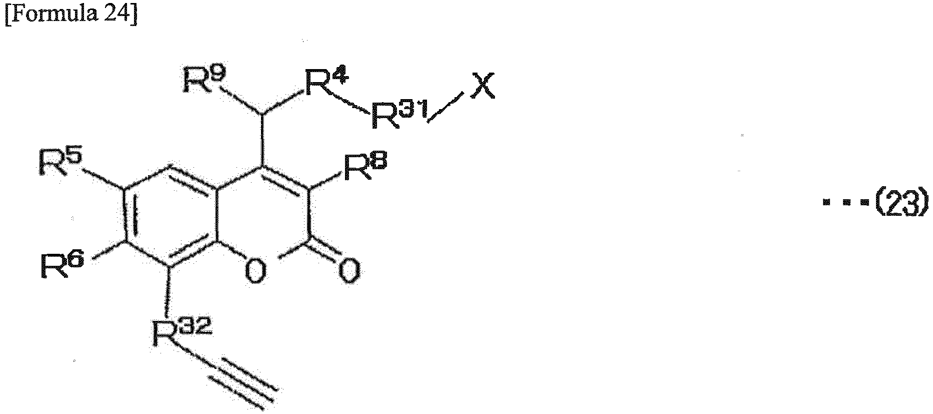 [Formula 25]

Y-R³³-Si(OR³)₃ (58)

Here, R³¹ represents (CH₂)ᵣ or the like; R³² represents CH₂NHCH₂, CH₂NCH₃CH₂, or the like; R³³ represents (CH₂)ᵣ or the like; X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; Y represents a group selected from the group consisting of an azide group, an amino group, an epoxy group, and a cyanate group; and r is an integer of 0 to 20.

For example, the combination of the general formula (22) and the general formula (58) (wherein Y is an azide group) yields general formula (24) below.

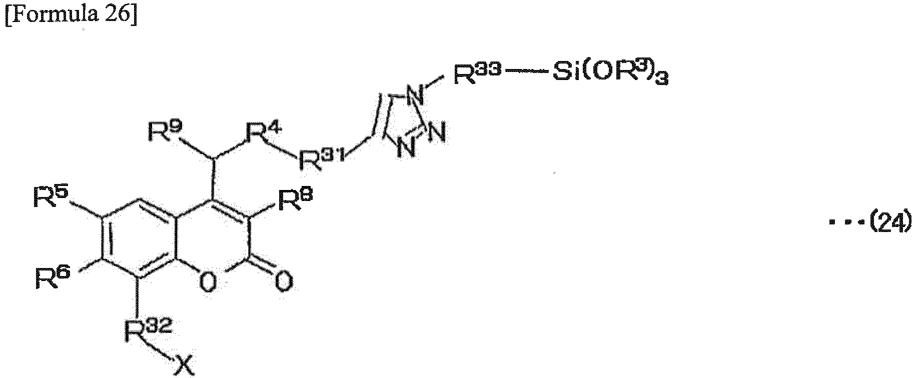

The combination of the general formula (22) (wherein X is a carboxyl group), the general formula (58) (wherein Y is an amino group), and the general formula (57) yields general formula (25) below.

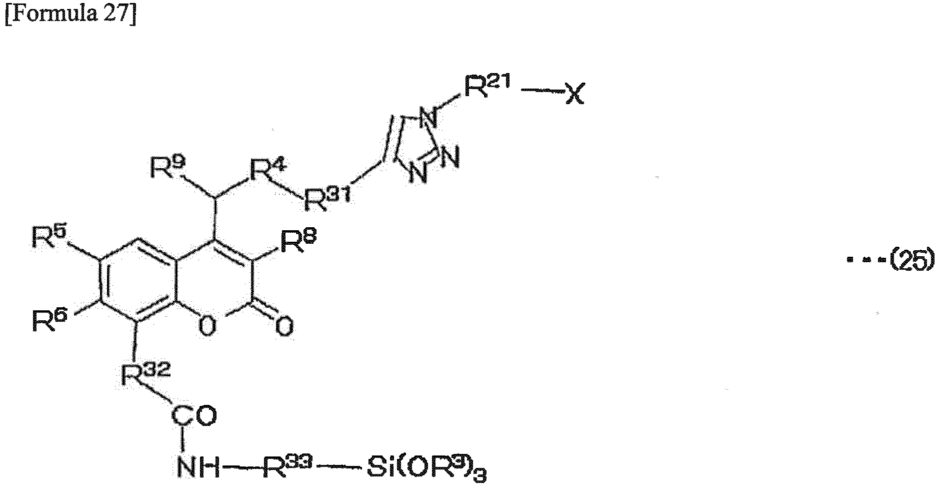

The combination of the general formula (23) and the general formula (58) (wherein Y is an azide group) yields general formula (26) below.

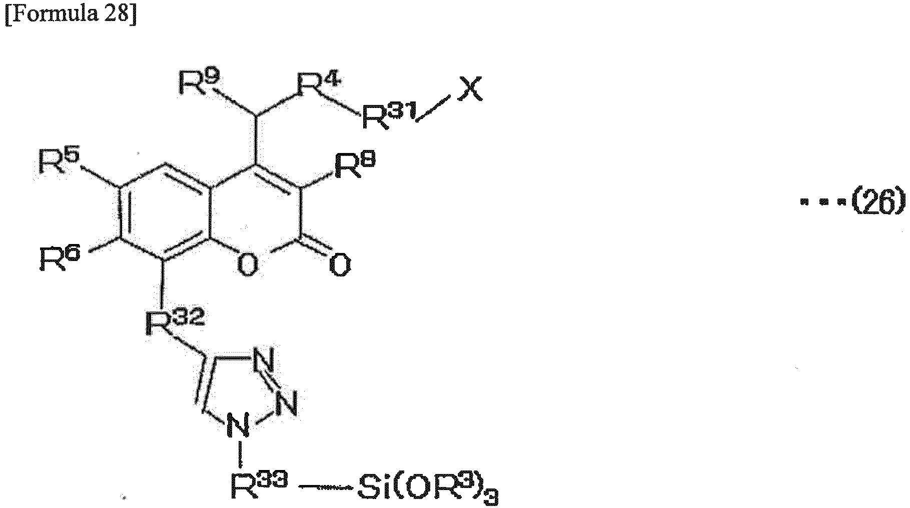

The combination of the general formula (23) (wherein X is a carboxyl group), the general formula (58) (wherein Y is an amino group), and the general formula (57) yields general formula (27) below.

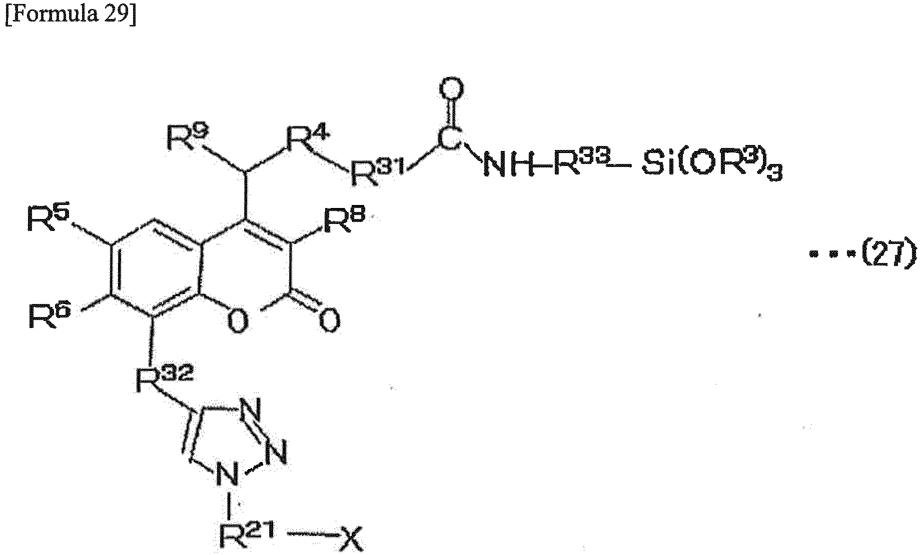

These compounds of the general formulas (24), (25), (26), and (27) may be once synthesized and then conducting a film formation of the compound on the surface of a base material or may be sequentially synthesized on the surface of the base material. When these compounds are synthesized through surface reaction, the remaining amino group or azide group can be made non-cell-adhesive using PEG-carboxylic acid, PEG-alkyne, or the like.

These cell-adhesive materials represented by X again include a material in which an extracellular matrix promoting adhesion to cells or an antibody capable of binding to a surface antigen of cells and a protein or the like to bind the antibody binds or adheres thereto.

The base material for the film formation of the cell-adhesive photocontrollable materials thereon may be a transparent plastic culture vessel or the like; however, a transparent glass culture vessel may be preferably used in view of optical performance and durability.

The method for analyzing and fractionating cells using the cell-adhesive photocontrollable base material will now be described in detail based on figures.

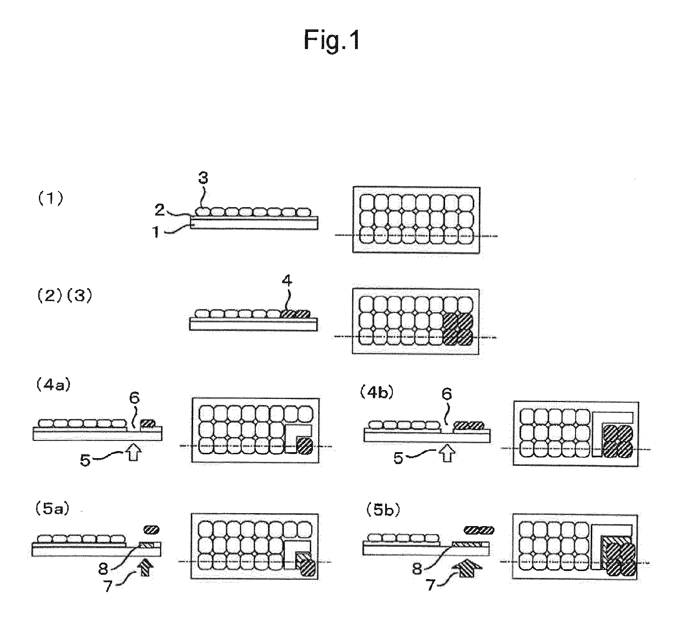

Figure 1 illustrates one aspect of the method for analyzing and fractionating cells using the cell-adhesive photocontrollable base material of the present invention. The method consists of steps (1), (2), (3), (4a), and (5a) or consists of steps (1), (2), (3), (4b), and (5b). In Figure 1, each step is illustrated in the set of right and left figures, and the left figure is a cross section of the portion indicated by a dashed line in the right figure. The description of the steps is as follows. (1) Cells 3 are seeded, adhered and cultured on a cell-adhesive photocontrollable material 2 formed as a film on a glass culture vessel (transparent base material) 1. (2) Cell images are detected by microscopic observation (including the observation of transmission images, phase contrast images, differential interference images, and the like), fluorescent observation, scattered light observation, Raman light observation, or the like; a characteristic amount of cells are extracted; and then (3) desired cells are identified and the positional information of the cells is obtained. The desired cells include necessary cells to be analyzed and fractionated and unnecessary cells. Here, cells are labeled with a fluorescent marker or the like; however, the fluorescent marker labeling or the like may be performed before or after culture. (4a) In the example of this aspect, the cells 4 shown in (3) are unnecessary cells, and the other cells 3 are necessary cells. The periphery of the side of the cell 4 and a cell-adhesive photocontrollable material 6 in the boundary between the cells 3 and the cells 4 are subjected to second light irradiation 5 for cutting the portion, indicated by a rectangular shape in (4a), having the cell-adhesive photocontrollable material 6 and the cells 4. Laser light can be preferably used for the second light irradiation 5 here. (5a) When the area on the base material in which the cells 4 are present is wide, first light irradiation 7 is performed on the region 8 in which the cells 4 are present to change the cell-adhesive photocontrollable material into a non-cell-adhesive one, and the remaining cells 4 are detached from the glass culture vessel 1 and recovered together with the culture solution. When the area on the base material in which the cells 4 are present is narrow, the second light irradiation 5 may be performed on all cells 4 and a cell-adhesive photocontrollable material 8 for cutting/destruction. Thereafter, the culture is continued, and the cells 3 left on the base material may continue to be cultured while sequentially removing unnecessary cells 4 for purification.

On the other hand, a step (4b) in Figure 1 is a step when the cells 4 are desired to be fractionated/isolated for analysis (when the cells 4 are necessary). The periphery of the side of the cells 3 in the boundary between the cell 3 and the cell 4 and the cell-adhesive photocontrollable material 6 are subjected to the second light irradiation 5 for cutting the cell 3 and the cell-adhesive photocontrollable material 6.

Laser light can be preferably used for the second light irradiation 5. Thereafter, (5b) the region 8 on the base material in which the cells 4 are present is subjected to the first light irradiation 7 to change the cell-adhesive photocontrollable material into a non-cell-adhesive one, and the cells 4 are detached from the glass culture vessel 1 and recovered together with the culture solution. The recovered fractionated cells can be analyzed.

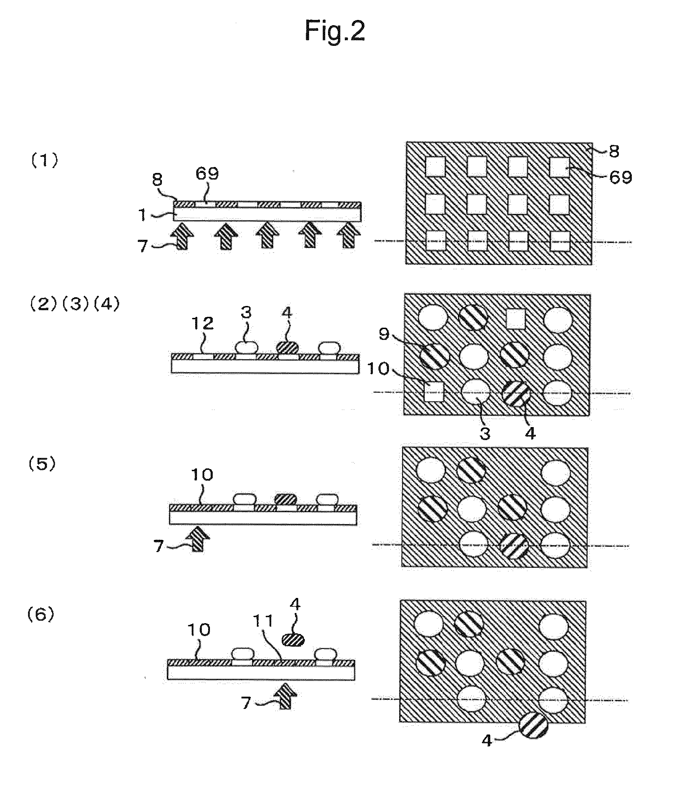

Figure 2 illustrates another aspect of the method for analyzing and fractionating cells using the cell-adhesive photocontrollable base material of the present invention. The method consists of steps (1), (2), (3), (4), (5), and (6). In Figure 2, each step is illustrated in the set of right and left figures, and the left figure is a cross section of the portion indicated by a dashed line in the right figure. (1) The cell-adhesive photocontrollable material formed as a film on a glass culture vessel 1 is subjected to first light irradiation 7 as shown in the figure to provide cell-adhesive regions 69 and a non-cell-adhesive region 8. The cell-adhesive regions 69 and the non-cell-adhesive region 8 can be set together to any pattern by changing the irradiation pattern of light. In the example shown in Figure 2, the cell-adhesive regions 69 were arranged in a lattice form as areas of single cells. In other words, the first light irradiation 7 was performed so that the cell-adhesive regions 69 were arranged in a lattice form. Here, addresses are preferably allocated so that the cell-adhesive regions can be identified.

(2) An already cultured cell mass is then separated into individual cells by treatment with trypsin or the like and seeded and adhered on the base material. (3) Cell images are detected by microscopic observation (including the observation of transmission images, phase contrast images, differential interference images, and the like), fluorescent observation, scattered light observation, Raman light observation, or the like; a characteristic amount of cells are extracted; and then (4) desired cells are identified and the positional information of the cells is obtained. The desired cells include necessary cells to be analyzed and fractionated and unnecessary cells. Here, cells are labeled with a fluorescent marker or the like; however, the fluorescent marker labeling or the like may be performed before or after seeding. In the example shown in Figure 2, it is assumed that in addition to the cells 3, other cells (for example, cells 4 and cells 9) are present. (5) When cells do not adhere to all addresses (the cell-adhesive regions 69), the address regions to which no cells adhere are subjected to the first light irradiation 7 to make them non-cell-adhesive (the region of reference symbol 10 shown in the figure). (6) Then, a region 11 of a desired cell, a cell 4 here, is subjected to the first light irradiation 7, and the cell 4 is detached from the glass culture vessel 1 and recovered together with the culture solution. The step (6) can be sequentially repeated to further fractionate and isolate the cells 3 and 9.

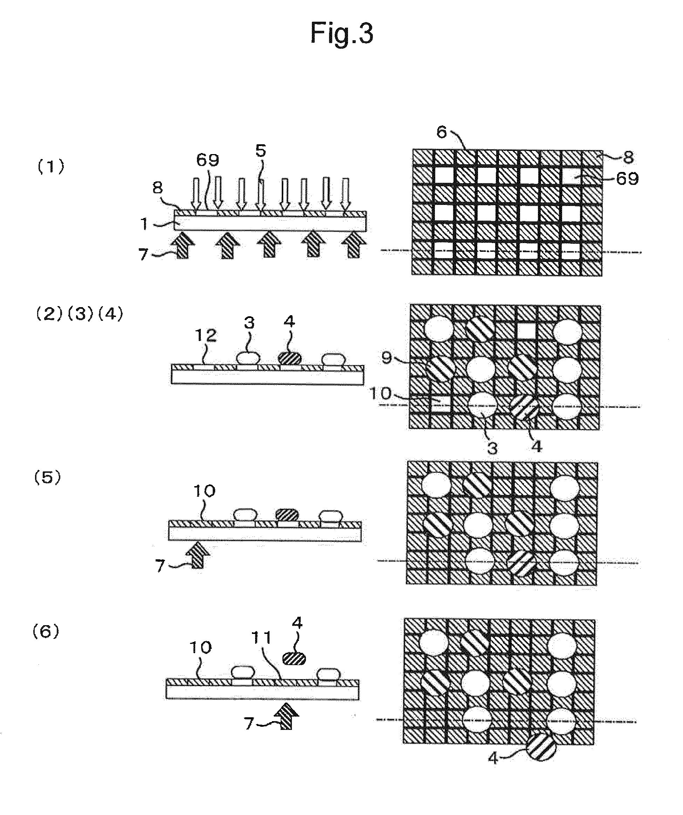

Figure 3 illustrates still another aspect of the method for analyzing and fractionating cells using the cell-adhesive photocontrollable base material of the present invention. The method consists of steps (1), (2), (3), (4), (5), and (6). In Figure 3, each step is illustrated in the set of right and left figures, and the left figure is a cross section of the portion indicated by a dashed line in the right figure. (1) A cell-adhesive photocontrollable material formed as a film on a glass culture vessel 1 is subjected to second light irradiation (for example, irradiation with laser light) 5 in columns and rows at predetermined intervals to cut the adjacent cell-adhesive photocontrollable material (the cutting-plane line is indicated by symbol 6 in Figure 3). Predetermined positions are also subjected to first light irradiation 7 to provide cell-adhesive regions 69 and non-cell-adhesive regions 8. Here, addresses are preferably allocated so that the cell-adhesive regions can be identified.

The cell-adhesive regions 69 and the non-cell-adhesive regions 8 can be set together to any pattern by changing the irradiation pattern of light; however, here, the cell-adhesive regions 69 were arranged in a lattice form as areas of single cells. (2) An already cultured cell mass is then separated into individual cells by treatment with trypsin or the like and seeded and adhered on the base material. (3) Cell images are detected by microscopic observation (including the observation of transmission images, phase contrast images, differential interference images, and the like), fluorescent observation, scattered light observation, Raman light observation, or the like; a characteristic amount of cells are extracted; and then (4) desired cells are identified and the positional information of the cells is obtained. The desired cells include necessary cells to be analyzed and fractionated and unnecessary cells. Here, cells are labeled with a fluorescent marker or the like; however, the fluorescent marker labeling or the like may be performed before or after seeding. In the example shown in Figure 3, it is assumed that in addition to cells 3, cells 4 and cells 9 are present.

(5) When cells do not adhere to all cell-adhesive regions (addresses) 69, the address 10 region to which no cell adheres is subjected to the first light irradiation 7 to make it non-cell-adhesive. (6) Then, a region 11 in which a desired cell, a cell 4 here, is present is subjected to the first light irradiation 7, and the cell 4 is detached from the glass culture vessel 1 and recovered together with the culture solution. The step (6) can be sequentially repeated to further fractionate and isolate the cells 3 and 9. The difference with Figure 2 lies in that whereas in the method shown in Figure 2, the first light irradiation 7 by itself does not cut the cell-adhesive material into the regions when a fibrous or membranous material such as an extracellular matrix or feeder cells is present on the upper layer of the cell-adhesive material, in the method shown in Figure 3, the second light irradiation 5 is performed to cut it between the regions.

The device for performing the method for analyzing and fractionating cells using the cell-adhesive photocontrollable base material (for example, a cell-adhesive photocontrollable material formed as a film on a transparent base material) of the present invention at least comprises (1), (2), (3), (4), (6), and (7) below:

(1) the cell-adhesive photocontrollable base material;
(2) a stage on which the base material is placed;
(3) an optical detection means for obtaining cell images;
(4) a means for obtaining positional information from cell images;
(5) a second light irradiation means for cutting or destructing areas among cells and a cell-adhesive photocontrollable material;
(6) a first light irradiation means for subjecting the cell-adhesive photocontrollable material on the base material to photoreaction to make it non-cell-adhesive; and
(7) a means for controlling the motion of each means.

The optical detection means for obtaining cell images described in (3) above may use a well-known optical system. In obtaining cell images, light is irradiated which has a wavelength not affecting the photolabile group of the cell-adhesive photocontrollable material. For example, using a lamp or LED providing broad emission spectra as a light source, light is irradiated through a short-wavelength cut filter for at least removing light of a wavelength not more than the photoreaction wavelength to detect transmitted light, reflected light, or the like using a 2-dimensional sensor such as CCD. In the case of fluorescent images from cells, light of the absorption wavelength range of a desired fluorochrome, having the range of a wavelength longer than the photoreaction wavelength is irradiated as an exciting light by dispersion with a bandpass interference filter or the like. Laser light of the above wavelength range may also be used. Fluorescence is detected with a 2-dimensional sensor such as CCD through a wavelength filter such as an exciting light cut filter or a fluorescence wavelength transmission filter (a bandpass interference filter or the like). If the exciting light is sharply restricted and a 2-dimensional-scanning mode is adopted, the fluorescent image can also be measured using a photomultiplier tube. Measurement can be made by switching a plurality of wavelength filters to provide fluorescent images of a plurality of fluorescence wavelengths, enabling application to a plurality of fluorophores. Passage through a dispersive element such as a prism or a diffracting grating and detection with a line sensor or the like also enable a finer wavelength spectrum image to be obtained.

The second irradiation means of (5) above can be performed by using an infrared laser or an ultraviolet laser as the light source for laser scanning based on the positional information obtained by the means of (4) above. The laser scanning uses an XY deflector and light irradiation is performed on desired positions. Light pattern irradiation can also be performed by one operation through a photomask reflecting the positional information obtained by the means of (4) above. In that case, an optical system for condensing laser light on the base material through a spatial light modulation device to avoid preparing a fixed photomask each time the experiment is performed is preferable as a pattern generator. The spatial light modulation device may be a reflex or transmissive spatial light modulation device. The reflex spatial light modulation device may be a digital mirror device, and the transmissive spatial light modulation device may be a liquid crystal spatial light modulation device. Here, the laser wavelength usable in the digital mirror device or the liquid crystal spatial light modulation device is mainly in the range from visible light to near-infrared light. Thus, a near infrared laser, which can be strongly absorbed by water, can be used as a laser light source. When the wavelength is set within the ultraviolet region, visible to near infrared laser light may be passed through a spatial light modulation device and then passed through a wavelength conversion device such as a nonlinear crystal or a ferroelectric crystal to use a second harmonic or a third harmonic, which has a 1/2 or 1/3 wavelength, respectively.

The first light irradiation means of (6) above uses a wavelength of the photoreaction of the cell-adhesive photocontrollable material as a light source. A lamp, LED, or the like providing a broad emission spectrum is used, and light of a wavelength of 360 nm or less or even light of a wavelength of not less than the fluorescence excitation wavelength is cut by a wavelength filter; or a laser of a photoreaction wavelength can be used. It is also possible that light scanning is performed using an XY deflector based on the positional information obtained by the means of (4) above for light irradiation on desired positions, or light pattern irradiation is performed by one operation through a photomask reflecting the positional information obtained by the means of (4) above. In that case, an optical system for condensing light on the base material through a spatial light modulation device to avoid preparing a fixed photomask each time the experiment is performed is preferable as a pattern generator. The spatial light modulation device may be a reflex or transmissive spatial light modulation device. The reflex spatial light modulation device may be a digital mirror device, and the transmissive spatial light modulation device may be a liquid crystal spatial light modulation device.

The optical systems of (3), (5), and (6) above preferably use parts as common as possible.

Possible usage of the present invention is broadly classified into (1) the case where a cell population comprising desired target cells is captured into a vessel and analyzed, followed by fractionation, and (2) the case where a cell population other than the desired target is captured into a vessel and analyzed while a cell population comprising target cells is directly recovered without being captured. As used herein, the vessel refers to a vessel such as a dish or a microplate capable of aseptically accommodating cells and preferably has a cell-adhesive photocontrollable base material on the inside bottom surface. Cells derived from different animal species may be cocultured in the field of stem cell research or regenerative medicine. Examples thereof include an example of coculturing human iPS cells with mouse fibroblasts as feeder cells and an example of coculturing human epidermal stem cells also with mouse fibroblasts as feeder cells. In these examples, human iPS or epidermal stem cells or human cells derived therefrom may have to be fractionated from mouse cells. When human-derived cells and mouse-derived cells are selected as target cells and cells other than the target, respectively, the human-derived cell group which is target cells may be bound to the cell-adhesive photocontrollable base material, for example, in the case of (1) described above, or the mouse-derived cell group which is cells other than the target may be bound to the cell-adhesive photocontrollable base material in the case of (2) described above. In either case, it is convenient to provide a cell-adhesive photocontrollable base material (hereinafter, abbreviated to a base material) capable of binding to human (or mouse)-derived cells.

For this purpose, a base material capable of binding to cells derived from a particular animal species can be provided by using a cell-adhesive material in which, for example, an antibody against an antigen specific for the animal species is bound to the cell-adhesive group X. However, special properties are necessary for selecting this antibody. For the purpose described above, it is required that, for example, not only human iPS cells but various types of human cells obtained by differentiation induction therefrom should be completely captured for analysis. Antibodies usually have exceedingly high specific reactivity (specificity) for particular antigens. That is, only minor changes in the expression levels or structures of the antigens as a result of slight changes in the properties of cells have a sharp impact on the reactivity of the antibodies against the cells. Thus, some cells might be unable to bind to the antibodies even if all cells are derived from the same animal species. For the purpose described above, a typical antibody having exceedingly high specificity is rather inconvenient, and instead, extensive (in that context, low specific) properties against antigens common to the desired animal species are convenient. It is further required that the antibody for the purpose described above should have exceedingly low reactivity (in that context, high specificity) against similar antigens of unintended animal species. In the present invention, a major histocompatibility complex (MHC) was studied as a candidate of such an animal species-specific cell surface antigen. MHC is also called a major histocompatible antigen or a human leukocyte antigen (HLA) in humans. A glycoprotein encoded thereby is called an MHC antigen or an MHC molecule. Of MHC class molecules, MHC class I molecules reside on the surface of almost all of nucleated cells. Particularly, class Ia molecules are classified into three types, HLA-A, B, and C, in humans and into three types, H-2K, 2D, and 2L, in mice. The MHC class I molecules function as markers for discriminating between self and non-self. The HLA-A, B, and C molecules correspond to the nonpolymorphic determinants of the human HLA class I molecules and are reportedly expressed on the surface of all human nucleated cells. Clone W6/32 and clone B9.12.1 are commercially available as anti-human HLA-A/B/C antibodies. Clone M1/42, which is an anti-mouse H-2 antibody, reacts with many mouse MHC haplotypes including H-2 molecules a, b, d, j, k, s, and u. Thus, the antibodies described above are considered to bind to all human cells or many mouse cells.

For applicability to the purpose of the present invention, these antibodies need not only to react with target cells but to have high reactivity against the target and low cross reactivity against unintended cells. As a result of experimental studies, the clone W6/32 and the clone B9.12.1, among the antibodies described above, were shown to be not only highly reactive against a human cell line HCT116 but exceedingly low reactive against a mouse cell line NIH/3T3, demonstrating that these antibodies can be preferably used for the purpose of the present invention. On the other hand, the clone M1/42 was shown to have exceedingly low cross reactivity against human HCT116 cells; however, there arose a problem of its less-than-satisfactory reactivity against mouse NIH/3T3 cells. In this way, whether a particular antibody can be used for the purpose of the present invention cannot be determined on the basis of public information (such as data sheets attached to the antibody) alone and can be revealed only after experimental evaluation of reactivity against cells. This means that the performance of an antibody is difficult to predict under the present circumstance.

The problem associated with the reactivity of the clone M1/42 described above was studied as follows: for the slightly limited purpose, an antibody that can be used for capturing mouse fibroblasts was searched for in a system of coculturing human iPS cells or the like with mouse fibroblasts as feeder cells. CD9, CD10, CD29, CD44, CD47, CD49b, CD54, CD81, CD90, CD91, CD121a, CD248, FSP1, SFA, and the like are known as surface antigens of fibroblasts. As a result of particularly confirming the reactivity of an anti-mouse CD9 antibody clone MZ3, this clone was shown to be exceedingly highly reactive against mouse NIH/3T3 cells and exceedingly low reactive with human HCT116 cells, demonstrating that this clone can be preferably used for the purpose of the present invention. The antibody was bound to a non-cell-adhesive material via a photolabile group by a method described later.

Meanwhile, a commercially available anti-human EpCAM antibody (clone name: Ber-EP4), an anti-human EpCAM antibody (clone name: hrk29, sold by Cosmo Bio Co., Ltd.) developed by the present inventors, and an anti-human CD9 antibody (provisional name: C1D3-G4D9) were also shown to be highly reactive with many human cells (HeLa, HCT116, and CaR-1). There is the possibility that these antibodies can also be preferably used as highly versatile antibodies for human cells.

In the case of the usage (1) mentioned above, that is, the case where a cell population comprising target cells is captured into a vessel by binding, one embodiment of the present invention can provide a cell-adhesive photocontrollable base material configured to capture and bind to human-derived cells and not to bind to cells derived from mice or the like by using the anti-human HLA-A/B/C antibody selected as described above. This embodiment has the advantage that only necessary human cells can be efficiently captured for the purpose of conducting detailed analysis on the human cells and unnecessary animal-derived cells can be efficiently removed beforehand. A cell-adhesive photocontrollable base material configured to bind to mouse-derived cells and not to bind to cells derived from humans or the like can also be provided by similarly using the anti-mouse H-2 antibody.

In the case of the usage (2) mentioned above, that is, the case where a cell population other than the target is removed by binding while a cell population comprising target cells is directly recovered without binding, a vessel to which mouse-derived cells bind and cells derived from humans do not bind or the like can be provided by the application of the latter configuration described above. An alternative embodiment can provide a vessel to which mouse feeder cells bind and human-derived cells do not bind using the anti-mouse CD9 antibody. Use of these two vessels has the advantage that unnecessary animal-derived cells can be efficiently removed beforehand for the purpose of conducting detailed analysis on the human cells and only necessary human cells can be efficiently recovered.

It may be preferable to capture all of plural types of cocultured cells. In this case, antibodies against these plural types of cells can be mixed for use. For example, the anti-human HLA-A/B/C antibody and the anti-mouse H-2 antibody, or the anti-human HLA-A/B/C antibody and the anti-mouse CD9 antibody can be mixed for use. Three or more antibodies against possible cells may be used in combination, as a matter of course. These embodiments have the advantage that every type of cells can be completely captured for the purpose of analyzing the cells after the capture and, if necessary, fractionating them by light irradiation.

A method suitable for the purpose of capturing all cells can be configured as follows: a material that exhibits exceedingly strong binding to cells, in other words, an association constant as very large as, for example, 10⁷ M or larger, and substantially irreversibly binds to cells (hereinafter, a cell-binding material) is used as a cell-adhesive material. Examples of the cell-binding material include a material having a cell-binding group in the terminal end. One example of the cell-binding group includes a material comprising a group of the above general formula (4) wherein X is a covalently-bound functional group. As used herein, the covalent-bound functional group refers to a functional group capable of performing covalent binding reaction with a functional group which is a component of a compound present on cell surface (hereinafter, referred to as a cell surface functional group). Another example of the cell-binding group includes a material comprising a group of the above general formula (4) wherein X is bound to an antibody against a surface antigen of cells.

Main differences between the terminal functional group according to conventional example 2 (JP Patent Publication (Kokai) No. 2006-6214 A) or the cell adhesion promoting substance adsorbed onto the terminal functional group and the cell-binding group according to the present invention are as follows: in the case of the terminal functional group or the cell adhesion promoting substance, cells adhere to the terminal functional group or the cell adhesion promoting substance by use of the functions of cell adhesion factors or the like carried on the surface thereof. This means that the adhesion is driven by the cells. On the other hand, in the case of the cell-binding group, the cell-binding group rather causes covalent binding or antigen-antibody reaction with a cell surface compound by use of the covalently binding functional group or the antibody. This means that the adhesion is driven by the cell-binding group. In other words, the terminal functional group or the cell adhesion promoting substance according to conventional example 2 has passive action in cell adhesion, whereas the cell-binding group of the present application has active action in cell adhesion. Use of the terminal functional group or the cell adhesion promoting substance according to conventional example 2 presents a problem of generally weak adhesive force as well as a problem of time-consuming adhesion due to a time long enough for cells to produce or secrete factors for exerting adhesive functions or to exhibit functions such as the molecular arrangement of the cell surface. By contrast, use of the cell-binding group according to the present application has the advantage that it not only produces high adhesive force but requires only a short time for adhesion because of being independent of the exhibition of functions by cells.

Specific examples of the covalently binding functional group will be shown below. For example, various types of cell surface proteins (cytoplasmic membrane protein) are present on cell surface. Proteins have a large number of amino groups. Thus, amino groups are widely present over the cell surface. Typical examples of the covalently binding functional group against these amino groups on the cell surface include active ester groups of carboxylic acids, specifically, N-hydroxysuccinimide ester and N-hydroxysulfosuccinimide ester. As another example of the covalently binding functional group against the amino groups, an activated carboxylic acid such as a carboxylic acid halide, a carboxylic acid anhydride, or a carboxylic acid azide may be used. Alternatively, a functional group with a largely strained 3-membered ring, such as an epoxide group, an aziridine group, an aziridinium group, or an episulfonium group, may be used. Further, a p-toluenesulfonyl group or the like may be used. Also, various types of sugar chains are also present on the cell surface. Saccharides constituting the sugar chains can take an isomeric structure having an aldehyde group or a ketone group. Thus, aldehyde groups or ketone groups are also widely present over the cell surface. Examples of the covalently binding functional group against these aldehyde groups or ketone groups on the cell surface include 1,3-diol, hydrazine, and hydroxylamine ether. The p-toluenesulfonyl group can also covalently bind to various types of hydroxy group-containing compounds, such as proteins or saccharides, on the cell surface through reaction with a hydroxy group or the like.

A cell-adhesive photocontrollable base material according to an embodiment in which a cell-binding material that exhibits exceedingly strong binding to cells as described above is used as the cell-adhesive material has the advantage that it can exceedingly strongly bind to a cell surface functional group via this functional group. Particularly, a cell-adhesive photocontrollable base material according to an embodiment in which a cell-binding material having a covalently binding functional group against a cell surface functional group, in the terminal end is used as the cell-adhesive material has the advantage that it can exceedingly strongly bind to the cell surface functional group through covalent binding via this functional group. Since the base materials according to these embodiments form strong covalent binding with cells, the cells are immobilized on the surface of the base materials merely by contact with the base materials, eliminating the need of waiting for the exhibition of adhesive functions by the cells. Thus, these cell-adhesive photocontrollable base materials also have the advantage that a time required for cell adhesion can be shortened.

When a cell-binding material is used as the cell-adhesive material and has a covalently binding functional group against a cell surface amino group, in the terminal end, this material can bind not only to the cells but to an antibody through covalent binding. This is because antibodies also have a large number of amino groups. Thus, a cell-adhesive photocontrollable material having a cell-adhesive material having a covalently binding functional group against an amino group, in the terminal end can first be prepared and then reacted by the addition of an antibody thereto to prepare the cell-adhesive photocontrollable material bound to the antibody.

In a related move, this material can bind not only to the cells or the antibody but to a protein such as an extracellular matrix through covalent binding. This is because extracellular matrix proteins also have a large number of amino groups. Thus, a cell-adhesive photocontrollable material having a cell-adhesive material having a covalently binding functional group against an amino group, in the terminal end can first be prepared and then reacted by the addition of an extracellular matrix thereto to prepare the cell-adhesive photocontrollable material bound to the extracellular matrix. Likewise, a protein, such as avidin, streptavidin, protein A, or protein G, which is routinely used for the binding of an antibody, can also bind to the material through covalent binding.

In the case of using a cell-binding material having a covalently binding functional group in the terminal end as the cell-adhesive material, a highly stable cell-adhesive photocontrollable base material can be achieved by properly selecting the functional group. For example, N-hydroxysuccinimide ester, an epoxide group, or a p-toluenesulfonyl group is known as a highly stable covalently binding functional group.

In the case of using a cell-adhesive material in which an antibody is bound to the cell-adhesive group X, the antibody can be bound to a cell-adhesive photocontrollable base material in the stage of producing the cell-adhesive photocontrollable base material formed by film formation of a cell-adhesive photocontrollable material on a base material, because a general antibody is highly stable and can stably maintain its binding performance. Likewise, an extracellular matrix may be bound thereto in this stage. In this case, the cell-adhesive photocontrollable base material can be filled with a buffer solution containing a preservative or refrigerated and thereby preserved for a longer effective period.

The binding of the covalently binding functional group or the antibody in advance in the stage of producing the cell-adhesive photocontrollable base material enables a user to use the cell-adhesive photocontrollable base material as it is. This embodiment has the advantage that a user can save time and labor for preparing the functional group or binding the antibody.

The binding of the covalently binding functional group to the cell-adhesive material in advance in the production stage also enables a user to use the cell-adhesive photocontrollable base material after binding of a desired antibody or extracellular matrix by himself or herself. This embodiment has the advantage that a user can save time and labor for preparing the functional group and can arbitrarily select the antibody or extracellular matrix used.

In the case of using a cell-adhesive material in which an animal species-specific antibody is bound to the cell-adhesive group X, a dispersion medium, such as a medium or a buffer solution, which is routinely used in general cell culture can be arbitrarily selected as a dispersion medium for dispersing cells. In the case of using a cell-adhesive material having a covalently binding functional group as the cell-adhesive group X in the general formula (4), a dispersion medium free from a component reactive with the covalently binding functional group can be used as the dispersion medium. In the case of using a cell-binding material having a covalently binding functional group against an amino group, an amino group-free buffer solution, for example, HBSS (Hanks' balanced salt solution, containing Ca²⁺ and Mg²⁺) (hereinafter, abbreviated to HBSS(+)) can be preferably used as the cell dispersion medium. HBSS(+) contains cell adhesion factors Ca²⁺ and Mg²⁺ and therefore has the advantage that it can promote cell adhesion. HBSS(+) is effective for maintaining the adhesiveness of cells not only in the cell adhesion step but over general cell manipulation steps involving, for example, analyzing or partially recovering cells that have adhered. When the adhesiveness of cells is no longer necessary after the completion of cell adhesion, the covalently binding functional group described above can be reacted by the introduction of, for example, an amino group-containing buffer such as trishydroxymethylaminomethane, a protein such as an extracellular matrix, or serum-containing medium and thereby lose its reactivity.

For shortening a time required for the adhesion of cells to the cell-adhesive photocontrollable base material, it is preferable to also adopt the following means to the present invention: improvement in cell adhesiveness as described above is effective only if cells contact with the base material. However, even if a cell sample is introduced to the base material, cells are suspended in a sample solution, in other words, most of the cells are floated in the sample solution. Thus, the cells cannot immediately contact with the base material. In general, a time on the order of 30 minutes is required for the floated cells in the sample solution to precipitate and contact with the base material. In the case of a usage form, as in one aspect of the present invention, in which cells are temporarily captured by the base material for analysis and only target cells are detached and recovered, there is a problem of poor time efficiency because approximately 30 minutes are spent for waiting for cells to precipitate in the step. Thus, to shorten the time required for the step of causing cells to precipitate, the acceleration of cell precipitation by the application of centrifugal force was studied in the present invention. A typical centrifuge (himac CF7D2 model manufactured by Hitachi Koki Co., Ltd.) is used in combination with a swing rotor (RT3 S3 model) and a bucket for microplates according to SBS specification (turning radius: 170 mm) and can allow centrifugal force to act substantially perpendicularly to a cell-adhesive photocontrollable base material formed on the bottom surface of a microplate-type cell culture vessel used to cause cells to uniformly precipitate on the cell-adhesive photocontrollable base material. In the case of allowing centrifugal force of approximately 400 x g to act on a cell suspension in the apparatus configuration described above (the number of revolutions: approximately 1450 rpm), it was shown that almost all of the cells in a suspended state can be caused to precipitate by centrifugation operation for a time as short as 30 seconds at the shortest and for 90 seconds at the longest. In the case of using a cell culture vessel such as a dish (Petri dish) or a flask, the vessel can be prevented from being moved or broken during the centrifugation operation by using a tool for fixing the vessel to the bucket for SBS plates. In the case of using the apparatus configuration described above, acceleration and deceleration require additional times of approximately 33 seconds and approximately 41 seconds, respectively. That is, the time required for cells to precipitate is approximately 1.5 to 3 minutes including acceleration and deceleration by adopting the mode of the present invention. Thus, this mode of the present invention has the advantage that the time required for cells to precipitate can be shortened by an order of magnitude or more compared with approximately 30 minutes required for spontaneous precipitation as conventionally performed.

The spontaneous precipitation also has a problem of a small contact area and weak adhesion because only the lowermost ends of substantially spherical cells contact with the bottom of the vessel. The mode of the present invention allows cells to pressure-contact with the vessel by the application of centrifugal force and therefore has the advantage that the cells are deformed into a flat shape to increase the contact area so that the cells can strongly adhere thereto by virtue of a wide adhesion area.

The effectiveness of the centrifugation is not limited to the cell precipitation step. A cell adhesion step is generally necessary following the cell precipitation step. A total of approximately 3 hours is usually required for both the steps. When a cell-binding material having a covalently binding functional group in the terminal end is used as the cell-adhesive material or a cell-adhesive material in which an animal species-specific antibody is bound to the cell-adhesive group X is used as in the present invention, the time required for cell adhesion can be shortened, as mentioned above, because of the strong binding. This cell adhesion step can be combined with centrifugation to increase the contact area of the cells with the vessel, promoting the adhesion. Specifically, this embodiment has the advantage that the centrifugation is not terminated even after 30 or 90 seconds required for cell precipitation and can be continued for a few minutes thereafter to promote the cell adhesion and also to cause the cells to more strongly adhere to the vessel (or the cell-adhesive photocontrollable base material formed on the vessel).

Examples for proof of principle as one aspect of the present invention will be presented below. However, the present invention is not intended to be limited thereto.

### [Example 1]

A glass culture vessel (bottom surface area: 9.6 cm²) is prepared on which a tercopolymer of a methacrylic acid ester polymer represented by the general formula (1) (R¹: CH₃ and n: 1), a methacrylic acid ester polymer represented by the general formula (2) (R¹: CH₃ and R²: CH₂CH₂CH₂), and a methacrylic acid ester polymer represented by the general formula (14) (R¹: CH₃, R⁴: OCONH, R⁵: Br, R⁶: OH, R⁸: H, R⁹: H, R¹³: CH₂CH₂OCH₂CH₂, R¹⁶: CH₂CH₂OCOCH₂NHCH₂, and X: CO₂H) is formed as a film. As a model of unnecessary cells, 120,000 NIH/3T3 cells (mouse fibroblast-derived cell line) are prepared and suspended in 1.6 mL of a medium (10% calf serum, 90% DMEM) exclusive for this cell line. This NIH/3T3 cell suspension is added to the glass culture vessel and cultured at 37°C for 1 day in a 5% CO₂ incubator. As a model of necessary cells, 240,000 HCT116 cells (human colon cancer-derived cell line) are prepared and suspended in 1.6 mL of a medium (10% FBS, 90% McCoy's 5a) exclusive for this cell line. The medium is removed from the glass culture vessel containing the cultured NIH/3T3 cells. The HCT116 cell suspension is added to the glass culture vessel and cultured at 37°C for 1 day in a 5% CO₂ incubator. Aside from this, these cells of both cell lines are cultured alone in the same way as above, and their phase-contrast microscopic images are obtained. The HCT116 cells exhibit pavement-like arrangement while the NIH/3T3 cells exhibit a spindle-like form. The glass culture vessel is loaded in the apparatus of the present invention and observed under phase-contrast microscope with a light of 450 nm or shorter cut off. The position of the unnecessary cells (that is, the NIH/3T3 cells in a spindle-like form) is confirmed with the monitor. The inner periphery of the unnecessary cell group is selected as a laser ablation region (see Figures 1(1), 1(2), 1(3), 1(4a), and 1(5a)). The boundary between the necessary cells (that is, the HCT116 cells arranged in a pavement-like pattern) and the unnecessary cells and the cell-adhesive photocontrollable material are cut by the laser scanning irradiation with a laser light of 337 nm. Then, an unintended cell group region within an inner region surrounded by the laser ablation irradiation is subjected to laser light scanning at 375 nm for photoreaction. The glass culture vessel is removed from the apparatus. The unnecessary cell group is recovered together with the medium. A fresh medium is added to the vessel, which is then brought back to an incubator to continue culture. As a result, almost all of the unnecessary cells can be removed, and culture can be continued with the necessary cells left.

In this Example, the principle of operation is shown using model cells; however, similar operation may be performed by replacing the necessary cells with normal cells and the unnecessary cells with abnormal cells. Specifically, this technique is similarly applicable to, for example, differentiation induction research on stem cells for the purpose of selective fractionation for removing abnormal cells (cells differentiated into unintended cells, undifferentiated cells with stem cell properties maintained, etc.) and leaving normal cells (cells differentiated as desired, etc.). On the other hand, similar operation may be performed by replacing the necessary cells with abnormal cells and the unnecessary cells with normal cells. Specifically, this technique is similarly applicable to, for example, research on cancer cells for the purpose of selective fractionation for removing normal cells (unintended non-cancerous cells) and leaving abnormal cells (desired cancer cells, etc.) for concentration.

In Example described above, cells are discriminated on the basis of the form of the cells using a phase-contrast microscope as an approach for observing the cells; however, cells may be discriminated by other approaches. For example, target cells and unintended cells may be discriminated therebetween using a cell-specific fluorescent staining reagent and a fluorescence microscopic image.

In this Example, various types of cells can be caused to adhere by properly selecting the cell-adhesive group of the cell-adhesive photocontrollable material or properly controlling the ratio of the cell-adhesive material to the non-cell-adhesive material. The cell-adhesive photocontrollable base material is excellent in selectivity in the adhesion between cells and the base material because it is efficiently irreversibly changed from a cell-adhesive one to a non-cell-adhesive one by photodissociation reaction, and further, in recovering desired cells present in arbitrary regions, the purity and recovery rate of the cells can be increased because the adhesion among cells and the cell-adhesive photocontrollable material is cut with a laser light. In addition, it is unnecessary that in culturing cells, the cells be once taken out of the culture base material and purified as for a flow cytometer or a sorting device, and the culture can be performed on the same culture base material while removing unnecessary unintended cells in real time, simplifying the culture/purification operation. Even when target cells contact or mix with unintended cells, the unintended cells are spatially separated from the target cells and an unintended cell region is compartmentalized. This enables the photodissociation reaction to be conducted by being limited to the unintended cell region, enabling the selective detachment of the unintended cells. The cells once detached do not re-adhere because the original place is irreversibly changed into a non-cell-adhesive one, enabling the effective removal of the unintended cells. In addition, an electrical stimulus and an impact as for a flow cytometer or a sorting device are not present, and the control of the photoreaction wavelength, the light wavelength for phase-contrast microscopic observation, and the exciting light wavelength for fluorescent observation can reduce phototoxicity to cells; thus, the viability of cells can be increased. Further, cells are arranged on a 2-dimensional plane, almost simultaneously exposed to light to each cells, and subjected to phase-contrast microscopic or fluorescent observation; thus, optical axis adjustment for 1-dimensionally arranging cells and exposing individual cells to a laser as for the flow cytometer is unnecessary.

### [Example 2]

An alkoxysilane represented by the general formula (17) (R²: CH₂CH₂CH₂, R³: CH₃, R⁴: OCOO, R⁵: Br, R⁶: OH, R⁸: H, R⁹: H, and R¹⁹: CH₂NHCH₂) was formed as a film on a glass culture vessel. Subsequently, an azide compound: RGD peptide-NHCOCH₂CH₂OCH₂CH₂N₃ was subjected to Huisgen reaction in the presence of Cu ions. On this glass culture vessel, NIH/3T3 cells and HCT116 cells were continuously cocultured for 4 days in the same way as in Example 1. Then, the glass culture vessel is removed from the incubator. The cells are washed with PBS and treated with a blocking solution for cell surface markers (JRH Biosciences, Inc.) for 1 hour. An FITC-labeled mouse anti-human HLA-A/B/C antibody (BioLegend, Inc., clone W6/32) for detecting a human cell marker HLA antigen and a PE (phycoerythrin)-labeled rat anti-mouse H-2 antibody (BioLegend, Inc., clone M1/42) for detecting a mouse cell marker H-2 antigen are separately diluted with a blocking solution for cell surface markers, and each resulting staining solution is added to the cells and reacted at room temperature for 1 hour. The cells are washed with PBS. The cells were observed and fractionated as follows: the glass culture vessel is loaded in the apparatus of the present invention. In microscopic observation and fluorescent observation, a light of 450 nm or shorter in the wavelength region of the light source is cut off to prevent photoreaction. The positions of the human cells (labeled with FITC, fluorescence wavelength: 520 nm, greenish orange) and the mouse cells (labeled with PE, fluorescence wavelength: 575 nm, orange) are confirmed with the monitor under fluorescent observation and phase-contrast microscopic observation. A laser ablation region within each cell group region is selected (see Figures 1(1), 1(2), 1(3), 1(4b), and 1(5b)). The boundary between the human cells and the mouse cells and the cell-adhesive photocontrollable material are cut by the scanning of a laser light of 337 nm to separate each cell group region. Then, the human cell region is first subjected to laser light scanning at 375 nm for photoreaction. Then, the human cells detached and floated in the medium are recovered together with the medium. If necessary, after washing and addition of a medium, the different region, that is, the mouse cell region is subjected to laser light scanning at 375 nm for photoreaction. Then, the mouse cells detached and floated in the medium are recovered together with the medium.

In this Example, unlike Example 1, desired necessary cells are separated by fractionation with high purity, a high recovery rate, and high viability by reversing a region in which photodissociation reaction occurs.

Examples of an advantage unique to fluorescent observation performed as shown in this Example include the advantage that, even if necessary cells are contaminated with a very small amount of unnecessary cells, the necessary cells and the unnecessary cells can be selectively fluorescently labeled and detected and discriminated therebetween with high sensitivity by fluorescent detection to separate only the necessary cells by selective fractionation. In this Example, two or more types of selected necessary cells may be sequentially recovered from a mixture of three or more types of cells.

In this Example, the principle of operation is shown using model cells; however, similar operation may be performed by replacing the necessary cells with human cells and the unnecessary cells with mouse feeder cells. Specifically, this technique is applicable to, for example, research on the undifferentiation maintenance or differentiation induction of human stem cells for the purpose of removing unnecessary cells (mouse feeder cells) and sequentially and selectively fractionating plural types of target cells (human stem cells maintaining their pluripotency and various types of cells differentiated from human stem cells). In this case, the undifferentiation properties of human stem cells may be discriminated by adopting fluorescent staining or the like with the form of cells or various stem cell markers as indexes using a phase-contrast microscope. Of course, the model cells used in this Example may be selected according to various purposes, as in Example 1. The other effects of this Example are the same as those of Example 1.

### [Example 3]

Another glass culture vessel on which cells are cultured in the same way as in Example 1 is washed by the addition of PBS. Then, a trypsin/EDTA solution is added thereto, and the vessel is left at room temperature for a few minutes to detach the cells from the glass culture vessel. Then, the reaction was terminated by the addition of a trypsin-inhibiting solution. The cells were recovered into a centrifuge tube by pipetting and centrifuged for a few minutes. The supernate was discarded, and a medium was added to the residue to prepare a cell suspension. Cell staining using a human cell marker and a mouse cell marker as indexes was performed in the same way as in Example 2. Next, a glass culture vessel was prepared on which a tercopolymer of a methacrylic acid ester polymer represented by the general formula (I) (R¹: CH₃ and n: 1), a methacrylic acid ester polymer represented by the general formula (2) (R¹: CH₃ and R²: CH₂CH₂CH₂), and a methacrylic acid ester polymer represented by the general formula (11) (R¹: CH₃, R⁴: OCOO, R⁵: Br, R⁶: OH, R⁸: H, R⁹: H, R¹¹: CH₂NHCH₂CH₂OCH₂CH₂, R¹⁴: CH₂CH₂, and X: CO₂H) was formed as a film. PBS was added thereto, and the resulting vessel was loaded in the apparatus of the present invention. So as to arrange cell-adhesive regions of 20 µm square in a lattice form with a pitch of 40 µm, the other regions were subjected to laser light scanning at 375 nm,and the reaction product was removed together with PBS. The resulting vessel was washed again with PBS. The cell suspension described above was seeded on this glass culture vessel, which was then shaken and then left standing for 3 hours. Alternatively, to accelerate adhesion, the cells after seeding and shaking were caused to precipitate on the bottom of the glass culture vessel using a plate centrifuge or the like and then left standing for 30 minutes. To remove cells that did not adhere, the medium was replaced with a fresh one, and then this vessel was loaded in the apparatus of the present invention. The cells were observed and fractionated as follows: in microscopic observation and fluorescent observation, a light of 450 nm or shorter in the wavelength region of the light source is cut off to prevent photoreaction. The positions of human cells, mouse cells, and regions to which no cells adhere are detected on the basis of color information on each address by microscopic observation and fluorescent observation. The address regions to which no cells adhere among the cell-adhesive regions are subjected to laser light scanning at 375 nm for photoreaction to prevent recovered cells from adhering thereto again. Then, the address regions of the human cells are subjected to laser light scanning at 375 nm for photoreaction, and the human cells are detached and recovered together with the medium. If necessary, after addition of a medium, the address regions of the mouse cells were subjected to laser light scanning at 375 nm for photoreaction, and the mouse cells were detached and recovered together with the medium.

In addition to effects similar to those of Examples 1 and 2, this Example can enhance the speed of optical detection, analysis, and fractionation by causing individual cells to adhere to addresses arranged in a lattice form in analyzing and fractionating the cells. In addition, such a method has the advantage that immediate fractionation can be performed concurrently with the observation and analysis of cell reaction or the like with a compound, enabling real-time operation.

### [Example 4]

150,000 Colo 320 HSR cells were suspended in a medium (10% FBS, 90% RPMI1640) for this cell line, seeded on a collagen-coated glass culture vessel, and cultured for 7 days. Then, the cells were detached with a 0.25% trypsin-PBS solution. After termination of the reaction, the cells were suspended in a fresh medium having the same composition as above.

Aside from this, a glass culture vessel was prepared on which a tercopolymer of a methacrylic acid ester polymer represented by the general formula (1) (R¹: CH₃ and n:1), a methacrylic acid ester polymer represented by the general formula (2) (R¹: CH₃ and R²: CH₂CH₂CH₂), and a methacrylic acid ester polymer represented by the general formula (12) (R¹: CH₃, R⁴: OCOO, R⁵: Br, R⁶: OH, R⁷: H, R⁸: H, R¹²: CH₂CH₂(C₂HN₃)CH₂CH₂OCH₂CH₂, R¹⁴: CH₂CH₂, and X: CO₂H, was formed as a film and further coated with collagen. PBS was added thereto, and the resulting vessel was loaded in the apparatus of the present invention. So as to arrange cell-adhesive regions of 20 µm square in a lattice form with a pitch of 40 µm, laser scanning was first performed in a lattice pattern with a laser light of 337 nm to cut the cell-adhesive photocontrollable material. Subsequently, the regions other than the cell-adhesive regions were subjected to laser light scanning at 375 nm, and the reaction product was removed together with with PBS. The resulting vessel was washed again with with PBS.

The cell suspension described above was seeded on this vessel, which was then shaken and then left standing for 3 hours. Alternatively, to accelerate adhesion, the cells after seeding and shaking were caused to precipitate on the bottom of the glass culture vessel using a plate centrifuge or the like and then left standing for 30 minutes. To remove cells that did not adhere, the medium was replaced with a fresh one. The cells were observed and fractionated as follows: in microscopic observation, a light of 450 nm or shorter in the wavelength region of the light source was cut off to prevent photoreaction. The position of Colo 320 HSR cells was detected on the basis of cell image information on each address by microscopic observation. The address regions to which no cells adhered among the cell-adhesive regions were subjected to laser light scanning at 375 nm for photoreaction to prevent recovered cells from adhering thereto again. Then, the address regions of the Colo 320 HSR cells were subjected to laser light scanning at 375 nm for photoreaction, and the Colo 320 HSR cells were detached and recovered together with the medium.

In addition to effects similar to those of Examples 1 and 2, this Example effectively acts on the patterning of a fibrous or membranous material such as an extracellular matrix or feeder cells in the case of using the material in cell adhesion. The first light irradiation by itself may not cut the cell-adhesive material into the regions when the fibrous or membranous material is present on the upper layer. In this case, the second light irradiation is performed to cut it between the regions.

In this Example, collagen was used as the extracellular matrix, and low adhesive Colo 320 HSR cells were used as the model. In this Example, other extracellular matrixes or low adhesive cells may also be preferably used. Examples of the extracellular matrix include the collagen described above as well as fibronectin, laminin, and gelatin. As the feeder cells, mouse embryonic fibroblast (MEF) cells, STO cells, 3T3 cells, SNL cells, or the like treated with gamma ray irradiation or an antibiotic for the arrest of proliferation can be used. Examples of the cells that require the extracellular matrix include pluripotent stem cells such as ES cells and iPS cells, and also corneal epithelial stem cells.

### [Example 5]

Another glass culture vessel on which cells are cultured in the same way as in Example 3 is washed by the addition of PBS. Then, a trypsin/EDTA solution is added thereto, and the vessel is left at room temperature for a few minutes to detach the cells from the glass culture vessel. Then, the reaction was terminated by the addition of a trypsin-neutralizing solution. The cells were recovered into a centrifuge tube by pipetting and centrifuged for a few minutes. The supernate was discarded, and a medium was added to the residue to prepare a cell suspension. Fluorescent staining using a human cell marker and a mouse cell marker as indexes was also performed in the same way as in Example 3. Next, a glass culture vessel was prepared on which a tercopolymer of a methacrylic acid ester polymer represented by the general formula (1) (R¹: CH₃ and n: 1), a methacrylic acid ester polymer represented by the general formula (2) (R¹: CH₃ and R²: CH₂CH₂CH₂), and a methacrylic acid ester polymer represented by the general formula (13) (R¹:CH₃, R⁴:OCONH, R⁵:Br, R⁷:H, R⁸:H, R⁹:H, R¹³:CH₂CH₂OCH₂CH₂, R¹⁵:CH₂CH₂OCOCH₂O, X:CO₂H) was formed as a film. PBS was added thereto, and the resulting vessel was loaded in the apparatus of the present invention. So as to arrange cell-adhesive regions of 20 µm square in a lattice form with a pitch of 40 µm, the other regions were subjected to laser light scanning at 365 nm, and the reaction product was removed together with PBS. The resulting vessel was washed again with PBS. The cell suspension described above was seeded on this glass culture vessel, which was then shaken and then left standing for 3 hours. Alternatively, to accelerate adhesion, the cells after seeding and shaking were caused to precipitate on the bottom of the glass culture vessel using a plate centrifuge or the like and then left standing for 30 minutes. To remove cells that did not adhere, the medium was replaced with a fresh one, and then this vessel was loaded in the apparatus of the present invention. The cells were observed and fractionated as follows: in microscopic observation and fluorescent observation, a light of 450 nm or shorter in the wavelength region of the light source is cut off to prevent photoreaction. The positions of human cells, mouse cells, and regions to which no cells adhere are detected on the basis of color information on each address by microscopic observation and fluorescent observation. The address regions to which no cells adhere among the cell-adhesive regions are subjected to light pattern irradiation at 365 nm for photoreaction to prevent recovered cells from adhering thereto again. Then, the address regions of the human cells are subjected to light pattern irradiation at 365 nm for photoreaction, and the human cells are detached and recovered together with the medium. If necessary, after addition of a medium, the address regions of the mouse cells were subjected to light pattern irradiation at 365 nm for photoreaction, and the mouse cells were detached and recovered together with the medium.

In addition to effects similar to those of Examples 1 and 2, this Example can enhance the speed of optical detection, analysis, and fractionation by causing individual cells to adhere to addresses arranged in a lattice form in analyzing and fractionating the cells. In addition, such a method has the advantage that immediate fractionation can be performed concurrently with the observation and analysis of cell reaction or the like with a compound, enabling real-time operation.

### [Example 6]

Cells cultured in the same way as in Example 3 were detached and recovered. Then, fluorescent staining was performed independently for each cell line. The HCT116 cells and NIH/3T3 cells thus fluorescently stained were separately suspended in a buffer solution (HBSS(+)) to obtain cell suspensions. The buffer solution used in the description below is HBSS(+) unless otherwise specified.

Next, a culture vessel was prepared which was provided at its bottom with a glass member on which a tercopolymer of a methacrylic acid ester polymer represented by the general formula (1) (R¹: CH₃ and n: 1), a methacrylic acid ester polymer represented by the general formula (2) (R¹: CH₃ and R²: CH₂CH₂CH₂), and a methacrylic acid ester polymer represented by the general formula (14) (R¹:CH₃,R⁴:OCONH,R⁵:Br,R⁶:OH,R⁸:H,R⁹:H,R¹³:CH₂CH₂OCH₂CH₂,R¹⁶:CH₂CH₂OCOCH₂N HCH₂,X:CO₂H,) was formed as a film.

A reaction solution containing 0.4 mg of 1-ethyl-3-(3-dimethylaminopropyl)carbodiimide hydrochloride (EDC) dissolved in 1 mL of an activating buffer solution (0.1 M MES, 0.5 M NaCl, pH 6.0) was added to the culture vessel described above on which a film of polymer was formed. Further, sulfo-NHS (N-hydroxysulfosuccinimide) was added at a final concentration of 5 mM to the reaction solution, and the vessel was shaken at room temperature for 15 minutes. The reaction solution was discarded, and the vessel was washed three times with PBS (pH 7.4) to prepare a culture vessel provided with a cell-binding photocontrollable base material having a covalently binding functional group and the substituent X represented by CO-Z (provided that Z is a N-oxysulfosuccinimide group). All of the above operations were performed in a light-shielded state. This cell-binding photocontrollable base material has an active ester group of a carboxylic acid as the covalently binding functional group and therefore has a property of performing covalent binding reaction with an amino group. In addition, this cell-binding photocontrollable base material is stable unless reacted with a nucleophilic reagent such as an amino group, and can thus be stored for a long period with its binding properties maintained in various environments such as PBS or HBSS(+) in the nitrogen atmosphere, in the air, or in an aseptic state.

A buffer solution was added to the culture vessel described above, which was then loaded in the apparatus of the present invention. So as to arrange cell-adhesive regions of 20 µm square in a lattice form with a pitch of 40 µm, the other regions were subjected to laser light scanning at 375 nm, and the reaction product was removed together with the buffer solution. The resulting vessel was washed again with a buffer solution. The cell suspension described above was seeded on this culture vessel, which was then shaken and then loaded in the swing rotor for plates of a centrifuge. After fixing with a fixture, the vessel was centrifuged for approximately 2 minutes at a centrifugal acceleration of 400 x g to cause the cells to precipitate on the bottom of the culture vessel. Then, the vessel was left standing for 5 minutes. Easy centrifugation operation can be achieved with the light-shielded conditions maintained by using a fixture that is not only capable of fixing the culture vessel to the swing rotor but has a cover for shielding the culture vessel against outside light. This operation allows the cells to contact by precipitation and pressure-contact onto the cell-binding photocontrollable base material while allowing the covalently binding functional group of the cell-binding photocontrollable base material to covalently bind to an amino group which is a cell surface functional group so that the cells adhere strongly and rapidly to the cell-binding photocontrollable base material. A very small number of cells that did not adhere were removed by the replacement of the cell buffer solution, and then the resulting vessel was loaded in the apparatus of the present invention. All of the above operations were performed in a light-shielded state. The cells were observed and fractionated in the same way as in Example 3. Specifically, in microscopic observation and fluorescent observation, a light of 450 nm or shorter in the wavelength region of the light source is cut off to prevent photoreaction. The positions of human cells, mouse cells, and regions to which no cells adhere are detected on the basis of color information on each address by microscopic observation and fluorescent observation. The address regions to which no cells adhere among the cell-adhesive regions are subjected to laser light scanning at 375 nm for photoreaction to prevent recovered cells from adhering thereto again. Then, the address regions of the human cells are subjected to laser light scanning at 375 nm for photoreaction, and the human cells are detached and recovered together with the buffer solution. If necessary, after addition of a buffer solution, the address regions of the mouse cells were subjected to laser light scanning at 375 nm for photoreaction, and the mouse cells were detached and recovered together with the buffer solution.

In addition to effects similar to those of Example 3, this Example can shorten a time required for cell adhesion and enhance the speed of operation by using the cell-binding photocontrollable base material in the cell adhesion step and using an amino group-free HBSS(+) containing Ca²⁺ or Mg²⁺ as a cell dispersion medium. In addition, such a method can achieve immediate fractionate by shortening a pretreatment time and suppress the damage or degeneration of cells, enabling real-time operation.

### [Example 7]

Cells cultured in the same way as in Example 6 were detached and recovered. Then, fluorescent staining was performed independently for each cell line. The HCT116 cells and NIH/3T3 cells thus fluorescently stained were separately suspended in a buffer solution (HBSS(+)) to obtain cell suspensions. The buffer solution used in the description below is HBSS(+) unless otherwise specified.

Next, a culture vessel was prepared which was provided at its bottom with a glass member on which a tercopolymer of a methacrylic acid ester polymer represented by the general formula (1) (R¹: CH₃ and n: 1), a methacrylic acid ester polymer represented by the general formula (2) (R¹: CH₃ and R²: CH₂CH₂CH₂), and a methacrylic acid ester polymer represented by the general formula (14) (R¹:CH₃,R⁴:OCONH,R⁵:Br,R⁶:OH,R⁸:H,R⁹:H,R¹³:CH₂CH₂OCH₂CH₂,R¹⁶:CH₂CH₂OCOCH₂N HCH₂,X:CO₂H,) was formed as a film.

A reaction solution containing 0.4 mg of 1-ethyl-3-(3-dimethylaminopropyl)carbodiimide hydrochloride (EDC) dissolved in 1 mL of an activating buffer solution (0.1 M MES, 0.5 M NaCl, pH 6.0) was added to the culture vessel described above on which a film of polymer was formed. Further, sulfo-NHS (N-hydroxysulfosuccinimide) was added at a final concentration of 5 mM to the reaction solution, and the vessel was shaken at room temperature for 15 minutes. The reaction solution was discarded, and the vessel was washed three times with PBS (pH 7.4) to prepare a culture vessel having a covalently binding functional group and the substituent X represented by CO-Z (provided that Z is a N-oxysulfosuccinimide group). Next, a PBS solution containing 100 µg of a purified mouse anti-human HLA-A/B/C antibody (BioLegend, Inc., clone W6/32) was added to the culture vessel and shaken at room temperature for 30 minutes. The reaction solution was discarded, and the vessel was washed once with PBS (pH 7.4) to prepare a culture vessel provided with a cell-binding photocontrollable base material having the antibody against the cell surface antigen and the substituent X represented by CO-Z (provided that Z is a residue of the amino group of the antibody from which H was removed). All of the above operations were performed in a light-shielded state. This cell-binding photocontrollable base material has the antibody against the human cell surface antigen and therefore has a property of strongly binding to human cells through antigen-antibody reaction and not binding to mouse cells. In addition, since the antibody is stable, this cell-binding photocontrollable base material can be stored for a long period with its binding properties maintained in various environments such as PBS or HBSS(+) in an aseptic state.

A buffer solution was added to the culture vessel described above, which was then loaded in the apparatus of the present invention. So as to arrange cell-adhesive regions of 20 µm square in a lattice form with a pitch of 40 µm, the other regions were subjected to laser light scanning at 375 nm, and the reaction product was removed together with the buffer solution. The resulting vessel was washed again with a buffer solution. The cell suspension described above was seeded on this culture vessel, which was then shaken and then loaded in the swing rotor for plates of a centrifuge. After fixing with a fixture, the vessel was centrifuged for approximately 5 minutes at a centrifugal acceleration of 400 x g to cause the cells to precipitate and pressure-contact on the bottom of the culture vessel. This operation allows the cells to contact by precipitation and pressure-contact onto the cell-binding photocontrollable base material while allowing the antibody of the cell-binding photocontrollable base material to bind to the surface antigen of the human cells through antigen-antibody reaction so that the human cells adhere strongly and selectively to the cell-binding photocontrollable base material. Since this antibody does not react with the surface antigen of the mouse cells, the mouse cells do not adhere thereto. The mouse cells that did not adhere were removed by the replacement of the cell buffer solution, and then the resulting vessel was loaded in the apparatus of the present invention. All of the above operations were performed in a light-shielded state. The cells were observed and fractionated in the same way as in Example 3. Specifically, in microscopic observation and fluorescent observation, a light of 450 nm or shorter in the wavelength region of the light source is cut off to prevent photoreaction. The positions of human cells and regions to which no cells adhere are detected on the basis of color information on each address by microscopic observation and fluorescent observation. The address regions to which no cells adhere among the cell-adhesive regions are subjected to laser light scanning at 375 nm for photoreaction to prevent recovered cells from adhering thereto again. Then, the address regions of the human cells are subjected to laser light scanning at 375 nm for photoreaction, and the human cells are detached and recovered together with the buffer solution.

In addition to effects similar to those of Example 3, this Example can shorten a time required for cell adhesion and enhance the speed of operation by using the cell-binding photocontrollable base material having an antibody in the cell adhesion step. In addition, an excess of mouse cells coexisting with human cells can be removed in advance by using an antibody against an antigen specific for the human cells, enabling reduction in interference with analysis. Such a method can achieve immediate fractionate by shortening a pretreatment time and suppress the damage or degeneration of cells, enabling real-time operation with high precision.

nm, In a modification of this Example, the following method may be adopted: a vessel provided at its bottom with a commercially available base material having a COOH group on the surface was used in the same way as in Example 7 to prepare a vessel having a covalently binding functional group and the substituent X represented by CO-Z (provided that Z is a N-oxysulfosuccinimide group). Next, a PBS solution of a purified anti-mouse CD9 antibody (clone MZ3) was added thereto and reacted in the same way as in Example 7 to prepare a vessel having the antibody against the mouse cell surface antigen. The cell suspension was seeded on this vessel, which was then shaken and then centrifuged to cause the cells to precipitate and pressure-contact on the bottom of the vessel. This operation allows the mouse cells to selective bind through antigen-antibody reaction while human cells neither react nor adhere. Specifically, this supernate contains only the human cells. The cells are observed and fractionated in the same way as in Example 7 using this supernate as the cell suspension, enabling reduction in interference with analysis.

Hereinafter, Examples of other cell-adhesive photocontrollable materials used in cell manipulation operation similar to that in Examples 1 to 7 will be shown.

### [Example 8]

Material of the general formula (14) wherein (R¹:CH₃, R⁴:OCOO, R⁵:Br, R⁶:OH, R⁸:H, R⁹:H, R¹³:CH₂CH₂, R¹⁶:CH₂CH₂OCOCH₂CH₂CH₂CH₂CH₂(N₃CHC)NCH₃CH₂, X:CO₂H)

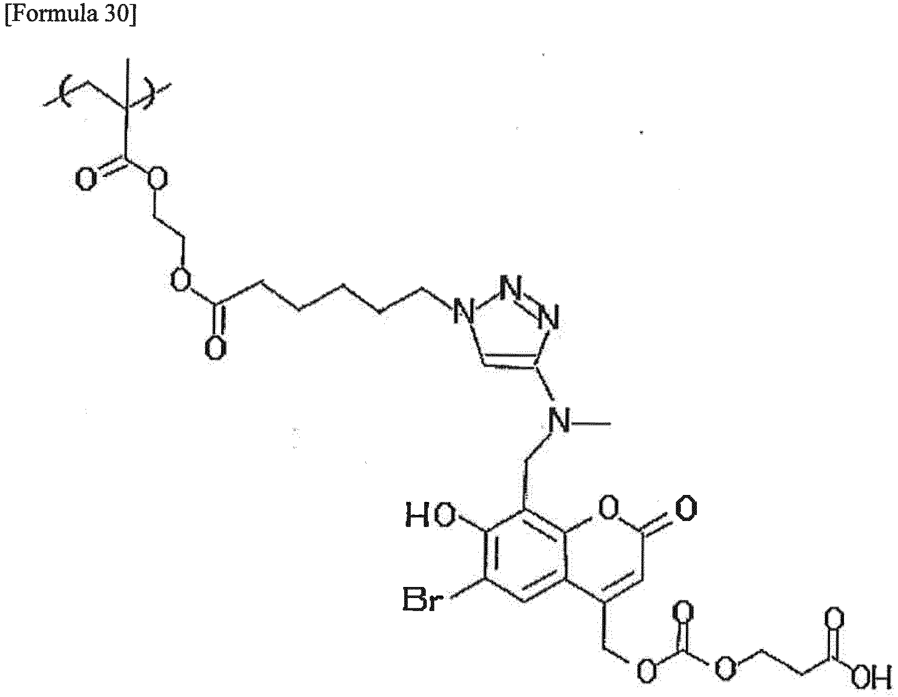

### (1) Synthesis of 6-bromo-7-hydroxy-4-(hydroxymethyl)-8-((methyl(prop-2-yn-1-yl)amino)methyl)-2H-chromen-2-one

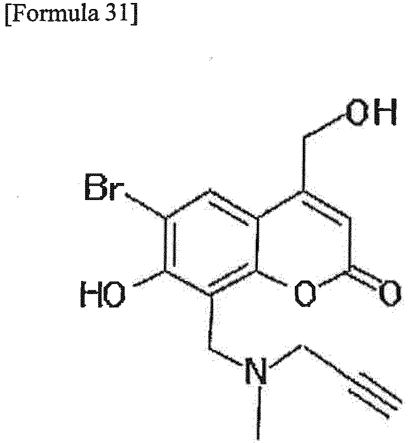

A magnetic stirrer bar and paraformaldehyde (446.4 mg, 14.87 mmol) were placed in a 50-mL eggplant-shaped flask equipped with a reflux condenser and a three-way valve with an argon-filled balloon. The flask was purged with argon, and then ethanol (5 mL) and N-methylpropargylamine (1.25 mL, 14.8 mmol) were added thereto in this order. To a solution formed by stirring at room temperature for 1 hour, 6-bromo-7-hydroxy-4-hydroxymethyl-coumarin (1.3668 g, 5.042 mmol) was added, and the mixture was continuously stirred for 12 hours with heating to 80°C in an oil bath. The reaction mixture was cooled to room temperature, and then the formed precipitates were collected by suction filtration, washed with ethanol, and dried in a vacuum desiccator to obtain 1.3933 g (3.956 mmol, yield: 78%) of the desired compound: 6-bromo-7-hydroxy-4-(hydroxymethyl)-8-((methyl(prop-2-yn-1-yl)amino)methyl)-2H-chromen-2-one as a pale yellow solid.

¹H NMR δ (CDCl₃) 7.62(1H,s), 6.45(1H, t, J 1.5Hz), 4.84(2H, d, J 1.5Hz), 4.19(2H, s), 3.47(2H, d, J 2Hz), 2.48(3H, s), 2.40(1H, t, J 2Hz)

### (2) Synthesis of tert-butyl 3-((((6-bromo-7-hydroxy-8-((methyl(prop-2-yn-1-yl)amino)methyl)-2-oxo-2H-c omen-4-yl)methoxy)carbonyl)oxy)propanoate

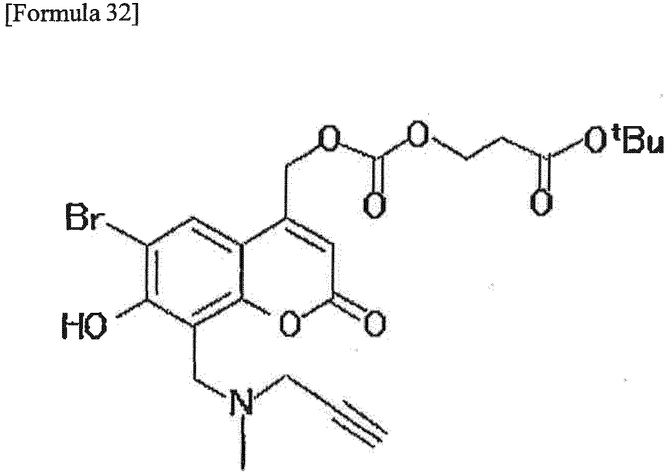

A magnetic stirrer bar and tert-butyl 3-(((4-nitrophenoxy)carbonyl)oxy)propanoate (100.0 mg, 0.321 mmol) were placed in a 50-mL eggplant-shaped flask equipped with a CaCl₂ tube. CH₂Cl₂ (2 mL), 4-dimethylaminopyridine (48.8 mg, 0.400 mmol), and 6-bromo-7-hydroxy-4-(hydroxymethyl)-8-((methyl(prop-2-yn-l-yl)amino)methyl)-2H-chromen-2-one (70.0 mg, 0.199 mmol) were added thereto in this order, and the mixture was stirred at room temperature for 13 hours. The reaction was terminated by the addition of water, and then a separated organic layer was dried over anhydrous magnesium sulfate. The desiccant was filtered off, and then the filtrate was concentrated to obtain a crude product, which was then purified by flash column chromatography (20 g of silica gel 60, Merck KGaA, elution solvent: 2.4% methanol-dichloromethane) to obtain 83.1 mg (0.153 mmol, yield: 77%) of the desired compound: tert-butyl 3-((((6-bromo-7-hydroxy-8-((methyl(prop-2-yn-1-yl)amino)methyl)-2-oxo-2H-chromen-4-yl)methoxy)carbonyl)oxy)propanoate.

¹H NMR δ (CDCl₃) 7.60(1H, s), 6.34(1H, t, J1Hz), 5.26(2H, d, J1Hz), 4.45(2H, t, J6Hz), 4.18(2 , s), 3.47(2H, d, J 2Hz), 2.92(1H, brs), 2.65(2H, t, J6Hz), 2.49(3H, s), 2.42(1H, t, J 2Hz), 1.46(9H, s)

### (3) Synthesis of 3-((((6-bromo-7-hydroxy-8-((methyl(prop-2-yn-1-yl)amino)methyl)-2-oxo-2H-chromen-4-yl)methoxy)carbonyl)oxy)propanoic acid (Synthesis of material of the general formula (23) wherein R⁴: OCOO, R⁵: Br, R⁶: OH, R⁸: H, R⁹: H, R³¹: CH₂CH₂, R³²: CH₂NCH₃CH₂, and X: CO₂H)

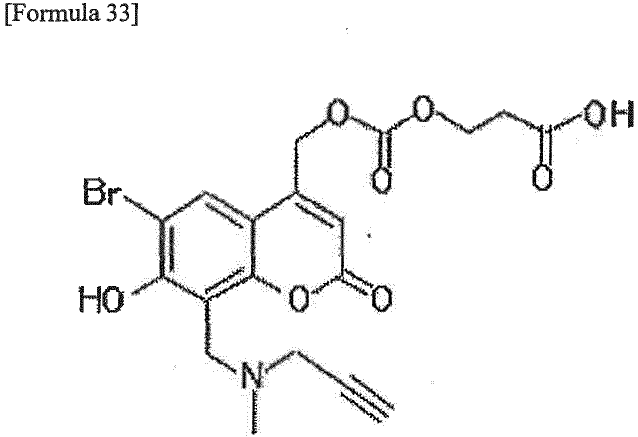

A magnetic stirrer bar and tert-butyl 3-((((6-bromo-7-hydroxy-8-((methyl(prop-2-yn-1-yl)amino)methyl)-2-oxo-2H-chromen-4-yl) methoxy)carbonyl)oxy)propanoate (83.1 mg, 0.15 mmol) were placed in a 50-mL eggplant-shaped flask equipped with a CaCl₂ tube. While the flask was cooled in an ice bath, CH₂Cl₂ (1 mL) and trifluoro-acetic acid (0.5 mL) were added thereto in this order, and the mixture was stirred for 3 hours in the ice bath. The solvent was distilled off under reduced pressure to obtain 90.3 mg (0.15 mmol) of the desired compound: 3-((((6-bromo-7-hydroxy-8-((methyl(prop-2-yn-1-yl)amino)methyl)-2-oxo-2H-chromen-4-yl)methoxy)carbonyl)oxy)propanoic acid as a trifluoro-acetic acid salt.

¹H NMR δ (CDCl₃) 7.77(1H, s), 6.40(1H, s), 5.29(2H, s), 4.56(2H, s), 4.49(2H,t, J 6Hz), 3.97(2H, d, J 2Hz), 2.95(1H, s), 2.90(3H, s), 2,73(2H, t, J 6Hz), 2.78(1H, t, J 2Hz)

### (4) Synthesis of poly(2-(6-azido-hexanoate) ethyl methacrylate)

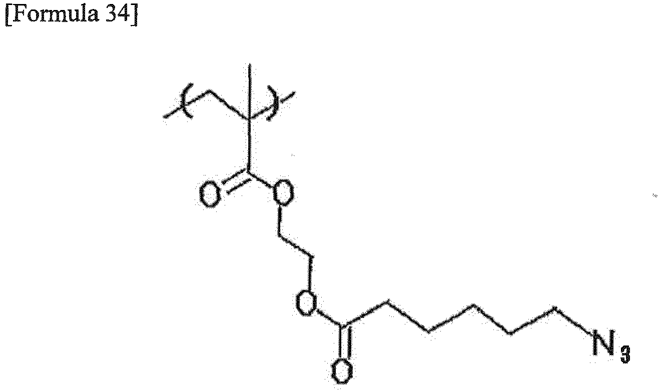

A magnetic stirrer bar and 6-azidohexanoic acid (195.2 mg, 1.24 mmol) were placed in a 10-mL eggplant-shaped flask equipped with a three-way valve with an argon-filled balloon. The flask was purged with argon, and then DMF (1.5 mL), 65.0 mg of poly(2-hydroxyethyl methacrylate) (PolyHEMA, Average MV: to 300,000, Aldrich 192066-10G), N,N'-diisopropylcarbodiimide (156.6 µL, 1.00 mmol), and 4-dimethylaminopyridine (6.5 mg, 0.29 mmol) were added thereto in this order, and the mixture was stirred at room temperature for 3 days. The formed precipitates were filtered off, and water was added to the filtrate to form white precipitates. The obtained white precipitates were collected by suction filtration and then dissolved in THF. To this solution, hexane was added to reprecipitate a solid, which was then separated by decantation to obtain the desired compound: poly(2-(6-azido-hexanoate) ethyl methacrylate).

¹H NMR δ (CDCl₃) 4.28(broad), 4.15(broad), 3.31(broad), 2.40(broad), 1.9-1.4, 1.03(broad), 0.88(broad)

### (5) Synthesis of material of the general formula (14) wherein (R¹:CH₃, R⁴:OCOO, R⁵:Br, R⁶:OH, R⁸:H, R⁹:H, R¹³:CH₂CH₂, R¹⁶:CH₂CH₂OCOCH₂CH₂CH₂CH₂CH₂(N₃CHC)NCH₃CH₂, X:CO₂H)

A magnetic stirrer bar, 3-((((6-bromo-7-hydroxy-8-((methyl(prop-2-yn-1-yl)amino)methyl)-2-oxo-2H-chromen-4-yl)methoxy)carbonyl)oxy)propanoic acid (material of the general formula (23) wherein (R⁴:OCOO, R⁵:Br, R⁶:OH, R⁸:H, R⁹:H, R³¹:CH₂CH₂, R³²:CH₂NCH₃CH₂, X:CO₂H) and X: CO₂H)) (60.8 mg, 0.12 mmol), CuBr(I) (4.2 mg, 0.028 mmol), and 4-dimethylaminopyridine (16.1 mg, 0.13 mmol) were placed in a 5-mL eggplant-shaped flask equipped with a three-way valve with an argon-filled balloon. The flask was purged with argon, and then DMF (1.8 mL), N,N,N',N",N"-pentamethyldiethylenetriamine (6 µL, 0.029 mmol), and poly(2-(6-azido-hexanoate) ethyl methacrylate) (23.5 mg) were added thereto in this order, and the mixture was stirred at room temperature for 19 hours. DMF was removed under reduced pressure, and then CHCl₃ (5 mL) was added to the residue to obtain precipitates of the desired compound: material of the general formula (14) wherein (R¹:CH₃, R⁴:OCOO, R⁵:Br, R⁶:OH, R⁸:H, R⁹:H, R¹³:CH₂CH₂, R¹⁶:CH₂CH₂OCOCH₂CH₂CH₂CH₂CH₂(N₃CHC)NCH₃CH₂, X:CO₂H)

The following preparation methods (6) and (7) employed surface reaction on a glass culture vessel.

### (6) Film formation of poly(2-(6-azido-hexanoate) ethyl methacrylate) on glass substrate

Poly(2-(6-azido-hexanoate) ethyl methacrylate) was dissolved in DMF to prepare a 0.2 w/v% solution. Subsequently, a glass culture vessel was washed with an organic solvent and further washed with a mixed solution of H₂SO₄:H₂O₂ = 3:1. The polymer solution described above was added dropwise onto the glass culture vessel. The vessel was dried overnight to form a thin film of the polymer on the glass surface.

### (7) Click reaction with 3-((((6-bromo-7-hydroxy-8-((methyl(prop-2-yn-1-yl)amino)methyl)-2-oxo-2H-chromen-4-yl)methoxy)carbonyl)oxy)propanoic acid

A DMF solution of 1 mM 3-((((6-bromo-7-hydroxy-8-((methyl(prop-2-yn-1-yl)amino)methyl)-2-oxo-2H-chromen-4-yl)methoxy)carbonyl)oxy)propanoic acid and 1.35 mM dimethylaminopyridine was mixed with 1.25 v% of a DMF solution of 1 mM N,N,N',N",N"-pentamethyldiethylenetriamine and 1 mM CuBr to prepare a reaction solution. This reaction solution was added to the glass surface described above to which a film of azide polymer was formed and reacted at room temperature for 24 hours in the dark to conduct the film formation of the material of the general formula (14) wherein (R¹:CH₃, R⁴:OCOO, R⁵:Br, R⁶:OH, R⁸:H, R⁹:H, R¹³:CH₂CH₂, R¹⁶:CH₂CH₂OCOCH₂CH₂CH₂CH₂CH₂(N₃CHC)NCH₃CH₂, X:CO₂H) on the glass culture vessel.

### [Example 9]

Material of the general formula (26) wherein (R³:CH₃, R⁴:OCONH, R⁵:Br, R⁶:OH, R⁸:H, R⁹:H, R³¹:CH₂CH₂CH₂CH₂CH₂CH₂, R³²:CH₂NCH₃CH₂, R³³:CH₂CH₂CH₂CH₂CH₂CH₂CH₂CH₂CH₂CH₂CH₂,X:NH₂)

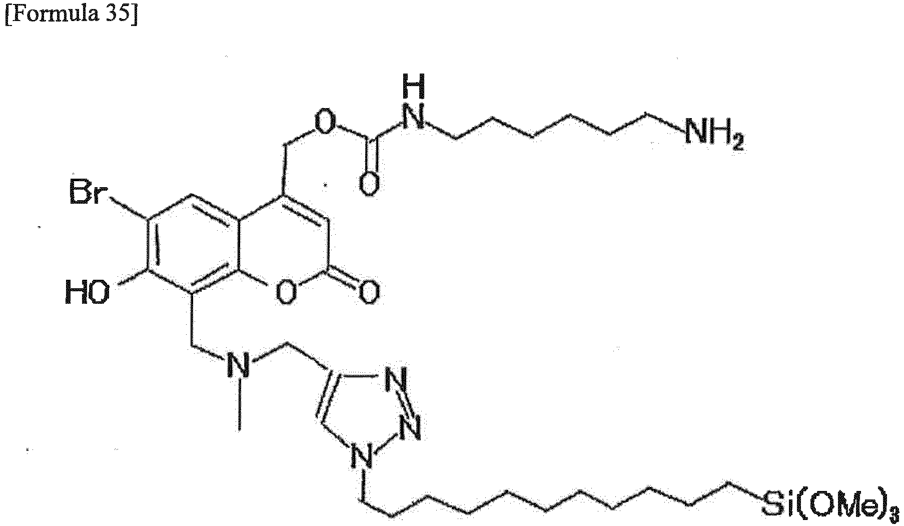

### (1) Synthesis of (6-bromo-7-hydroxy-8-((methyl(prop-2-yn-1-yl)amino)methyl)-2-oxo-2H-chromen-4-yl)methyl tert-butyl hexane-1,6-diyldicarbamate

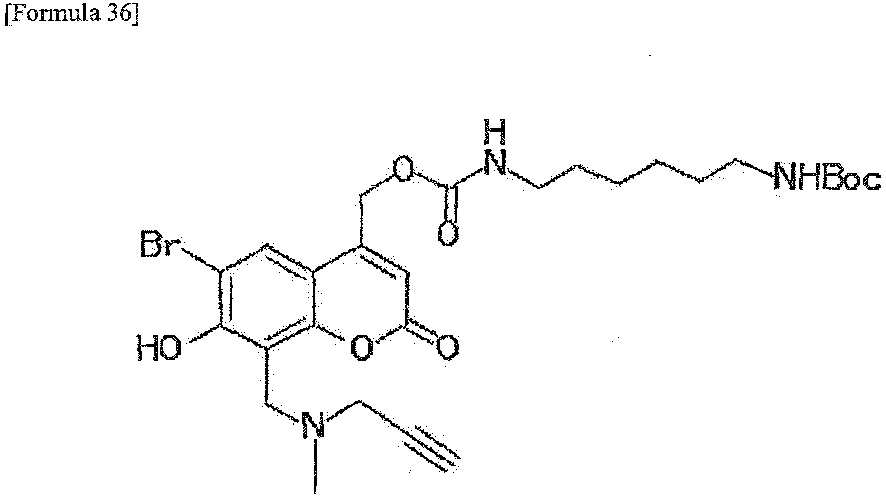

A magnetic stirrer bar and N,N'-carbonyldiimidazole (159.1 mg, 0.981 mmol) were placed in a 30-mL eggplant-shaped flask equipped with a CaCl₂ tube. CH₂Cl₂ (3 mL) and 6-bromo-7-hydroxy-4-(hydroxymethyl)-8-((methyl(prop-2-yn-1-yl)amino)methyl)-2H-chromen-2-one (344.5 mg, 0.978 mmol) were added thereto, and the mixture was stirred at room temperature for 1 hour. To the resulting reaction mixture, 4-dimethylaminopyridine (136.6 mg, 1.12 mmol) and tert-butyl (6-aminohexyl)carbamate (211.8 mg, 0.979 mmol) dissolved in 1 mL of CH₂Cl₂ were added, and the mixture was stirred at room temperature for 3 hours. The formed precipitates were filtered off with washing with CH₂Cl₂, and the filtrate was concentrated to obtain a crude product (988.0 mg), which was then purified by flash column chromatography (50 g of silica gel 60, Merck KGaA, elution solvent: dichloromethane/methanol: 30/1) to obtain 376.6 mg (0.633 mmol, yield: 65%) of the desired compound: (6-bromo-7-hydroxy-8-((methyl(prop-2-yn-1-yl)amino)methyl)-2-oxo-2H-chromen-4-yl) methyl tert-butyl hexane-1,6-diyldicarbamate.

¹H NMR δ (DMSO-d₆) 7.82 (1H, s), 7.52 (1H, t, J 5Hz), 6.76 (1H, brs), 6.14 (1H, s), 5.24 (2H, s), 4.12 (2H, s), 3.62 (2H, d, J 2Hz), 3.04 (2H, m), 2.90 (2H, m), 2.50 (1H, t, J 2Hz), 2.40 (3H, s), 1.40-1.20 (8H, m), 1.37 (9H,s)

### (2) Synthesis of (6-bromo-7-hydroxy-8-((methyl(prop-2-yn-1-yl)amino)methyl)-2-oxo-2H-chromen-4-yl) methyl(6-aminohexyl)carbamate (Synthesis of material of the general formula (23) wherein (R⁴:OCONH, R⁵:Br, R⁶:OH, R⁸:H, R⁹:H, R³¹:CH₂CH₂CH₂CH₂CH₂CH₂, R³²:CH₂NCH₃CH₂, X:NH₂)

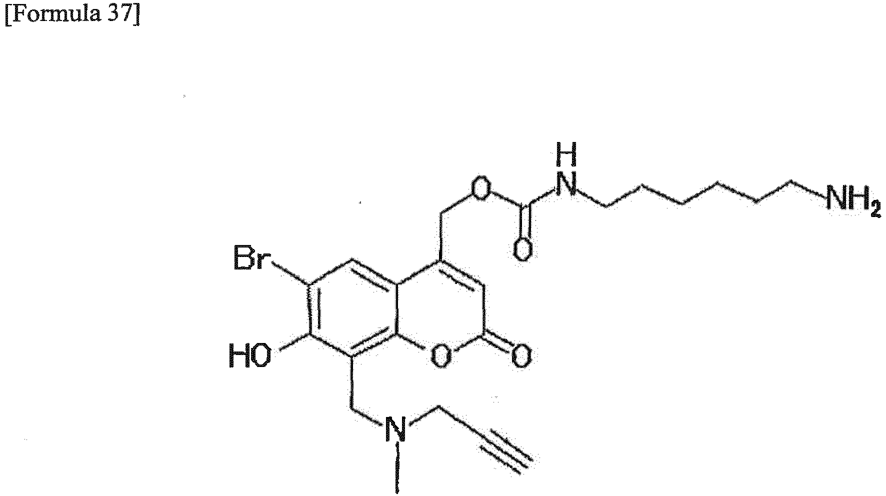

A magnetic stirrer bar and (6-bromo-7-hydroxy-8-((methyl(prop-2-yn-1-yl)amino)methyl)-2-oxo-2H-chromen-4-yl) methyl tert-butyl hexane-1,6-diyldicarbamate (15.6 mg, 0.026 mmol) were placed in a 20-mL eggplant-shaped flask equipped with a CaCl₂ tube. While the flask was cooled in an ice bath, dichloromethane (0.5 mL) and trifluoroacetic acid (0.5 mL) were added thereto in this order, and the mixture was stirred for 3 hours in the ice bath. The solvent was distilled off under reduced pressure to obtain 26.5 mg of the desired compound: (6-bromo-7-hydroxy-8-((methyl(prop-2-yn-1-yl)amino)methyl)-2-oxo-2H-chromen-4-yl) methyl(6-aminohexyl)carbamate (material of the general formula (23) wherein (R⁴:OCONH, R⁵:Br, R⁶:OH, R⁸:H, R⁹:H, R³¹:CH₂CH₂CH₂CH₂CH₂CH₂, R³²:CH₂NCH₃CH₂, X:NH₂) as a trifluoroacetic acid salt.

¹H NMR δ (DMSO-d₆) 8.01 (1H, s), 7.74 (2H, brs), 7.58 (1H, t, d 6Hz), 6.24 (1H, s), 5.29 (2H, s), 4.47 (2H, s), 4.14 (2H, s), 3.84 (1H, s), 3.05 (2H, m), 2.80 (2H, m), 2.71 (3H, s), 1.60 -1.25 (8H, m)

### (3) Film formation of 11-azido-undecyl trimethoxysilane on glass substrate

A glass culture vessel was washed in the same way as in Example 8(6). Subsequently, a 1 mM ethanol solution of 11-azido-undecyl trimethoxysilane (Altech Co., Ltd., SI005-m11) was added dropwise thereonto and reacted at room temperature for 24 hours. The vessel was washed with water and the dried at 95°C for 15 minutes to form a self-assembled monolayer film of the azido alkoxysilane described above on the glass substrate.

### (4) Click reaction with (6-bromo-7-hydroxy-8-((methyl(prop-2-yn-1-yl)amino)methyl)-2-oxo-2H-chromen-4-yl)methyl(6-aminohexyl)carbamate

A DMF solution of 1 mM (6-bromo-7-hydroxy-8-((methyl(prop-2-yn-1-yl)amino)methyl)-2-oxo-2H-chromen-4-yl) methyl(6-aminohexyl)carbamate and 1.35 mM dimethylaminopyridine was mixed with 1.25 vol% of a DMF solution of 1 mM N,N,N',N",N"-pentamethyldiethylenetriamine and 1 mM CuBr to prepare a reaction solution. This reaction solution was added to the glass surface described above to which a film of azido alkoxysilane self-assembled monolayer was formed and reacted at room temperature for 24 hours in the dark to form a self-assembled monolayer film of the material of the general formula (26) wherein (R³:CH₃, R⁴:OCONH, R⁵:Br, R⁶:OH, R⁸:H, R⁹:H, R³¹:CH₂CH₂CH₂CH₂CH₂CH₂, R³²:CH₂NCH₃CH₂, R³³:CH₂CH₂CH₂CH₂CH₂CH₂CH₂CH₂CH₂CH₂CH₂, X:NH₂) on the glass culture vessel.

All publications, patents, and patent applications cited in this specification are intended to be incorporated herein by reference in their entirety.

### Reference Signs List

- 1: Transparent Base Material
- 2: Cell-Adhesive Photocontrollable Material
- 3, 4, 9: Cell
- 5: Second Light Irradiation
- 6: Cut Region between Cells and of Cell-Adhesive Photocontrollable Material
- 7: First Light Irradiation
- 8: Region Changed from Cell-Adhesive One to Non-Cell-Adhesive One by Photoreaction (Non-Cell-Adhesive Region)
- 10, 11: Region which is changed from cell-adhesive to non cell-adhesive by light irradiation
- 69: Cell-Adhesive Region

## Claims

1. A cell-adhesive photocontrollable base material, wherein light irradiation causes the bond dissociation of a photolabile group comprising a coumarinylmethyl skeleton to separate a cell-adhesive material and leave a non-cell-adhesive material on a base material.

2. A cell-adhesive photocontrollable base material obtained by conducting a film formation of a cell-adhesive photocontrollable material on the base material, wherein a cell-adhesive material binds to a non-cell-adhesive material via a photolabile group comprising a coumarinylmethyl skeleton in the cell-adhesive photocontrollable material in which the non-cell-adhesive material, the photolabile group and the cell-adhesive material are bound in this order from the base material side. to

3. A cell-adhesive photocontrollable base material, wherein light irradiation causes the bond dissociation of a photolabile group comprising a coumarinylmethyl skeleton to irreversibly change the surface of the irradiated portion from the cell-adhesive material to the non-cell-adhesive material.

4. The cell-adhesive photocontrollable base material according to any of claims 1 to 3, wherein the photolabile group comprises a divalent coumarinylmethyl skeleton represented by general formula (5) below: 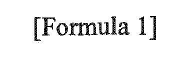 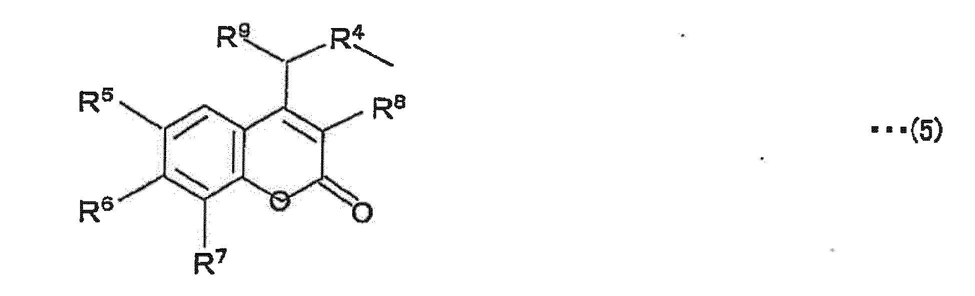 wherein R⁴ represents a divalent group selected from the group consisting of O, CO, CO₂, OCO, OCO₂, OCONH, OCONR, NHCO₂, NH, SO₃, and (OPO(OH))₁₋₃OPO₂; R⁵ represents a group selected from the group consisting of hydrogen, a halogen group, and an alkoxy group; R⁶ represents a group selected from the group consisting of hydrogen, a hydroxy group, an alkoxy group, and a dialkylamino group or is absent; R⁷ represents hydrogen or a halogen group or is absent; R⁸ represents hydrogen or a halogen group; R⁹ represents hydrogen or is absent; and one of two bonds formed by the divalent group represented by the general formula (5) is positioned at R⁴ and the other bond is positioned at R⁶ or R⁷ on the benzene skeleton or R⁹ on the coumarinylmethyl group.

5. The cell-adhesive photocontrollable base material according to any one of claims 1 to 3, wherein the cell-adhesive photocontrollable material comprises a structure in which a cell-adhesive group represented by general formula (4) directly or indirectly binds to a photolabile group represented by general formula (5) at a position of R⁶ or R⁷ on the benzene skeleton or at a position of R⁹ on the coumarinylmethyl:
[Formula 2]
-X (4)
wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; and 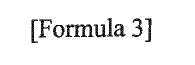 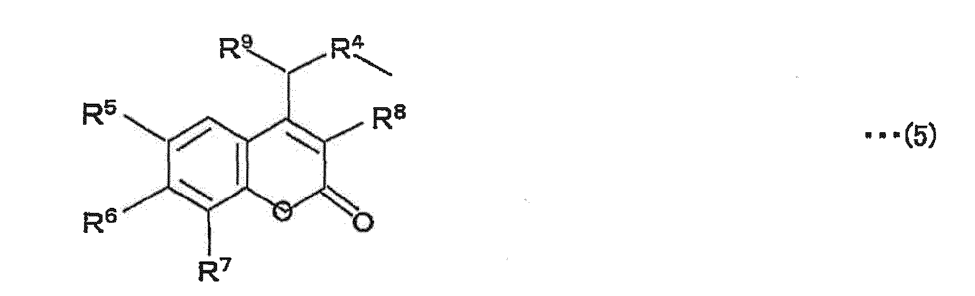 wherein R⁴ represents a divalent group selected from the group consisting of O, CO, CO₂, OCO, OCO₂, OCONH, OCONR, NHCO₂, NH, SO₃, and (OPO(OH))₁₋₃OPO₂; R⁵ represents a group selected from the group consisting of hydrogen, a halogen group, and an alkoxy group; R⁶ represents a group selected from the group consisting of hydrogen, a hydroxy group, an alkoxy group, and a dialkylamino group or is absent; R⁷ represents hydrogen or a halogen group or is absent; R⁸ represents hydrogen or a halogen group; R⁹ represents hydrogen or is absent; and one of two bonds formed by the divalent group represented by the general formula (5) is positioned at R⁴ and the other bond is positioned at R⁶ or R⁷ on the benzene skeleton or R⁹ on the coumarinylmethyl group.

6. The cell-adhesive photocontrollable base material according to any one of claims 1 to 3, wherein the cell-adhesive photocontrollable material comprises a structure represented by general formula (6) below in which the cell-adhesive group binds to the photolabile group on the benzene skeleton via a divalent linking group R¹⁰, a structure represented by general formula (7) below in which the cell-adhesive group binds to the photolabile group on the benzene skeleton via a divalent linking group R¹¹, or a structure represented by general formula (8) below in which the cell-adhesive group binds to the photolabile group on the coumarinylmethyl group via a divalent linking group R¹²: 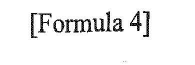 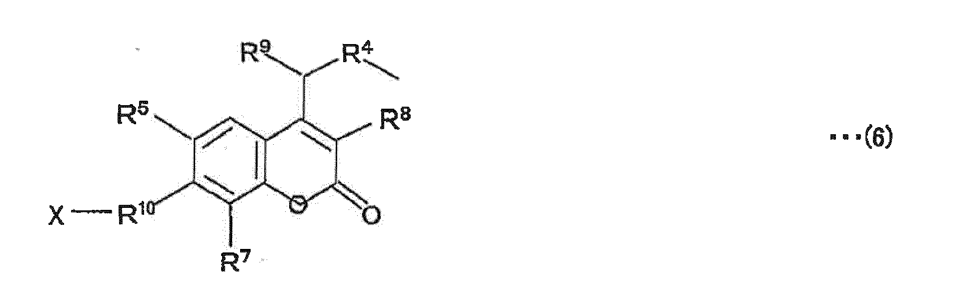 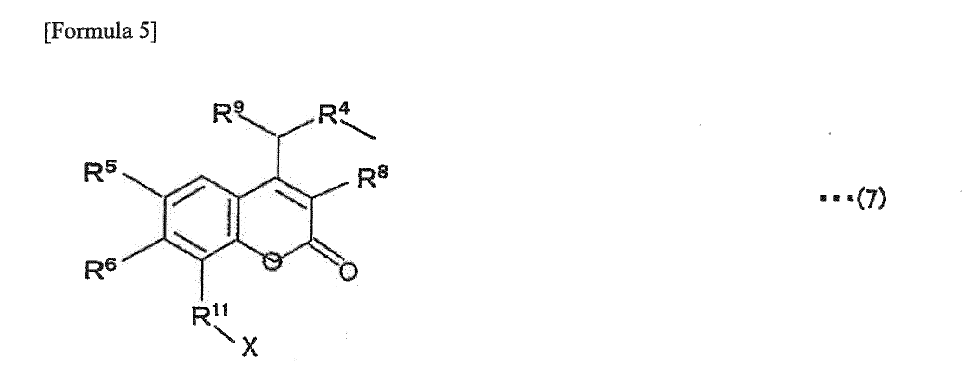 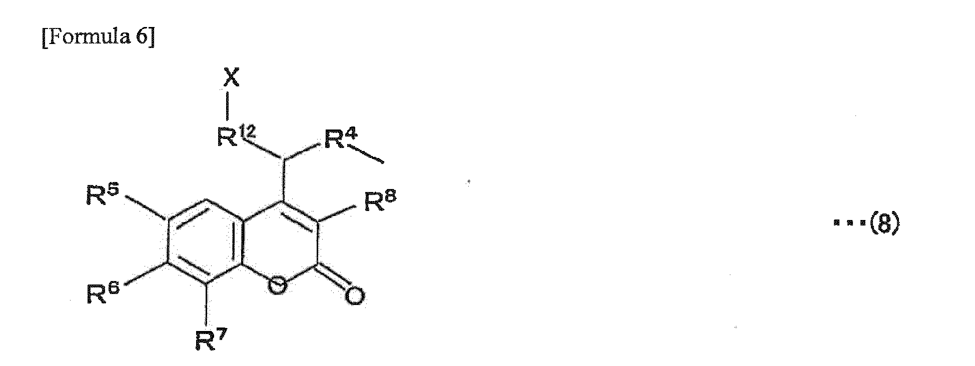 wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; R⁴ represents a divalent group selected from the group consisting of O, CO, CO₂, OCO, OCO₂, OCONH, OCONR, NHCO₂, NH, SO₃, and (OPO(OH))₁₋₃OPO₂; R⁵ represents a group selected from the group consisting of hydrogen, a halogen group, and an alkoxy group; R⁶ represents a group selected from the group consisting of hydrogen, a hydroxy group, an alkoxy group, and a dialkylamino group; R⁷ represents hydrogen or a halogen group; R⁸ represents hydrogen or a halogen group; R⁹ represents hydrogen; the divalent linking group R¹⁰ represents a group selected from the group consisting of O(CH₂)ₘ, O(CH₂CH₂O)ₘ, OCO(CH₂)ₘ, OCOCH₂O(CH₂CH₂O)ₘ, OCH₂CO₂(CH₂CH₂O)ₘ(CH₂)_{m'}, OCH₂CONHCH₂(C₂HN₃)(CH₂)_{m'} (OCH₂CH₂)ₘ, OCH₂CONCH₃CH₂(C₂HN₃)(CH₂)_{m'} (OCH₂CH₂)ₘ, and OCH₂CON(CH₂(C₂HN₃)(CH₂)_{m'}(OCH₂CH₂)ₘX) CH₂(C₂HN₃)(CH₂)_{m'} (OCH₂CH₂)ₘ (wherein each of m and m' is an integer of 0 to 20); the divalent linking group R¹¹ represents a group selected from the group consisting of CH₂NH(CH₂CH₂O)ₘ(CH₂)_{m'}, CH₂N((CH₂CH₂O)ₘ(CH₂)_{m'} X) (CH₂CH₂O)ₘ(CH₂)_{m'}, CH₂NHCH₂(C₂HN₃)(CH₂)_{m'}(OCH₂CH₂)ₘ, CH₂N(CH₂(C₂HN₃)(CH₂)_{m'}(OCH₂CH₂)ₘX) CH₂(C₂HN₃)(CH₂)_{m'}(OCH₂CH₂)ₘ, CH₂N(CH₃), and CH₂N(CH₃) CH₂(C₂HN₃)(CH₂)_{m'}(OCH₂CH₂)ₘ (wherein each of m and m' is an integer of 0 to 20); and the divalent linking group R¹² represents a group CH₂OCO(CH₂)_{m'} or (CH₂)_{m"}(C₂HN₃)(CH₂)_{m'}(OCH₂CH₂)ₘ (wherein each of m, m', and m" is an integer of 0 to 20).

7. The cell-adhesive photocontrollable base material according to any one of claims 1 to 3, wherein the cell-adhesive photocontrollable material comprises a structure in which the photolabile group binds to the cell-adhesive group at a position of R⁴ on the coumarinylmethyl skeleton via a divalent linking group R¹³, represented by general formula (9) below: 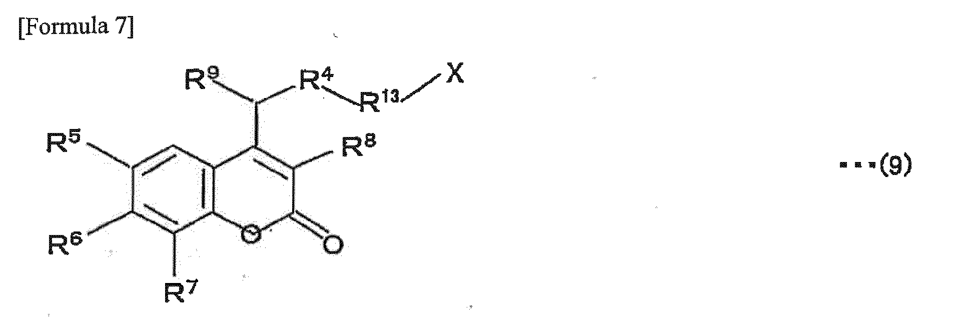 wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; R⁴ represents a divalent group selected from the group consisting of O, CO, CO₂, OCO, OCO₂, OCONH, OCONR, NHCO₂, NH, SO₃, and (OPO(OH))₁₋₃OPO₂; R⁵ represents a group selected from the group consisting of hydrogen, a halogen group, and an alkoxy group; R⁶ represents a group selected from the group consisting of hydrogen, a hydroxy group, an alkoxy group, and a dialkylamino group or is absent; R⁷ represents hydrogen or a halogen group or is absent; R⁸ represents hydrogen or a halogen group; R⁹ represents hydrogen or is absent; and the divalent linking group R¹³ represents a group (CH₂CH₂O)ₘ(CH₂)_{m'} or (CH₂)ₘ(C₂HN₃)(CH₂)_{m'} (wherein each of m and m' is an integer of 0 to 20).

8. The cell-adhesive photocontrollable base material according to any one of claims 1 to 3, wherein the cell-adhesive photocontrollable material comprises a structure in which a cell-adhesive group represented by general formula (4) directly or indirectly binds to a photolabile group represented by general formula (5) at a position of R⁶ or R⁷ on the benzene skeleton or at a position of R⁹ on the coumarinylmethyl, or at a position of R⁴, directly or indirectly binds to the non-cell-adhesive material:
[Formula 8]
-X (4) 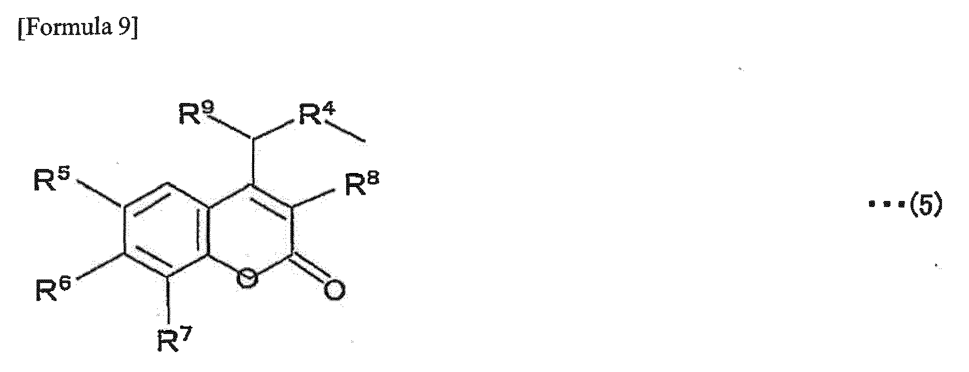
wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; R⁴ represents a divalent group selected from the group consisting of O, CO, CO₂, OCO, OCO₂, OCONH, OCONR, NHCO₂, NH, SO₃, and (OPO(OH))₁₋₃OPO₂; R⁵ represents a group selected from the group consisting of hydrogen, a halogen group, and an alkoxy group; R⁶ represents a group selected from the group consisting of hydrogen, a hydroxy group, an alkoxy group, and a dialkylamino group or is absent; R⁷ represents hydrogen or a halogen group or is absent; R⁸ represents hydrogen or a halogen group; and R⁹ represents hydrogen or is absent.

9. The cell-adhesive photocontrollable base material according to any one of claims 1 to 3, wherein the cell-adhesive photocontrollable material comprises a (meth)acrylic acid ester polymer structure represented by any of general formulas (10), (11), and (12) below: 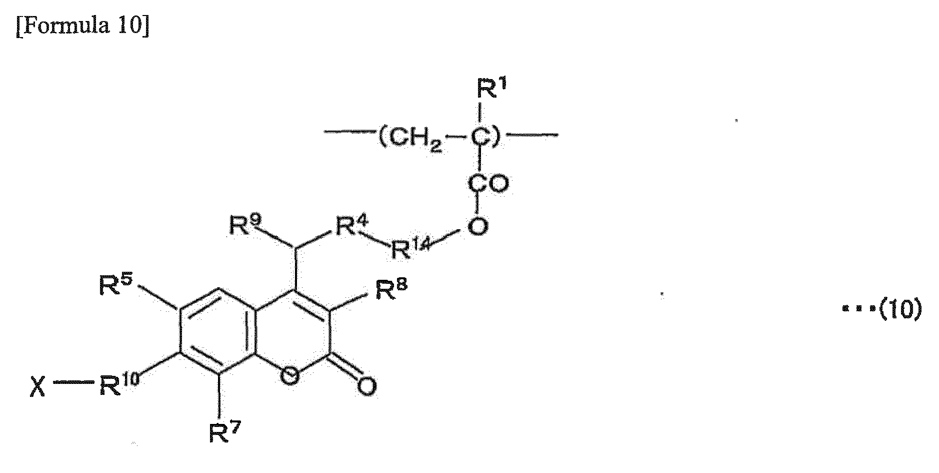 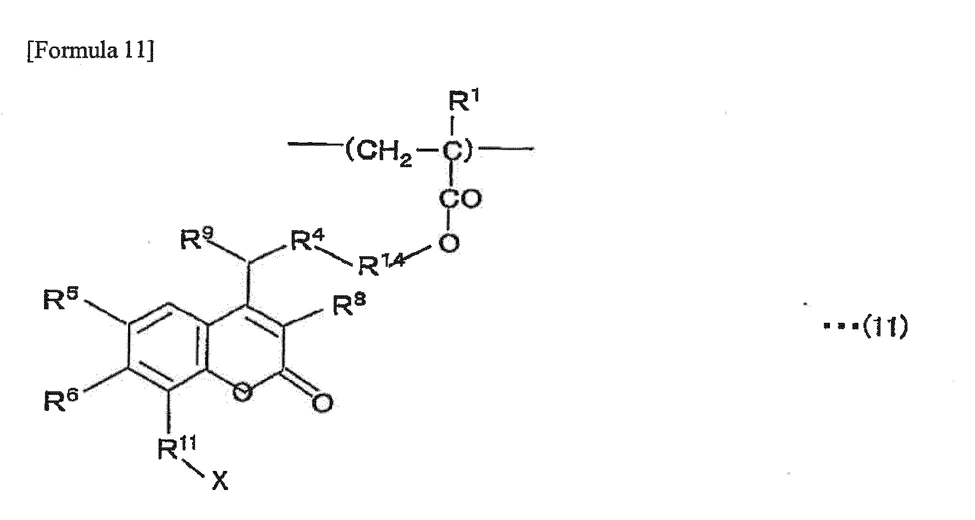 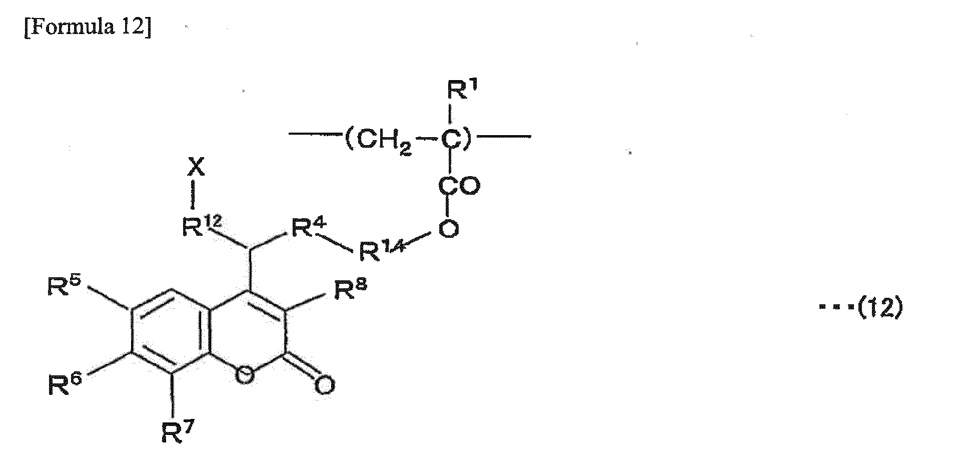 wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; R¹ represents hydrogen or a methyl group; R⁴ represents a divalent group selected from the group consisting of O, CO, CO₂, OCO, OCO₂, OCONH, OCONR, NHCO₂, NH, SO₃ and (OPO(OH))₁₋₃OPO₂ ; R⁵ represents a group selected from the group consisting of hydrogen, a halogen group, and an alkoxy group; R⁶ represents a group selected from the group consisting of hydrogen, a hydroxy group, an alkoxy group, and a dialkylamino group; R⁷ represents hydrogen or a halogen group; R⁸ represents hydrogen or a halogen group; R⁹ represents hydrogen; the divalent linking group R¹⁰ represents a group selected from the group consisting of O(CH₂)ₘ, O(CH₂CH₂O)ₘ, OCO(CH₂)ₘ, OCOCH₂O(CH₂CH₂O)ₘ, OCH₂CO₂(CH₂CH₂O)ₘ(CH₂)_{m'}, OCH₂CONHCH₂(C₂HN₃)(CH₂)_{m'}(OCH₂CH₂)ₘ, OCH₂CONCH₃CH₂(C₂HN₃)(CH₂)_{m'} (OCH₂CH₂)ₘ, and OCH₂CON(CH₂(C₂HN₃)(CH₂)_{m'}(OCH₂CH₂)ₘX) CH₂(C₂HN₃)(CH₂)_{m'}(OCH₂CH₂)ₘ (wherein each of m and m' is an integer of 0 to 20); the divalent linking group R¹¹ represents a group selected from the group consisting of CH₂NH(CH₂CH₂O)ₘ(CH₂)_{m'}, CH₂N((CH₂CH₂O)ₘ(CH₂)_{m'}X) (CH₂CH₂O)ₘ(CH₂)_{m'}, CH₂NHCH₂(C₂HN₃) (CH₂)_{m'} (OCH₂CH₂)ₘ, CH₂N(CH₂(C₂HN₃)(CH₂)_{m'}(OCH₂CH₂)ₘX) CH₂(C₂HN₃)(CH₂)_{m'}(OCH₂CH₂)ₘ, CH₂N(CH₃), and CH₂N(CH₃)CH₂(C₂HN₃) (CH₂)_{m'} (OCH₂CH₂)ₘ (wherein each of m and m' is an integer of 0 to 20); the divalent linking group R¹² represents a group CH₂OCO(CH₂)_{m'} or (CH₂)_{m"}(C₂HN₃)(CH₂)_{m'}(OCH₂CH₂)ₘ (wherein each of m, m', and m" is an integer of 0 to 20); and the divalent linking group R¹⁴ represents a group selected from the group consisting of an alkylene, (CH₂)_{p'}CO(OCH₂CH₂)ₚ, and (CH₂)_{p"}(C₂HN₃)(CH₂)_{p'}CO(OCH₂CH₂)ₚ (wherein each of p, p', and p" is an integer of 1 to 20).

10. The cell-adhesive photocontrollable base material according to any one of claims 1 to 3, wherein the cell-adhesive photocontrollable material comprises a (meth)acrylic acid ester polymer structure represented by any of general formulas (13), (14), and (15) below: 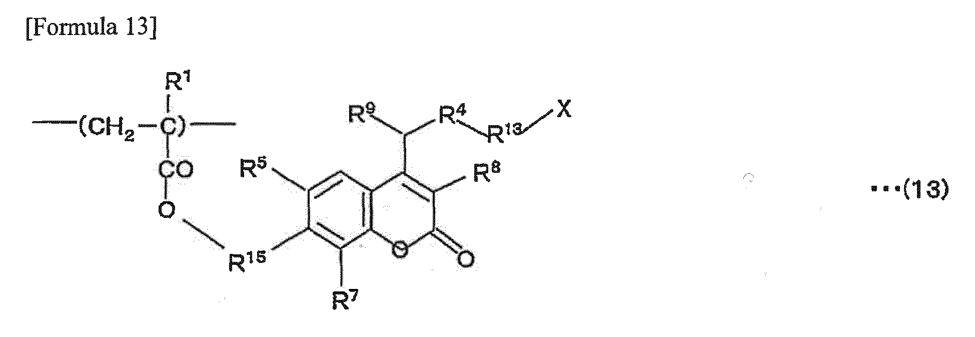 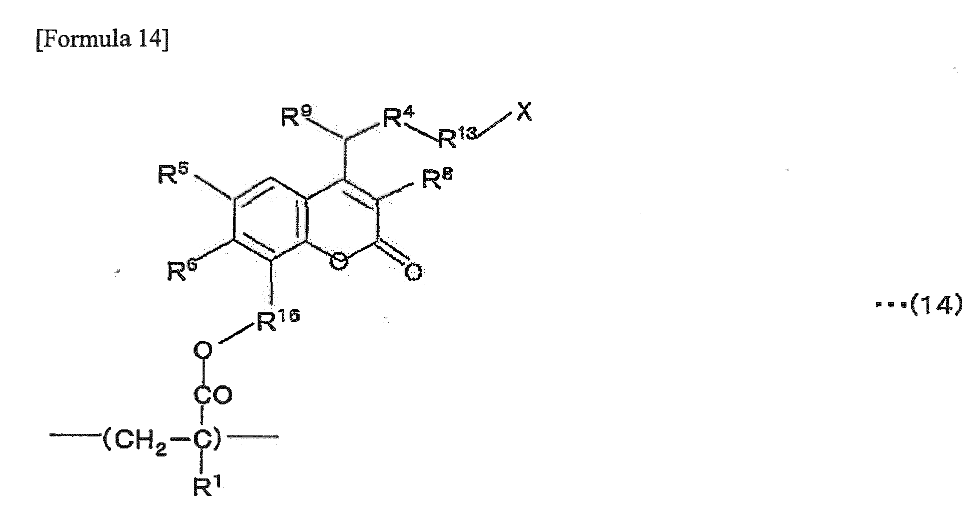 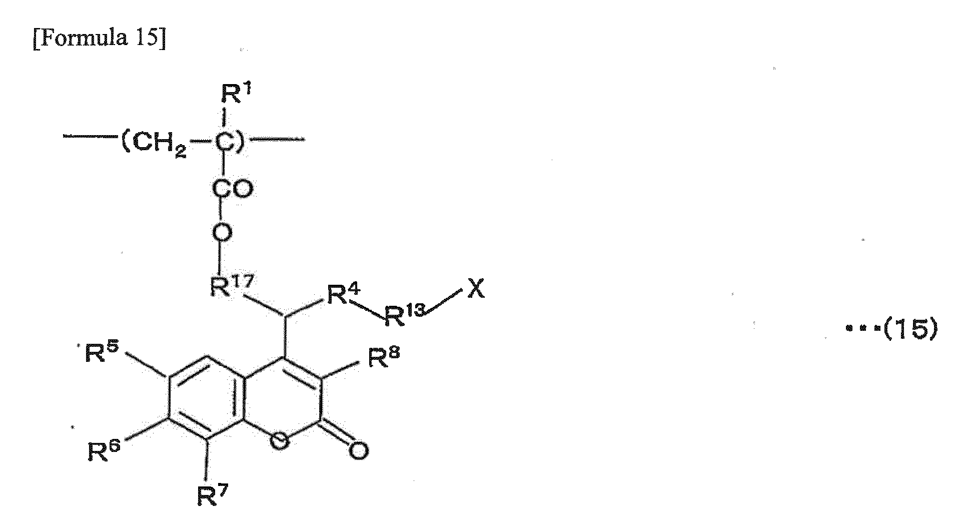 wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; R¹ represents hydrogen or an alkyl group; R⁴ represents a divalent group selected from the group consisting of O, CO, CO₂, OCO, OCO₂, OCONH, OCONR, NHCO₂, NH, SO₃ and (OPO(OH)₁₋₃OPO₂; R⁵ represents a group selected from the group consisting of hydrogen, a halogen group, and an alkoxy group; R⁶ represents a group selected from the group consisting of hydrogen, a hydroxy group, an alkoxy group, and a dialkylamino group; R⁷ represents hydrogen or a halogen group; R⁸ represents hydrogen or a halogen group; R⁹ represents hydrogen; the divalent linking group R¹³ represents a group (CH₂CH₂O)ₘ(CH₂)_{m'} or (CH₂)ₘ(C₂HN₃)(CH₂)_{m'} (wherein each of m and m' is an integer of 0 to 20); the divalent linking group R¹⁵ represents a group (CH₂CH₂O)ₚOCOCH₂O (wherein p is an integer of 1 to 20); and R¹⁶ represents a group selected from the group consisting of (CH₂CH₂O)ₚCOCH₂NHCH₂, (CH₂CH₂O)ₚCOCH₂N(CH₃)CH₂, (CH₂CH₂O)ₚCO(CH₂)_{p'}(N₃C₂H)CH₂NHCH₂, (CH₂CH₂O)ₚCO(CH₂)_{p'}(N₃C₂H)CH₂N(CH₃)CH₂, (wherein each of p and p' is an integer of 1 to 20); and R¹⁷ represents a group (CH₂)ₚCO₂CH₂ (wherein p is an integer of 1 to 20).

11. The cell-adhesive photocontrollable base material according to any one of claims 1 to 3, wherein the cell-adhesive photocontrollable material comprises any of (meth)acrylic ester copolymers represented by general formulas (1) or (2), and (10) below; general formulas (1) or (2), and (11) below; general formulas (1) or (2), and (12) below; general formulas (1) or (2), and (13) below; general formulas (1) or (2), and (14) below; general formulas (1) or (2), and (15) below; general formulas (1), (10), and (2) below; general formulas (1), (11), and (2) below; general formulas (1), (12), and (2) below; general formulas (1), (13), and (2) below; general formulas (1), (14), and (2) below; or general formulas (1), (15), and (2) below: 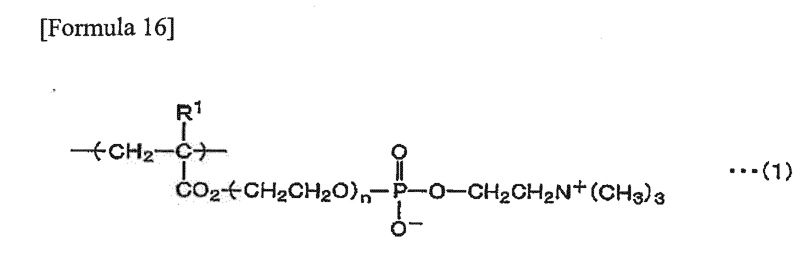 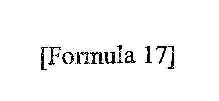 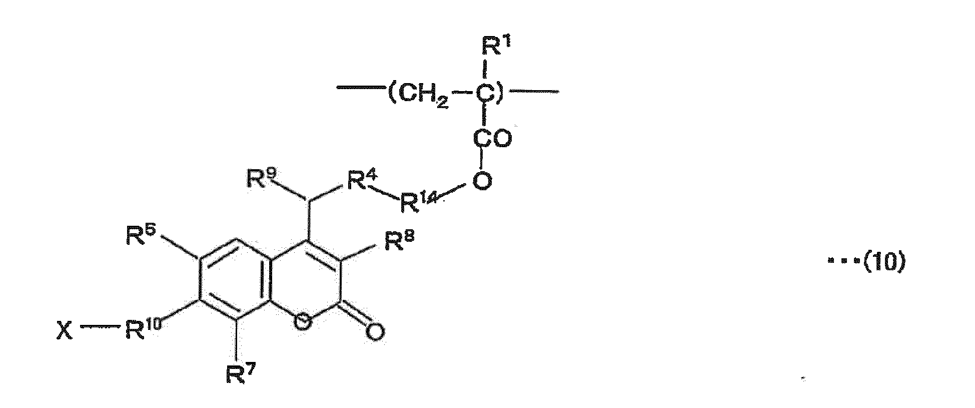 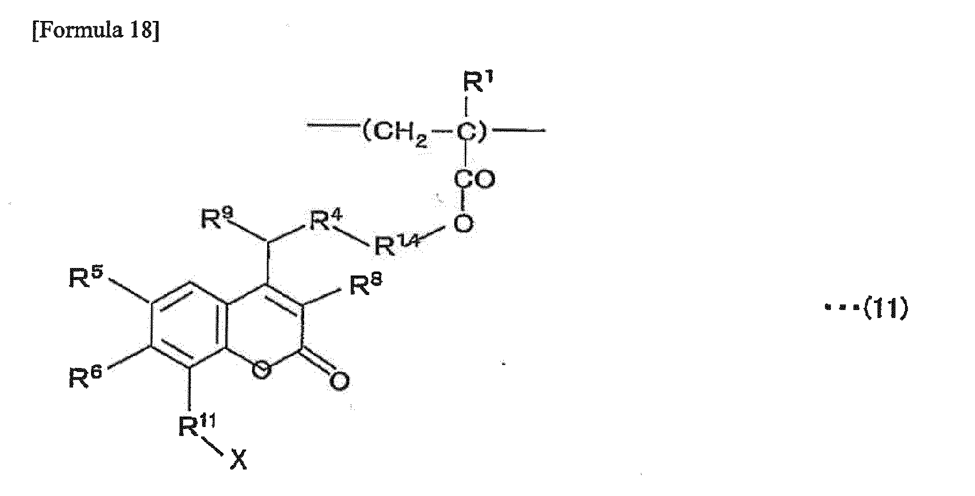 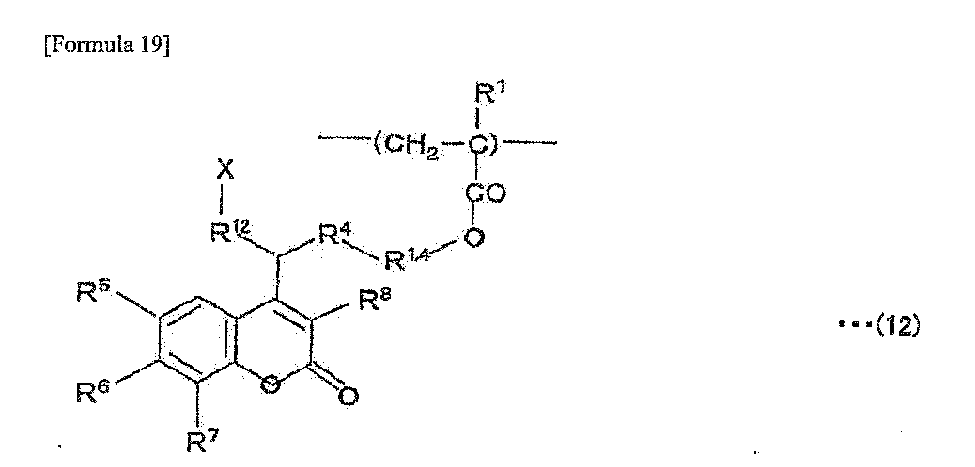 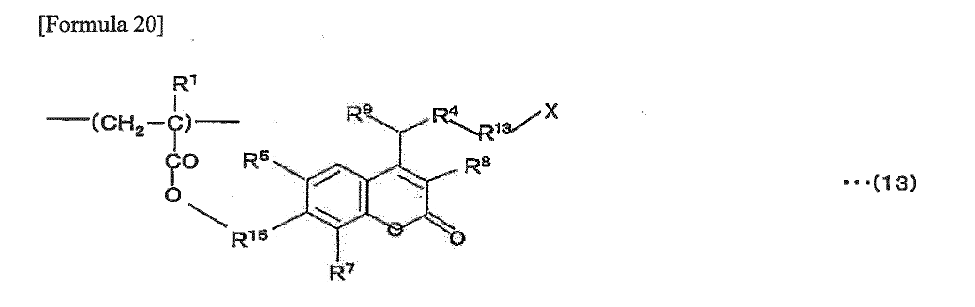 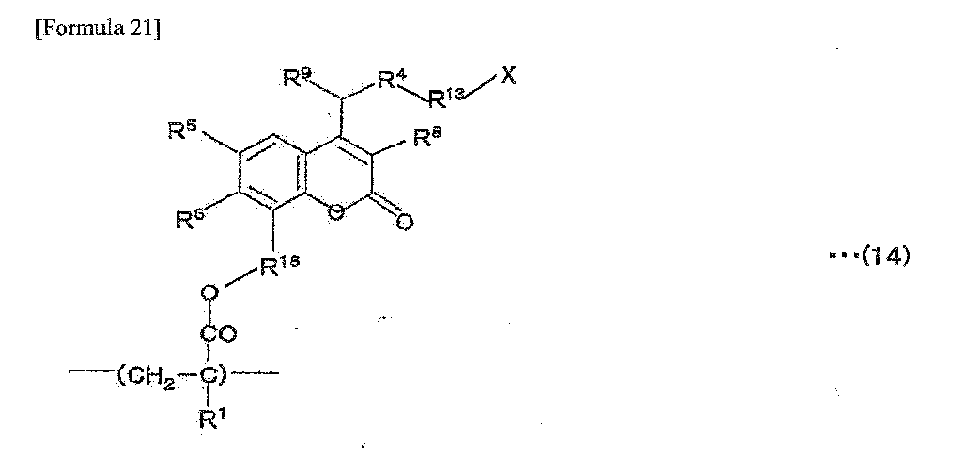 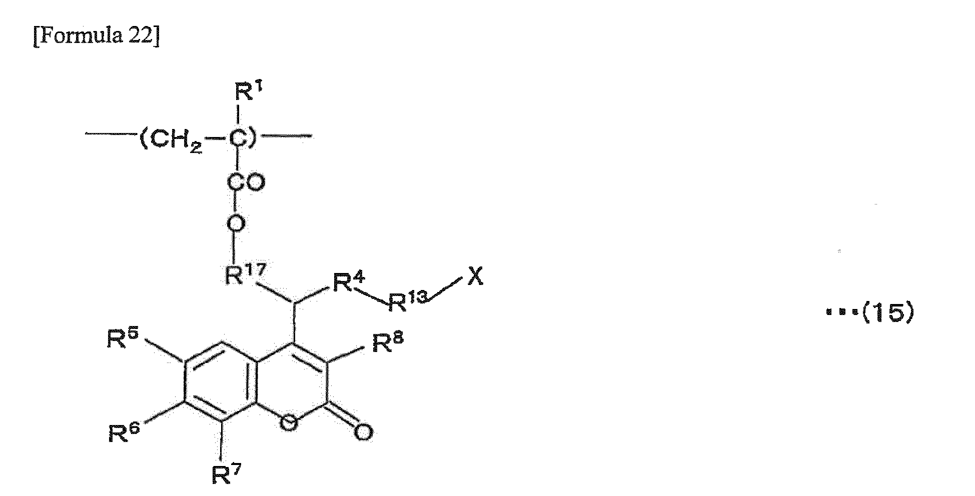 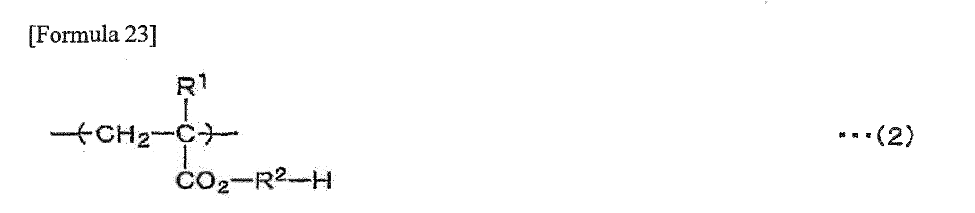 wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; R¹ represents hydrogen or a methyl group; n represents an integer of 1 to 20; R² represents 1 to 20 alkylene groups or 1 to 20 polyoxyethylene groups; R⁴ represents a divalent group selected from the group consisting of O, CO, CO₂, OCO, OCO₂, OCONH, OCONR, NHCO₂, NH, SO₃ and (OPO(OH))₁₋₃OPO₂ ; R⁵ represents a group selected from the group consisting of hydrogen, a halogen group, and an alkoxy group; R⁶ represents a group selected from the group consisting of hydrogen, a hydroxy group, an alkoxy group, and a dialkylamino group; R⁷ represents hydrogen or a halogen group; R⁸ represents hydrogen or a halogen group; R⁹ represents hydrogen; the divalent linking group R¹⁰ represents a group selected from the group consisting of O(CH₂)ₘ, O(CH₂CH₂O)ₘ, OCO(CH₂)ₘ, OCOCH₂O(CH₂CH₂O)ₘ, OCH₂CO₂(CH₂CH₂O)ₘ(CH₂)_{m'}, OCH₂CONHCH₂(C₂HN₃)(CH₂)_{m'}(OCH₂CH₂)ₘ, OCH₂CONCH₃CH₂(C₂HN₃)(CH₂)_{m'} (OCH₂CH₂)ₘ, and OCH₂CON(CH₂(C₂HN₃)(CH₂)_{m'}(OCH₂CH₂)ₘX) CH₂(C₂HN₃)(CH₂)_{m'}(OCH₂CH₂)ₘ (wherein each of m and m' is an integer of 0 to 20); the divalent linking group R¹¹ represents a group selected from the group consisting of CH₂NH(CH₂CH₂O)ₘ(CH₂)_{m'}, CH₂N((CH₂CH₂O)ₘ(CH₂)_{m'}X) (CH₂CH₂O)ₘ(CH₂)_{m'}, CH₂NHCH₂(C₂HN₃)(CH₂)_{m'}(OCH₂CH₂)ₘ, CH₂N(CH₂(C₂HN₃)(CH₂)_{m'}(OCH₂CH₂)ₘX) CH₂(C₂HN₃)(CH₂)_{m'}(OCH₂CH₂)ₘ, CH₂N(CH₃), and CH₂N(CH₃)CH₂(C₂HN₃)(CH₂)_{m'}(OCH₂CH₂)ₘ (wherein each of m and m' is an integer of 0 to 20); the divalent linking group R¹² represents a group CH₂OCO(CH₂)_{m'} or (CH₂)_{m"}(C₂HN₃)(CH₂)_{m'}(OCH₂CH₂)ₘ (wherein each of m, m', and m" is an integer of 0 to 20); the divalent linking group R¹³ represents a group (CH₂CH₂O)ₘ(CH₂)_{m'} or (CH₂)ₘ(C₂HN₃)(CH₂)_{m'} (wherein each of m and m' is an integer of 0 to 20); the divalent linking group R¹⁴ represents a group selected from the group consisting of an alkylene, (CH₂)_{p'}CO(OCH₂CH₂)ₚ, and (CH₂)_{p"}(C₂HN₃)(CH₂)_{p'}CO(OCH₂CH₂)ₚ (wherein each of p, p', and p" is an integer of 1 to 20); the divalent linking group R¹⁵ represents a group (CH₂CH₂)ₚOCOCH₂O (wherein p is an integer of 1 to 20); the divalent linking group R¹⁶ represents a group selected from the group consisting of (CH₂CH₂O)ₚCOCH₂NHCH₂, (CH₂CH₂O)ₚCOCH₂N(CH₃)CH₂, (CH₂CH₂O)ₚCO(CH₂)_{p'}(N₃C₂H)CH₂NHCH₂, (CH₂CH₂O)ₚCO(CH₂)_{p'}(N₃C₂H)CH₂N(CH₃)CH₂, (wherein each of p and p' is an integer of 1 to 20); and the divalent linking group R¹⁷ represents a group (CH₂)ₚCO₂CH₂ (wherein p is an integer of 1 to 20).

12. The cell-adhesive photocontrollable base material according to any one of claims 1 to 3, wherein the cell-adhesive photocontrollable material has a (meth)acrylic acid ester comprising an alkoxysilane copolymerized at the side chain.

13. The cell-adhesive photocontrollable base material according to any one of claims 1 to 3, wherein a film formation of an alkoxysilane represented by general formula (16), (17), or (18) below is conducted on the base material and then a cell-adhesive group X is formed in the terminal end by the reaction with an azide compound comprising the cell-adhesive group X, represented by general formula (57) below in the cell-adhesive photocontrollable material: 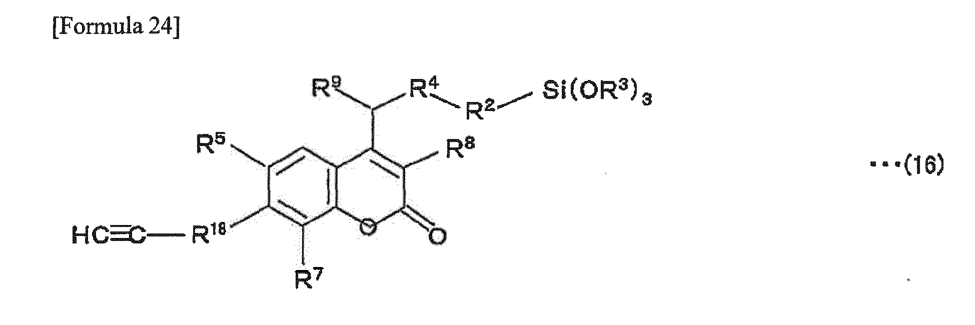 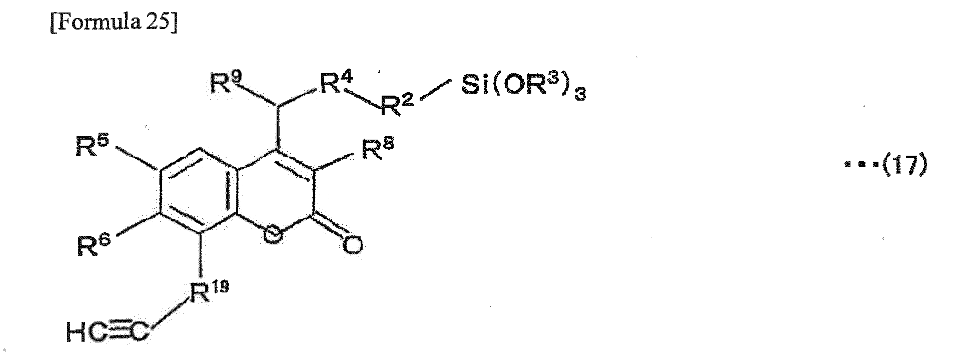 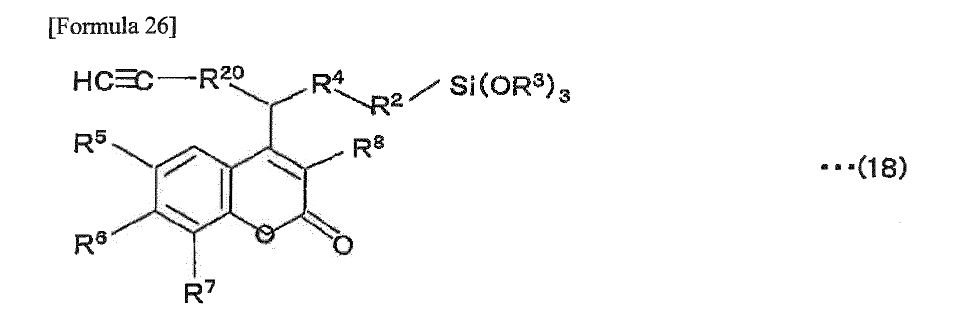 wherein R² represents a group selected from the group consisting of 1 to 20 alkylene groups, 1 to 20 polyoxyethylene groups, and (CH₂)_{q}CONH(CH₂)_{q'} (wherein each of q and q' is an integer of 0 to 20); R³ represents hydrogen or an alkyl group; R⁴ represents a divalent group selected from the group consisting of O, CO, CO₂, OCO, OCO₂, OCONH, OCONR, NHCO₂, NH, SO₃ and (OPO(OH))₁₋₃OPO₂ ; R⁵ represents a group selected from the group consisting of hydrogen, a halogen group, and an alkoxy group; R⁶ represents a group selected from the group consisting of hydrogen, a hydroxy group, an alkoxy group, and a dialkylamino group; R⁷ represents hydrogen or a halogen group; R⁸ represents hydrogen or a halogen group; R⁹ represents hydrogen; the divalent linking group R¹⁸ represents a group selected from the group consisting of CH₂NHCOCH₂O, CH₂NCH₃COCH₂O, and CH₂N(CH₂CCH)COCH₂O; the divalent linking group R¹⁹ represents a group selected from the group consisting of CH₂NHCH₂, CH₂NCH₃CH₂, and CH₂N(CH₂CCH)CH₂; and the divalent linking group R²⁰ represents (CH₂)_{q} (wherein q is an integer of 0 to 20); and
[Formula 27]
X-R²¹-N₃ (57)
wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkylmono- or polyhydrazide group, an amino acid group, a polypeptide group, and a nucleic acid group; and the divalent linking group R²¹ represents (CH₂)_{q'} (OCH₂CH₂)_{q} (wherein q and q' are an integer of 0 to 20).

14. The cell-adhesive photocontrollable base material according to any one of claims 1 to 3, wherein the cell-adhesive photocontrollable material is an alkoxysilane represented by general formulas (24), (25), (26), or (27) below: 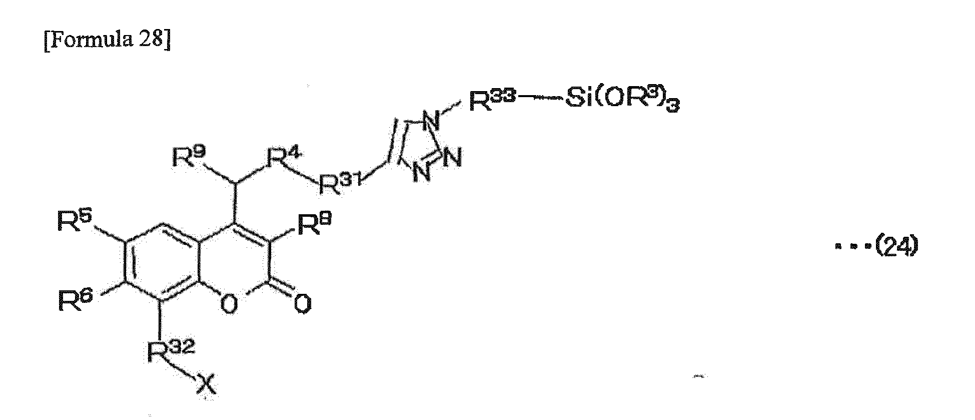 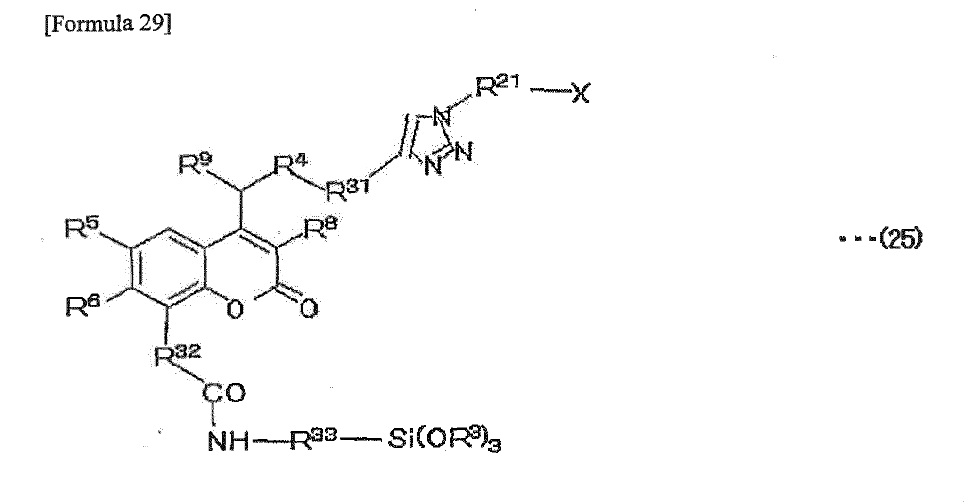 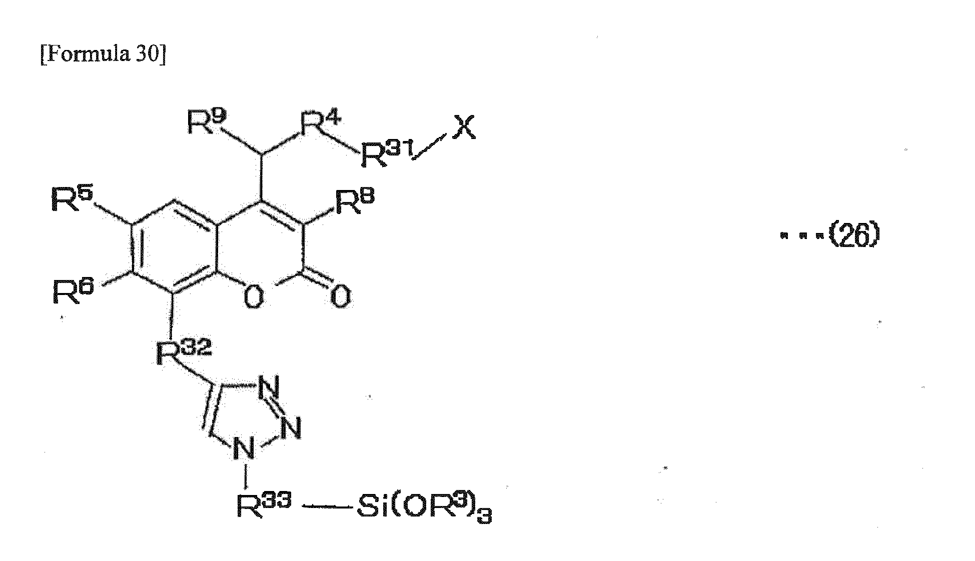 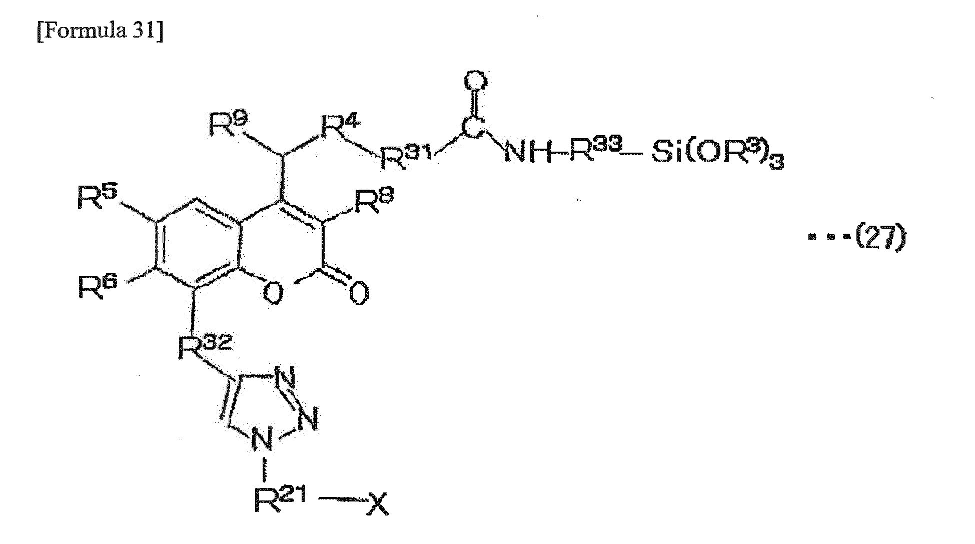 wherein R³ represents hydrogen or an alkyl group; R⁴ represents a divalent group selected from the group consisting of O, CO, CO₂, OCO, OCO₂, OCONH, OCONR, NHCO₂, NH, SO₃ and (OPO(OH))₁₋₃OPO₂ ; R⁵ represents a group selected from the group consisting of hydrogen, a halogen group, and an alkoxy group; R⁶ represents a group selected from the group consisting of hydrogen, a hydroxy group, an alkoxy group, and a dialkylamino group;
R⁸ represents hydrogen or a halogen group; R⁹ represents hydrogen; the divalent linking group R³¹ represents (CH₂)ᵣ; the divalent linking group R³² represents CH₂NHCH₂ or CH₂NCH₃CH₂; the divalent linking group R³³ represents (CH₂)ᵣ; the divalent linking group R²¹ represents a group (CH₂)_{q'}(OCH₂CH₂)_{q}; X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; Y represents a group selected from the group consisting of an azide group, an amino group, an epoxy group, and a cyanate group; r is an integer of 0 to 20; and q and q' are an integer of 0 to 20.

15. An intermediate material represented by any of general formulas (22) and (23) below, wherein the intermediate material is used in a cell-adhesive photocontrollable material according to any one of claims 1 to 3: 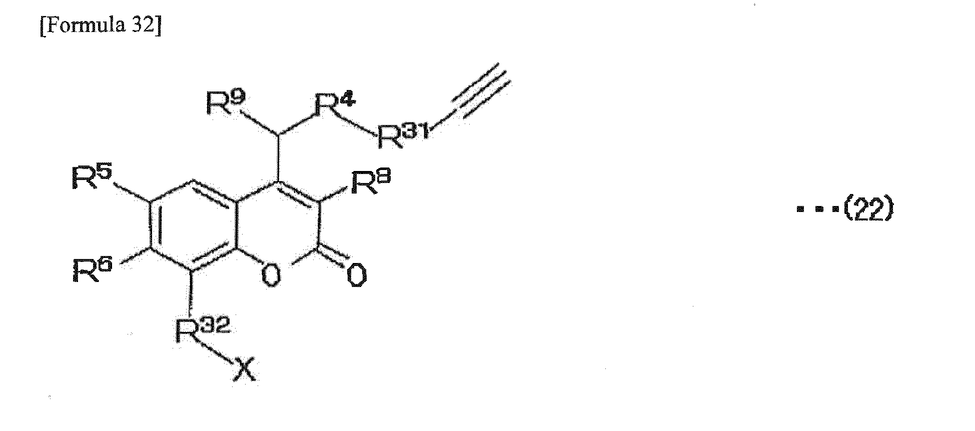 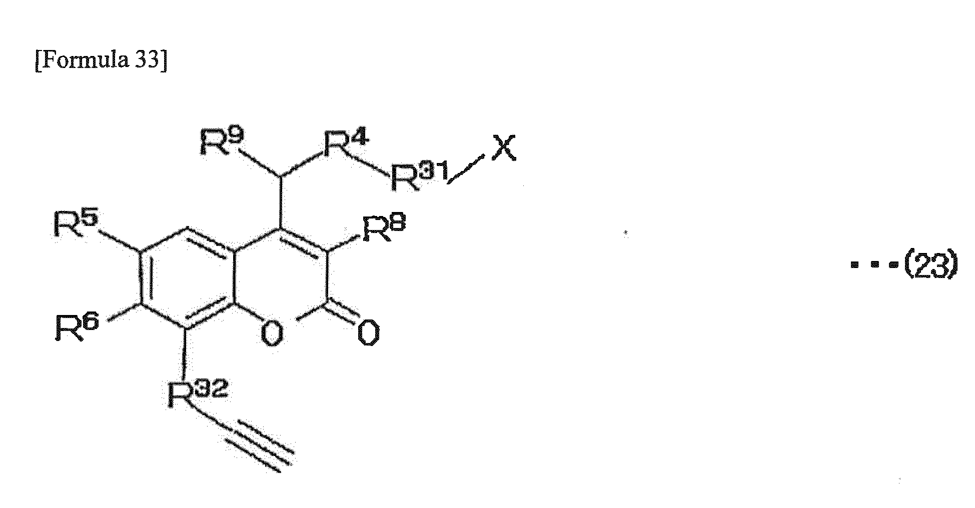 wherein R⁴ represents a divalent group selected from the group consisting of O, CO, CO₂, OCO, OCO₂, OCONH, OCONR, NHCO₂, NH, SO₃ and (OPO(OH))₁₋₃OPO₂ ; R⁵ represents a group selected from the group consisting of hydrogen, a halogen group, and an alkoxy group; R⁶ represents a group selected from the group consisting of hydrogen, a hydroxy group, an alkoxy group, and a dialkylamino group; R⁸ represents hydrogen or a halogen group; R⁹ represents hydrogen; the divalent linking group R³¹ represents (CH₂)ᵣ; the divalent linking group R³² represents CH₂NHCH₂ or CH₂NCH₃CH₂; X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; and r represents an integer of 0 to 20.

16. The cell-adhesive photocontrollable base material according to any one of claims 1 to 3, wherein the cell-adhesive material is a material in which an extracellular matrix promoting adhesion to cells, an antibody capable of binding to a surface antigen of cells, or a protein to bind the antibody binds or adheres to the cell-adhesive group.

17. The cell-adhesive photocontrollable base material according to claim 16, wherein the extracellular matrix is a material selected from the group consisting of: collagens; non-collagenous glycoproteins including fibronectin, vitronectin, laminin, nidogen, teneinosine, thrombospondi, von Willebrand, osteopontin, and fibrinogen; elastins; and proteoglycans.

18. The cell-adhesive photocontrollable base material according to claim 16, wherein the protein to bind the antibody is a material selected from the group consisting of avidin/biotin, protein A, and protein G.

19. The cell-adhesive photocontrollable base material according to any one of claims 1 to 3, wherein the base material is a glass culture vessel.

20. The cell-adhesive photocontrollable base material according to any one of claims 1 to 3, wherein the cell-adhesive material comprises a cell-adhesive group represented by general formula (4) below:
[Formula 34]
-X (4)
wherein X comprises a covalently-bound functional group which is a functional group having a property of performing covalent binding reaction.

21. The cell-adhesive photocontrollable base material according to claim 20, wherein the covalently-bound functional group is a functional group having a property of performing covalent binding reaction with an amino group, a hydroxy group, an aldehyde group, or a ketone group.

22. The cell-adhesive photocontrollable base material according to claim 21, wherein the covalently-bound functional group is a functional group comprising: an active ester group of a carboxylic acid; an activated carboxylic acid; a functional group with a largely strained 3-membered ring; a functional group reacting with a hydroxy group; or a functional group reacting with an aldehyde group or a ketone group.

23. The cell-adhesive photocontrollable base material according to claim 22, wherein the active ester group of a carboxylic acid includes N-hydroxysuccinimide ester and N-hydroxysulfosuccinimide ester; the activated carboxylic acid includes a carboxylic acid halide, a carboxylic acid anhydride, and a carboxylic acid azide; the functional group with a largely strained 3-membered ring includes an epoxide group, an aziridine group, an aziridinium group, and an episulfonium group; the functional group reacting with a hydroxy group includes a p-toluenesulfonyl group; and the functional group reacting with an aldehyde group or a ketone group includes 1,3-diol, hydrazine, and hydroxylamine ether.

24. The cell-adhesive photocontrollable base material according to any one of claims 20 to 23, wherein the covalently-bound functional group is a functional group having a property of performing covalent binding reaction with a cell surface functional group which is a component of a compound present on cell surface.

25. The cell-adhesive photocontrollable base material according to any one of claims 20 to 23, wherein the covalently-bound functional group is a functional group having a property of performing covalent binding reaction with an extracellular matrix, an antibody capable of binding to a cell surface antigen, or a protein to bind the antibody.

26. The cell-adhesive photocontrollable base material according to claim 16 or 25, wherein the antibody is an antibody against a cell surface antigen including a major histocompatibility complex (MHC), a human leukocyte antigen (HLA), an epithelial cell adhesion molecule (EpCAM), and p24 (CD9).

27. The cell-adhesive photocontrollable base material according to claim 26, wherein the antibody is an antibody against an animal species-specific cell surface antigen including human MHC class Ia molecules HLA-A, B, and C and mouse H-2 and CD9 molecules.

28. The cell-adhesive photocontrollable base material according to claim 27, wherein the antibody is an antibody of clone W6/32, clone B9.12.1, clone MZ3, clone M1/42, or clone hrk29.

29. An animal species-specific cell-adhesive base material comprising a cell-adhesive base material prepared by binding a cell-adhesive material to a base material, wherein an antibody against an animal species-specific cell surface antigen including human MHC class Ia molecules HLA-A, B, and C and mouse H-2 and CD9 molecules is used as the cell-adhesive material.

30. A cell adhesion apparatus comprising a cell-adhesive photocontrollable base material according to any one of claims 1 to 14 and 16 to 29 and a member which fixes the base material to a rotator, wherein the apparatus has a function of allowing centrifugal force to act in a direction substantially perpendicular to the surface of the base material by rotating the rotator and has a function of causing cells to precipitate and/or pressure-contact onto the surface of the base material by the centrifugal force.

31. A cell adhesion method comprising allowing centrifugal force to act in a direction substantially perpendicular to the surface of a cell-adhesive photocontrollable base material according to any of claims 1 to 14 and 16 to 29, thereby causing cells precipitate and/or pressure-contact onto the surface of the base material.

32. A cell manipulation method comprising the step of manipulating cells using a cell-adhesive photocontrollable base material according to any of claims 1 to 29, wherein an amino group-free buffer solution, particularly preferably an amino group-free HBSS(+) comprising Ca²⁺ or Mg²⁺ is used as a buffer solution.
